# EUROPEAN PATENT APPLICATION

(11) **EP 2 204 379 A1**
(43) Date of publication of application: **07.07.2010**
(21) Application number: 08845535.7
(22) Date of filing: 28.10.2008
(51) Int. Cl.: C07K 7/56, A61K 38/00, A61P 31/10

(54) **POLYPEPTIDE COMPOUND**

(30) Priority: 29.10.2007 JP 2007280446
(71) Applicant: Astellas Pharma Inc., Tokyo 103-8411 (JP)
(72) Inventor: TOMISHIMA, Masaki, Tokyo 103-8411 (JP); MORIKAWA, Hiroshi, Tokyo 103-8411 (JP); MAKINO, Takuya, Tokyo 103-8411 (JP); IMANISHI, Masashi, Tokyo 103-8411 (JP); KAYAKIRI, Natsuko, Tokyo 103-8411 (JP); ASANO, Toru, Tokyo 103-8411 (JP); ARAKI, Takanobu, Tokyo 103-8411 (JP); NAKAGAWA, Toshiya, Kyoto-shi Kyoto 602-0838 (JP)
(74) Representative: Gille Hrabal Struck Neidlein Prop Roos
(86) International application number: PCT/JP2008/069486
(87) International publication number: WO 2009/057568

(57) **Abstract**

[Problems] To provide an antibacterial agent, particularly a novel polypeptide compound having an antifungal action.

[Means for Solution] As a result of an extensive study on a novel antifungal agent, the inventors have found that an excellent anti-deep-fungal activity is exhibited by introducing a cyclohexylmethylamino group or a piperidylmethylamino group as a partial structure of a natural polypeptide compound, and completed the present invetion.

The compound of the present invetion has an excellent antibacterial activity and is therefore useful as an agent for preventing or treating various fungal infections.

## Description

### Technical Field

The present invention relates to a pharmaceutical, particularly a novel polypeptide compound having an antifungal activity, or a pharmaceutically acceptable salt thereof.

### Background Art

Infections caused by fungi are divided into superficial cutaneous fungal diseases (Trichophyton, and the like) caused by skin infection, and deep mycosis (Candida and Aspergillus, and the like) caused by respiratory or oral infection. Deep mycosis has developed in patients who have various risk factors such as immune deficit or anticancer drug administration, and it has been frequently found that once it develops, it becomes refractory or a poor prognosis. Particularly, when invasive pulmonary aspergillosis develops, the mortality rate thereof is 58% of the total number of diseases, in particular, 87% in bone-marrow transplant patients. However, there are few effective therapeutic approaches or drugs and the rate of efficacy of voriconazole or AmBisome which are standard drugs is low at 60% or less. Therefore, a novel antifungal agent having potent activities is required.

Several polypeptide compounds having antifungal activities have been known (for example, Patent Documents 1-7), and Patent Document 1 discloses the following compound. 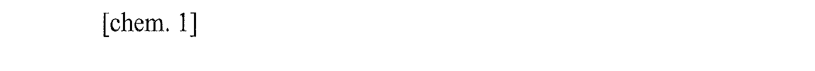 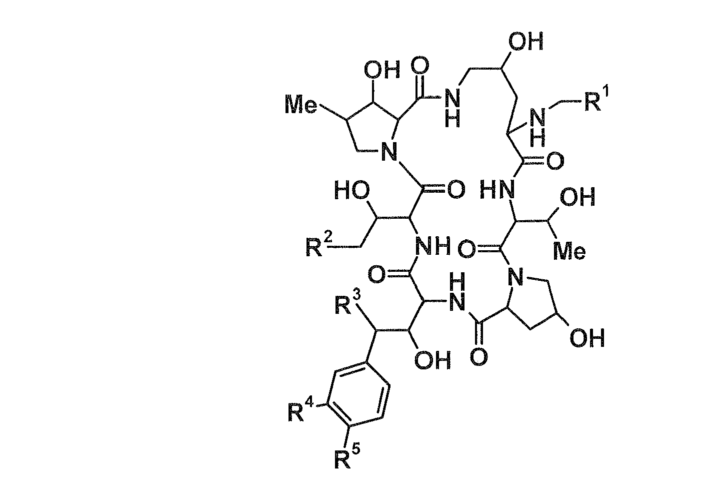 (In the formula, R¹ represents aryl which may be substituted.)

Patent Document 1; Pamphlet of International Publication WO2004/113368
Patent Document 2; Pamphlet of International Publication WO01/60846
Patent Document 3; Pamphlet of International Publication WO00/64927
Patent Document 4; Pamphlet of International Publication WO99/40108
Patent Document 5; Pamphlet of International Publication WO03/068807
Patent Document 6; US Patent US5569646
Patent Document 7; Pamphlet of International Publication WO2005/005463

### Disclosure of Invention

### Problem that the Invention is to Solve

An object of the present invention is to provide a novel compound having an antibacterial activity (particularly an antifungal activity).

### Means for Solving the Problem

As a result of an extensive study on a novel polypeptide compound having an antifungal activity, the inventors have found that the compound of the formula (I), wherein a specific ring group is bonded through a carbon atom to a nitrogen atom of a side chain linking portion at the 9-positon of a polypeptide ring, exhibits an excellent antifungal activity, and completed the present invention.
That is, the present invention relates to the compound of the following formula (I) or a pharmaceutically acceptable salt thereof. 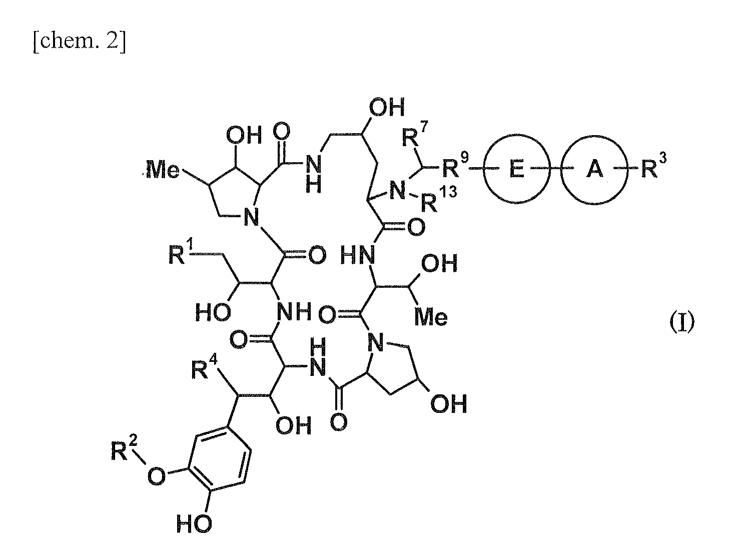 (wherein the symbols in the formula have the following meanings,
R¹ represents -CO-NR⁵R⁶, -R⁰⁰-NR⁵R⁶, -CO-(nitrogen-containing hetero ring) or -NH-R⁰⁰-NR⁵R⁶,
R⁵ and R⁶ are the same as or different from each other, and represent H, R⁰, R^{A} or R^{B},
R^{A} represents lower alkyl which may be substituted with one to three hydroxyl groups,
R^{B} represents lower alkyl which may be substituted with one to three groups selected from -NH₂, -NHR⁰ and -N(R⁰)₂,
R² represents H, -S(O)₂-OH, R⁰, R^{A}, R^{B} or -R⁰⁰-NH-R⁰⁰-OH,
R⁴ represents H or -OH,
R⁷ is the same as or different from each other, and represents H, R⁰ or -R⁰⁰-OR⁰, R⁹ represents a single bond,
R¹³ and R¹⁴ are the same as or different from each other, and represent H or R⁰,
E ring represents a group represented by the following formulae (II), (III), (IV), (V) or (XVIII), 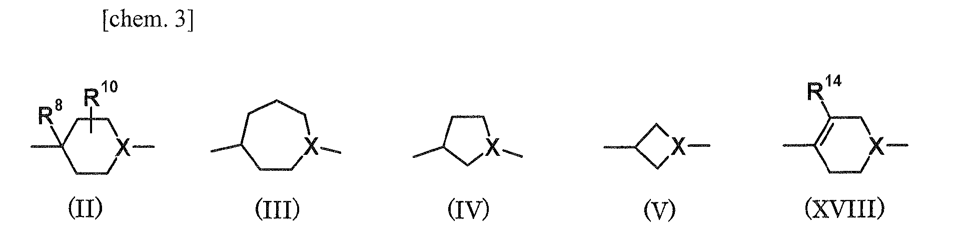 wherein X represents CH or N,
R⁸ is the same as or different from each other, and represents H, R⁰, -NH₂, -N(R⁰)₂, -NHR⁰, -OH or -R⁰⁰-OR⁰,
R¹⁰ represents H, R⁰, -NH₂, -N(R⁰)₂, -NHR°, -OH or -R⁰⁰-OR⁰,
A ring represents a nitrogen-containing hetero ring or phenyl,
R³ represents a group represented by the following formulae (VI),(VII),(VIII) or
(XIX), 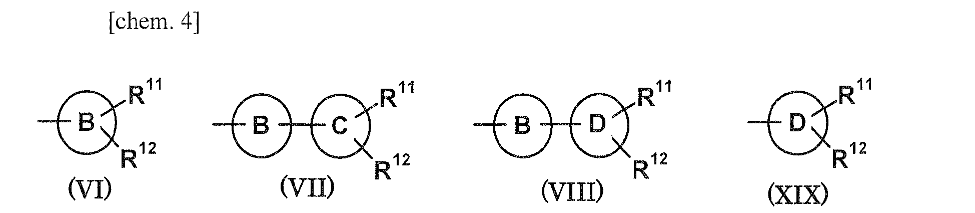 wherein B ring and D ring are the same as or different from each other, and represent aryl which may be substituted with halogen,
C ring represents a nitrogen-containing hetero ring,
R¹¹ and R¹² are the same as or different from each other, and represent a group selected from H, R⁰, cycloalkyl, -(cycloalkyl)-R⁰, -R⁰⁰-(cycloalkyl), -OR⁰, -O-(cycloalkyl), -O-(C₂-₁₀ alkylene)-OR°, -O-R⁰⁰-(cycloalkyl), -(nitrogen-containing hetero ring)-R⁰⁰-(cycloalkyl), -(nitrogen-containing hetero ring)-O-(cycloalkyl) and -(nitrogen-containing hetero ring)-O-R⁰⁰-(cycloalkyl),
R⁰ represents lower alkyl, and
R⁰⁰ represents lower alkylene.)

The present application further relates to a pharmaceutical composition comprising the compound of the formula (I) or a pharmaceutically acceptable salt thereof and a pharmaceutically acceptable excipient, and a pharmaceutical composition for preventing or treating a fungal infection comprising the compound of the formula (I) or a pharmaceutically acceptable salt thereof. Further, the present application relates to a method for preventing and/or treating a fungal infection in human or an animal (in particular, a mammal).

Further, the present application relates to use of the compound of the formula (I) or a pharmaceutically acceptable salt thereof for the manufacture of an agent for preventing or treating a fungal infection, and a method for preventing or treating a fungal infection comprising administering an effective amount of the compound of the formula (I) or a pharmaceutically acceptable salt thereof to a patient.

### Effect of the Invention

The compound of the formula (I) has an antifungal activity, and therefore is useful as an agent for preventing and/or treating fungal infections.

### Best Mode for Carrying Out the Invention

The invention is described in detail below.
In the present specification, the "lower alkyl" is linear or branched alkyl having from 1 to 6 carbon atoms (hereinafter, abbreviated to as C₁₋₆), specifically, methyl, ethyl, n-propyl, isopropyl, n-butyl, isobutyl, sec-butyl, tert-butyl, n-pentyl, n-hexyl groups, or the like. In an embodiment, it is C₁₋₃ alkyl, and in another embodiment is methyl, ethyl or isopropyl.
The "lower alkylene" means a divalent group (C₁₋₆ alkylene) formed by removing an arbitrary hydrogen atom from the "lower alkyl" above. In an embodiment, it is C₁₋₄ alkylene, and in another embodiment is methylene or ethylene.
The "C₂₋₁₀ alkylene" represents linear or branched alkylene having 2 to 10 carbon atoms. In an embodiment, it is C₅₋₆ alkylene, and in another embodiment is pentamethylene or hexamethylene.
The "cycloalkyl" means C₃₋₈ cyclic alkyl, which may be bridged. In an embodiment, it is C₃₋₆ cyclic alkyl, and in another embodiment is cyclopropyl, cyclobutyl, cyclopentyl or cyclohexyl.
The "halogen" means F, Cl, Br, or 1.

The "aryl" is a C₆₋₁₄ monocyclic to tricyclic aromatic hydrocarbon ring group, and includes partially hydrogenated ring group thereof. Specifically, it is phenyl, naphthyl, 5-tetrahydronaphthyl, 1-indanyl groups, or the like. In an embodiment it is phenyl and naphthyl, and in another embodiment is phenyl.

The "hetero ring" group means a ring group including i) monocyclic 3- to 8-membered, preferably 5- to 7-membered hetero ring, containing 1 to 4 hetero atoms selected from oxygen, sulfur and nitrogen, and, ii) a bicyclic or tricyclic hetero ring containing 1 to 5 hetero atoms selected from oxygen, sulfur and nitrogen, where the monocyclic hetero ring is condensed with one or two rings selected from the group consisting of a monocyclic hetero ring, benzene ring, C₅₋₈ cycloalkane and C₅₋₈ cycloalkene. The ring atom sulfur or nitrogen may be oxidized to form oxide or dioxide.
Preferred examples of the "hetero ring" group are the following groups.
(1) Monocyclic saturated hetero ring group
   i) containing 1 to 4 nitrogen atoms, specifically, azepanyl, diazepanyl, aziridinyl, azetidinyl, pyrrolidinyl, imidazolidinyl, piperidinyl, pyrazolidinyl, piperazinyl groups, or the like;
   ii) containing 1 to 3 nitrogen atoms, and 1 to 2 sulfur atoms and/or 1 to 2 oxygen atoms, specifically, thiomorpholinyl, thiazolidinyl, isothiazolidinyl, oxazolidinyl, morpholinyl groups, or the like;
   iii) containing 1- to 2 sulfur atoms, specifically, tetrahydrothinyl groups, or the like;
   iv) containing 1 to 2 sulfur atoms and 1 to 2 oxygen atoms, specifically, oxathiolane groups, or the like;
   v) containing 1 to 2 oxygen atoms, specifically, oxiranyl, dioxolanyl, oxolanyl, tetrahydropyranyl, 1, 4-dioxanyl groups, or the like;
(2) Monocyclic unsaturated hetero ring group
   i) containing 1 to 4 nitrogen atoms, specifically, pyrrolyl, imidazolyl, pyrazolyl, pyridyl, dihydropyridyl, pyrimidinyl, pyrazinyl, pyridazinyl, triazolyl, tetrazolyl, dihydrotriazinyl, azepinyl groups, or the like;
   ii) containing 1 to 3 nitrogen atoms, and 1 to 2 sulfur atoms and/or 1 to 2 oxygen atoms, specifically, thiazolyl, isothiazolyl, thiadiazolyl, dihydrothiazinyl, oxazolyl, isoxazolyl, oxadiazolyl, oxazinyl groups, or the like;
   iii) containing 1 to 2 sulfur atoms, specifically, thienyl, thiepinyl, dihydrodithiinyl, dihydrodithionyl groups, or the like;
   iv) containing 1 to 2 sulfur atoms and 1 to 2 oxygen atoms, specifically, dihydrooxathiinyl groups, or the like;
   v) containing 1 to 2 oxygen atoms, specifically, furyl, pyranyl, oxepinyl, dioxolyl groups, or the like;
(3) Condensed polycyclic saturated hetero ring groups
   i) containing 1 to 5 nitrogen atoms, specifically, quinuclidine, 7-azabicyclo[2.2.1]heptyl, 3-azabicyclo[3.2.2]nonanyl groups, or the like,
   ii) containing 1 to 4 nitrogen atoms, and 1 to 3 sulfur atoms and/or 1 to 3 oxygen atoms, specifically, trithiadiazaindenyl dioxoloimidazolidinyl groups, or the like;
   iii) containing 1 to 3 sulfur atoms and/or 1 to 3 oxygen atoms, specifically, 2, 6-dioxabicyclo[3.2.2]oct-7-yl groups, or the like;
(4) Condensed polycyclic unsaturated hetero ring groups
   i) containing 1 to 5 nitrogen atoms, specifically, indolyl, isoindolyl, indolinyl, indolidinyl, benzimidazolyl, quinolyl, tetrahydroquinolyl, isoquinolyl, tetrahydroisoquinolyl, indazolyl, imidazopyridyl, benzotriazolyl, tetrazolopyridazinyl, carbazolyl, quinoxalinyl, dihydroindazolyl, benzopyrimidinyl, naphthylidinyl, quinazolinyl, cinnolinyl groups, or the like;
   ii) containing 1 to 4 nitrogen atoms, and 1 to 3 sulfur atoms and/or 1 to 3 oxygen atoms, specifically, benzothiazolyl, dihydrobenzothiazolyl, benzothiadiazolyl, imidazothiazolyl, imidazothiadiazolyl, benzoxazolyl, benzoxadiazolyl groups, or the like;
   iii) containing 1 to 3 sulfur atoms, specifically, benzothienyl, benzodithiinyl groups, or the like;
   iv) containing 1 to 3 sulfur atoms and 1 to 3 oxygen atoms, specifically, benzoxathiinyl, phenoxazinyl groups, or the like;
   v) containing 1 to 3 oxygen atoms, specifically, benzodioxolyl, benzofuranyl, isobenzofuranyl, chromenyl, benzodihydrofuranyl groups, or the like.

These hetero rings are exemplified as monovalent groups, but may be divalent or more groups according to bonding position or the number of substituents.

The "nitrogen-containing hetero ring" means those containing one or plural nitrogen atom(s) as atoms constituting the ring among the "hetero ring" above and, for example, includes pyridyl, pyrimidinyl, imidazothiazolyl, thiadiazolyl, piperidinyl, piperazinyl, and the like.
The "monocyclic nitrogen-containing hetero ring" means a monocyclic ring among the "nitrogen-containing hetero ring" above and includes for example, pyridyl, pyrimidinyl, thiadiazolyl, piperidyl, and the like.

The "may be substituted" represents "unsubsitituted" or "having 1 to 5 substituents which are the same or different". Further, when plural substituents are contained, these substituents may be the same as or different from each other. For example, two R⁰ in -N(R⁰)₂ may be different from each other.

Embodiments of the invention are shown below.
(1) In an embodiment, R¹ is -CO-NR⁵R⁶ or -R⁰⁰-NR⁵R⁶, in another embodiment is -CO-NH₂, -CH₂-NH₂, -CH₂-NHR^{A}or -CH₂-NHR^{B}, in further embodiment is -CO-NH₂, -CO-NH-R⁰⁰-NH₂, -CH₂-NH₂ or -CH₂-NH-CH(CH₂OH)₂, and in yet another embodiment is -CO-NH₂, -CH₂-NH₂ or -CH₂-NH-CH(CH₂OH)₂.
(2) In an embodiment, R² is -S(O)₂-OH or R⁰, and in another embodiment is -S(O)₂-OH or methyl.
(3) In an embodiment, R⁴ is H.
(4) In an embodiment, R⁷ is H or R⁰, in another embodiment is H or methyl, and in further embodiment is H.
(5) In an embodiment, E ring is a group represented by the following formula (II).

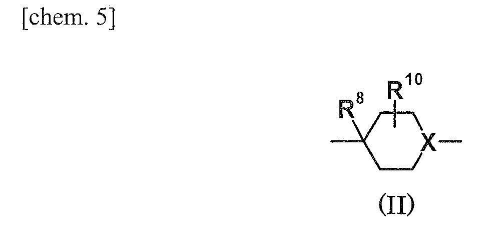

In the formula (II), in an embodiment, R⁸ is H, R⁰, -OH or -OR⁰, R¹⁰ is H, and in another embodiment, R⁸ is H or R⁰, R¹⁰ is H, in further embodiment, R⁸ is H or methyl, and R¹⁰ is H. Further, in an embodiment, E ring is cyclohexanediyl, and in another embodiment is piperidine-1, 4-diyl.
(6) In an embodiment, X is CH, and in another embodiment is N.
(7) In an embodiment, A ring is monocyclic nitrogen-containing hetero ring, in another embodiment is pyridyl, pyrimidinyl, thiadiazolyl or imidazothiazolyl, and in further embodiment is thiadiazolyl or imidazothiazolyl.
(8) In an embodiment, R³ is a group represented by the following formulae (VII) or (VIII), 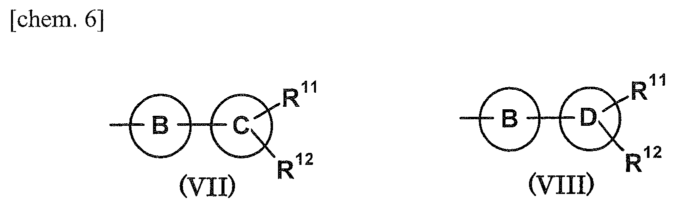 in another embodiment is the group represented by the formula (VII), and in further embodiment is the group represented by the formula (VIII).
(9) B ring and D ring are the same as or different from each other, in an embodiment are monocyclic aryl which may be substituted with halogen, in another embodiment are monocyclic aryl, and in further embodiment are phenyl.
(10) In an embodiment, C ring is a monocyclic nitrogen-containing hetero ring, in another embodiment is pyridyl, pyrimidinyl or piperidinyl, in further embodiment is pyrimidinyl or piperidinyl, and in yet another embodiment, is, together with R¹¹ and R¹², a group represented by the following formulae (XX) or (XXI). 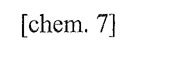 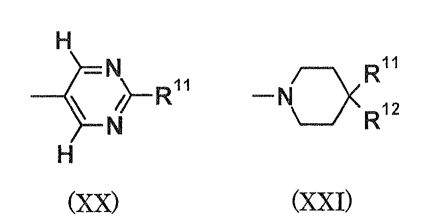
(11) In an embodiment, either of R¹¹ and R¹² is H or -OR⁰ and the other is a group selected from cycloalkyl, -R⁰⁰-(cycloalkyl), -O-(cycloalkyl), -O-R⁰⁰-(cycloalkyl), -(nitrogen-containing hetero ring)-R⁰⁰-(cycloalkyl), -(nitrogen-containing hetero ring)-O-(cycloalkyl) and -(nitrogen-containing hetero ring)-O-R⁰⁰-(cycloalkyl), in another embodiment either of R¹¹ and R¹² is H or -OR⁰ and the other is a group selected from cycloalkyl, -O-(cycloalkyl), -O-R⁰⁰-(cycloalkyl) and -(nitrogen-containing hetero ring)-O-(cycloalkyl), and in further embodiment either of R¹¹ and R¹² is H or -O-(methyl) and the other is a group selected from cyclohexyl, -O-(cyclohexyl), -O-CH₂-(cyclobutyl) and 4-(cyclohexyloxy)piperidin-1-yl. Further, in another embodiment, R¹¹ and R¹² are the same as or different from each other, and are each H, cycloalkyl, -O-R⁰, -O-(cycloalkyl), -(nitrogen-containing hetero ring)-O-(cycloalkyl) or -(nitrogen-containing hetero ring)-O-R⁰⁰-(cycloalkyl).

Another preferable embodiment is the combination of any of two or more groups described in the above (1) to (11), specifically include the compounds shown in the following (12) to (16).
(12) A compound wherein R¹ is -CO-NR⁵R⁶ or -R⁰⁰-NR⁵R⁶, R² is -S(O)₂-OH or R⁰, R⁴ is H, R⁷ is H or R⁰, R⁹ is a single bond, E ring is a group represented by the formula (II), A ring is monocyclic nitrogen-containing hetero ring, R⁸ is H or R⁰, R³ is a group represented by the formula (VII) or (VIII), B ring and D ring are the same as or different from each other and are each monocyclic aryl, C ring is monocyclic nitrogen-containing hetero ring, R¹¹ and R¹² are the same as or different from each other and are each H, cycloalkyl, -O-R⁰, -O-(cycloalkyl), -(nitrogen-containing hetero ring)-O-(cycloalkyl) or -(nitrogen-containing hetero ring)-O-R⁰⁰-(cycloalkyl).
(13) A compound wherein R¹ is -CO-NR⁵R⁶ or -R⁰⁰-NR⁵R⁶, R² is -S(O)₂-OH or R⁰, R⁴ is H, R⁷ is H or R⁰, E ring is a group represented by the formula (II), R⁸ is H or R¹⁰, R¹⁰ is H, A ring is monocyclic nitrogen-containing hetero ring, R³ is a group represented by the formula (VII) or (VIII), B ring and D ring are the same as or different from each other and are each monocyclic aryl which may be substituted with halogen, C ring is monocyclic nitrogen-containing hetero ring, either of R¹¹ and R¹² is H or -OR⁰ and the other is a group selected from cycloalkyl, -R⁰⁰-(cycloalkyl), -O-(cycloalkyl), -O-R⁰⁰-(cycloalkyl), -(nitrogen-containing hetero ring)-R⁰⁰-(cycloalkyl), -(nitrogen-containing hetero ring)-O-(cycloalkyl) and -(nitrogen-containing hetero ring)-O-R⁰⁰-(cycloalkyl).
(14) A compound wherein R¹ is -CO-NH₂, -CH₂-NH₂, -CH₂-NHR^{A} or -CH₂-NHR^{B}, R² is -S(O)₂-OH or methyl, R⁴ is H, R⁷ is H or methyl, E ring is a group represented by the formula (II), X is CH, R⁸ is H or methyl, R¹⁰ is H, A ring is pyridyl, pyrimidinyl, thiadiazolyl or imidazothiazolyl, R³ is a group represented by the formula (VII), B rign and D ring are monocyclic aryl, C ring is pyridyl, pyrimidinyl or piperidinyl, either of R¹¹ and R¹² is H or -OR⁰ and the other is a group selected from cycloalkyl, -O-(cycloalkyl), -O-R⁰⁰-(cycloalkyl) and -(nitrogen-containing hetero ring)-O-(cycloalkyl).
(15) A compound wherein R¹ is -CO-NH₂, -CH₂-NH₂ or -CH₂-NH-CH(CH₂OH)₂, R² is -S(O)₂-OH or methyl, R⁴ is H, R⁷ is H, E ring is cyclohexanediyl, A ring is thiadiazolyl or imidazothiazolyl, R³ is a group represented by the formula (VII), B ring and D ring are phenyl, C ring is, together with R¹¹ and R¹², a group represented by the formula (XX) or (XXI), either of R¹¹ and R¹² is H or -O-(methyl) and the other is a group selected from cyclohexyl, -O-(cyclohexyl), -O-CH₂-(cyclobutyl), and 4-(cyclohexyloxy)piperidin-1-yl.
(16) A compound selected from the following.
(3R)-3-{(2R, 6S, 11R, 14aS, 15S, 16S, 20S, 23S, 25aS)-9-[({trans-4-[5-(4-{2-[4-(cyclopentylmethyl)piperazin-1-yl]pyrimidin-5-yl}phenyl)-1, 3, 4-thiadiazol-2-yl]cyclohexyl}methyl)amino]-2, 11, 15-trihydroxy-6-[(1R)-1-hydroxyethyl]-23-[(1R)-1-hydroxy-2-(4-hydroxy-3-methoxyphenyl)ethyl]-16-methyl-5, 8, 14, 19, 22, 25-hexaoxotetracosahydro-1H-dipyrrolo[2, 1-c:2', 1'-1][1, 4, 7, 10, 13, 16]hexaazacyclohenicosin-20-yl}-3-hydroxypropanamide dihydrochloride, (2R, 6S, 9S, 11R, 14aS, 15S, 16S, 20S, 23S, 25aS)-9-{[(trans-4-{5-[4-(4-cyclohexyl-4-methoxypiperidin-1-yl)phenyl]-1, 3, 4-thiadiazol-2-yl}cyclohexyl)methyl]amino}-2, 11, 15-trihydroxy-6-[(1R)-1-hydroxyethyl]-20-[(1R)-1-hydroxy-3-{[2-hydroxy-1-(hydroxymethyl)ethyl] amino }propyl]-23-[(1R)-1-hydroxy-2-(4-hydroxy-3-methoxyphenyl)ethyl]-16-methylhexadecahydro-1H-dipyrrolo[2, 1-c:2', 1'-1][1, 4, 7, 10, 13, 16]hexaazacyclohenicosine-5, 8, 14, 19, 22, 25(9H, 25aH)-hexone dihydrochloride, 2-hydroxy-5-{(2R)-2-hydroxy-2-[(2R, 11R, 14aS, 15S, 16S, 20S, 23S, 25aS)-2, 11, 15-trihydroxy-6-[(1R)-1-hydroxyethyl]-20-[(1R)-1-hydroxy-3- [2-hydroxy-1-(hydroxymethyl)ethyl] amino}propyl]-9-({[trans-4-(2-{4-[(6-methoxyhexyl)oxy]phenyl}imidazo[2, 1-b][1, 3, 4]thiadiazol-6-yl)cyclohexyl]methyl}amino)-16-methyl-5, 8, 14, 19, 22, 25-hexaoxotetracosahydro-1H-dipyrrolo[2, 1-c:2', 1'-1][1, 4, 7, 10, 13, 16]hexaazacyclohenicosin-23-yl]ethyl}phenyl hydrogen sulfate, (2R, 6S, 11R, 14aS, 15S, 16S, 20S, 23S, 25aS)-20-[(1R)-3-amino-1-hydroxypropyl]-9-{[(trans-4-{5-[4-(4-cyclohexyl-4-methoxypiperidin-1-yl)phenyl]-1,3, 4-thiadiazol-2-yl}cyclohexyl)methyl]amino}-2, 11, 15-trihydroxy-6-[(1R)-1-hydroxyethyl]-23-[(1R)-1-hydroxy-2-(4-hydroxy-3-methoxyphenyl)ethyl]-16-methylhexadecahydro-IH-dipyrrolo[2, 1-c:2', 1'-1] [1, 4, 7, 10, 13, 16]hexaazacyclohenicosine-5, 8, 14, 19, 22, 25(9H, 25aH)-hexone dihydrochloride, 5-[(2R)-2-{(2R, 6S, 9S, 11R, 14aS, 15S, 16S, 20S, 23S, 25aS)-20-[(1R)-3-amino-1-hydroxy-3-oxopropyl]-9-[({trans-4-[5-(4-{2-[4-(cyclohexyloxy)piperidin-1-yl]pyrimidin-5-yl}phenyl)-1, 3, 4-thiadiazol-2-yl]cyclohexyl}methyl)amino]-2, 11, 15-trihydroxy-6-[(1R)-1-hydroxyethyl]-16-methyl-5, 8, 14, 19, 22, 25-hexaoxotetracosahydro-1H-dipyrrolo[2, 1-c:2', 1'-1][1, 4, 7, 10, 13, 16]hexaazacyclohenicosin-23-yl}-2-hydroxyethyl]-2-hydroxyphenyl hydrogen sulfate, 5-[(2R)-2-{(2R, 6S, 9S, 11R, 14aS, 15S, 16S, 20S, 23S, 25aS)-20-{(1R)-3-[(2-aminoethyl)amino]-1-hydroxy-3-oxopropyl}-9-[({1-[5-(4-{2-[4-(cyclohexyloxy)piperidin-1-yl]pyrimidin-5-yl}phenyl)-1, 3, 4-thiadiazol-2-yl]piperidin-4-yl}methyl)amino]-2, 11, 15-trihydroxy-6-[(1R)-1-hydroxyethyl]-16-methyl-5, 8, 14, 19, 22, 25-hexaoxotetracosahydro-1H-dipyrrolo[2, 1-c:2', 1'-1][1, 4, 7, 10, 13, 16]hexaazacyclohenicosin-23-yl}-2-hydroxyethyl]-2-hydroxyphenyl hydrogen sulfate, and 5-[(2R)-2-{(2R, 6S, 11R, 14a, 15S, 16S, 20S, 23S, 25aS)-9-({[trans-4-(5-{4-[4-(cyclohexyloxy)piperidin-1-yl]phenyl}-1, 3, 4-thiadiazol-2-yl)cyclohexyl]methyl}amino)-2, 11, 15-trihydroxy-6-[(1R)-1-hydroxyethyl]-20-[(1R)-1-hydroxy-3-{[2-hydroxy-1-(hydroxymethyl)ethyl]amino}propyl]-16-methyl-5, 8, 14, 19, 22, 25-hexaoxotetracosahydro-1H-dipyrrolo[2, 1-c:2', 1'-1][1, 4, 7, 10, 13, 16]hexaazacyclohenicosin-23-yl-2-hydroxyethyl]-2-hydroxyphenyl hydrogen sulfate.

The compound of the formula (I) may in some cases exist in the form of other tautomers or geometrical isomers, depending on the kinds of the substituents. In the present specification, the compound may be described in only one form of the isomers, but the present invention includes such isomers, isolated forms of the isomers, or a mixture thereof.
Furthermore, the compound of the formula (I) may have asymmetric carbon atoms or axial asymmetries in some cases, and correspondingly, it may exist in the form of optical isomers such as an (R)-form, an (S)-form, and the like. The present invention includes a mixture and an isolated form of these optical isomers.
In addition, pharmaceutically acceptable prodrugs of the compound of the formula (I) are also included in the present invention. The pharmaceutically acceptable prodrug refers to a compound having a group which can be converted into an amino group, -OH, -CO₂H, or the like, by solvolysis or under a physiological condition. Examples of the group for forming a prodrug include the groups as described in Prog. Med., 5, 2157-2161 (1985) or "Pharmaceutical Research and Development" (Hirokawa Publishing Company, 1990), vol. 7, Drug Design, 163-198.

Furthermore, the compound of the formula (I) may form an acid addition salt or a salt with a base, depending on the kind of the substituents, and the salt is included in the present invention, as long as it is a pharmaceutically acceptable salt. Specifically, examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, glutamic acid, and the like, and salts with inorganic bases such as sodium, potassium, magnesium, calcium, aluminum, and the like, and organic bases such as methylamine, ethylamine, ethanolamine, lysine, ornithine, and the like, ammonium salts, and others.
Furthermore, the present invention also includes various hydrates or solvates, and polymorphism of the compound of the formula (I) and a pharmaceutically acceptable salt thereof. Furthermore, the present invention also includes the compound of the formula (I) labeled with various radioactive isotopes or non-radioactive isotopes.

### (Production Processes)

The compound of the formula (I) and a pharmaceutically acceptable salt thereof can be prepared by applying various known synthesis methods, using the characteristics based on their basic skeletons or the kinds of the substituents. At this time, depending on the types of the functional groups, it is in some cases effective from the viewpoint of the preparation techniques to substitute the functional group with an appropriate protecting group (a group which is capable of being easily converted into the functional group), during the steps from starting materials to intermediates. Examples of such a functional group include an amino group, a hydroxyl group, a carboxyl group, and the like, and examples of the protecting group thereof include the protecting groups as described in "Protective Groups in Organic Synthesis (4^{th} edition, 2007)", edited by Greene and Wuts, and the like, which may be appropriately selected and used depending on the reaction conditions. In these methods, a desired compound can be obtained by introducing the protecting group to carry out the reaction, and then, if desired, removing the protecting group.
In addition, the prodrug of the compound of the formula (I) can be prepared by introducing a specific group during the steps from starting materials to intermediates, in the same manner as for the above protecting groups, or by carrying out the reaction using the compound of the formula (I) obtained. The reaction can be carried out by applying a method known by a person skilled in the art, such as general esterification, amidation, dehydration, and the like.
Hereinbelow, the representative production processes for the compound of the formula (I) will be explained. Each of the production processes may also be carried out with reference to the References appended in the present description. Further, the production processes of the present invention are not limited to the examples as shown below.

### Production Process 1

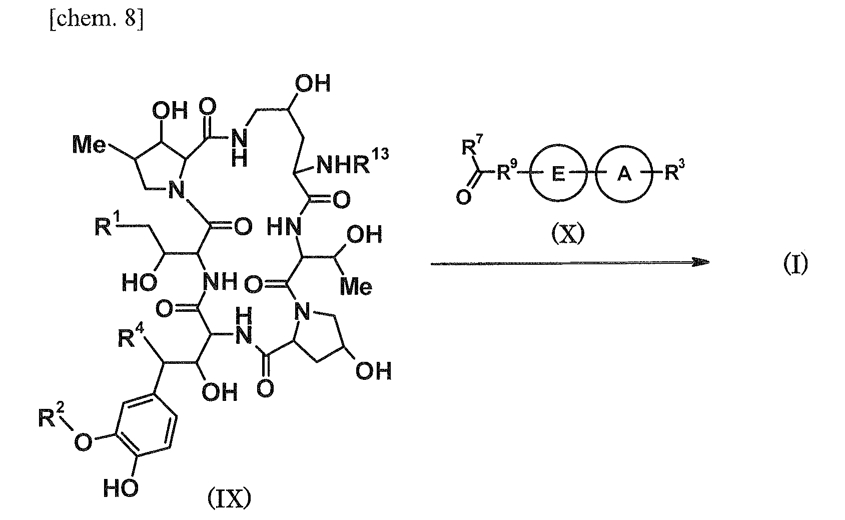

This production process is a method to obtain the compound of the formula (I) by the reductive amination of a compound (IX) and a compound (X).
The reaction is carried out using the compound (IX) and the compound (X) in equivalent amounts or either thereof in an exessive amount, in a solvent inert to the reaction under the presence of a reduction agent, at -45°C to heating under reflux, preferably at 0°C to room temperature, usually stirring for 0.1 hour to 5 days. The solvents are not particularly limited but, for example, include alcohols such as methanol and ethanol, ethers such as diethylether, tetrahydrofuran (THF), dioxane, and dimethoxyethane (DME), or mixtures thereof. The reduction agents include sodium cyanoborohydride, sodium triacetoxyborohydride, sodium borohydride, and the like. In some cases, it is preferable to carry out the reaction under dehydrating agents such as molecular sieves, or under the presence of acids such as acetic acid, hydrochloric acid, titanium (IV) isopropoxide complex. Depends on the reaction, in case the imine form which is generated as an intermediate in a reaction system can be isolated stably, the reduction reaction may be caried out separately after the imine form is obtained. Further, it can be carried out using a reduction catalyst (for example, palladium on carbon, Raney nickel, and the like) under the presence or absence of an acid such as acetic acid and hydrochloric acid in a solvent such as methanol, ethanol and ethyl acetate, instead of treatment by the reduction agent. In this case, the reaction is preferably carried out under a hydrogen atmosphere of from normal pressure to 50 atm, at 0°C to under heating. (Reference: "Comprehensive Organic Functional Group Transformations II", vol.2, Elsevier Pergamon, 2005, edited by A. R. Katritzky and R. J. K. Taylor; "Jikken Kagaku Koza (Courses in Experimental Chemistry) (5th edition)", edited by The Chemical Society of Japan, vol. 14(2005) (Maruzen)).
Further, in the case that a protecting group is required in R¹ of the compound (IX) when the reaction is carried out, a suitable protecting group is introduced in advance, and the protecting group can be removed after the reaction.

### (Starting Material Synthesis)

### Starting Material Production Process 1:

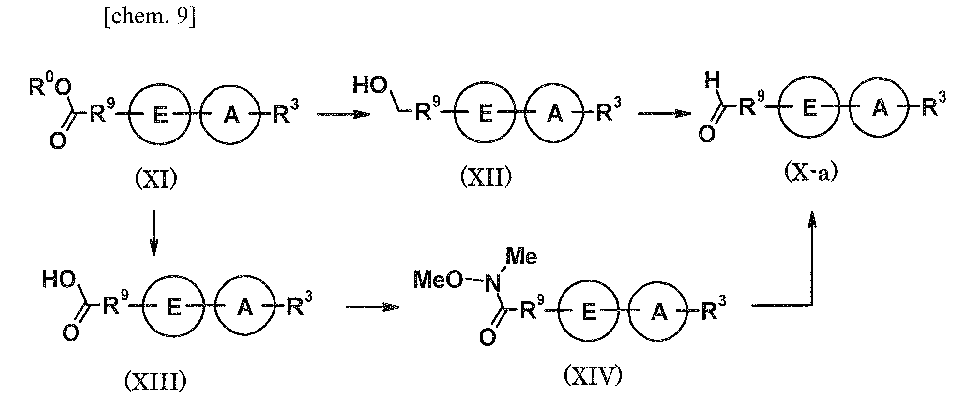

A compound (X-a) can be produced by subjecting a compound (XI) to reduction to alcohol to form a compound (XII), followed by oxidation to aldehyde. Alternatively, it can be produced by using carboxylic acid (XIII) and Weinreb amide (XIV).

### Starting Material Production Process 2:

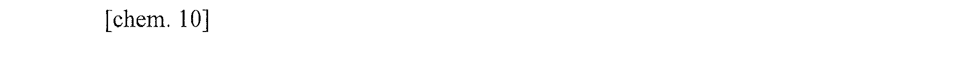

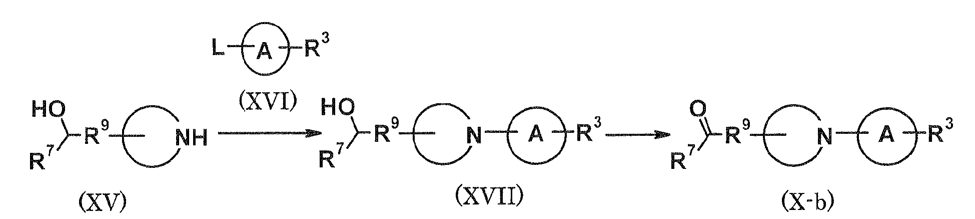

(In the formula, L represents a leaving group, for example, halogen. The same shall apply hereinafter.)
A compound (X-b) can be produced by oxidizing a compound (XVII) which is obtained by the nucleophilic substitution of a compound (XV) and a compound (XVI).

The starting compounds used in producing the Example compounds of the present application, which are represented by the general formula (IX), can be produced in the same manner as described in WO2005/005463, WO03/068807, WO00/06492 WO99/040108 and EP0431350.

The compound of the formula (I) is isolated and purified as free compound, pharmaceutically acceptable salts, hydrates, solvates, or polymorphism thereof. The pharmaceutically acceptable salt of the compound of the formula (I) can also be prepared in accordance with a conventional method for a salt formation reaction.
Isolation and purification are carried out by employing general chemical operations such as extraction, fractional crystallization, various types of fractional chromatography, and the like.
Various isomers can be separated by selecting an appropriate starting compound or by making use of the difference in the physicochemical properties between isomers. For example, the optical isomer can be lead into a stereochemically pure isomer by means of general optical resolution methods (for example, fractional crystallization for inducing diastereomer salts with optically active bases or acids, chromatography using a chiral column, etc., and the like). In addition, the isomers can also be prepared from an appropriate optically active starting compound.

The pharmacological activity of the compound of the formula (I) was confirmed by the following Test Example 1 and Test Example 2.

### Test Example 1: Measurement of susceptibility to C. albicans (Candida albicans) and A. fumigatus (Aspergillus fumigatus)

By the microdilution method where human serum buffered with 20mM-HEPES buffer (pH 7.3) was used as a test medium, MIC (minimum inhibitory concentration) in human serum was determined. The inoculum suspension of 10⁶ cells/mL was prepared by a hemocytometric procedure to obtain an inoculum size of approximately 5x10³ to 2x10⁴ cells/mL. Microplates were incubated at 37°C for 24 hours under 5% CO₂ atmosphere. The MICs to C. albicans were defined as the lowest concentrations at which growth was completely inhibited, and the MICs to A. fumigatus were defined as the lowest concentration at which growth was sufficiently inhibited in comparison with growth control. The values were shown as geometric mean of MIC with reference to three of clinical cell lines.

### Test Example 2: Effect to mouse respiratory infection model

4-week old male ICR mice were used so as to be eight mice in a group. 200 mg/kg of cyclophosphamide is intraperitoneally administered into mice at 4 days before infection and 1 day after infection. Conidium liquid (0.05 mL: approximately 2.2x10⁶) of A. fumigatus 20030 suspended in physiological saline was inoculated from respiratory passages of mice to cause respiratory infection. The compound was administered intravenously for 2 hours to 4 days after the infection. The lives and deaths of the mice were observed over a 10 day period starting from the next day.

According to the Test Example 1 and Test Example 2, MIC values in human serum of the representative compound of the invention and ED₅₀ values to mouse respiratory infection model were measured respectively. Ex represents Example compound number and Ref means comparative compounds represented by Example 3 (product) of W02004/113368 (the same shall apply hereinafter).

**[Table 1]**

| Compound | MIC[µg/ml] (mean of three cell lines) | | Infection experiments ED₅₀ [mg/kg] |
|---|---|---|---|
| | C. albicans | A. fumigatus | A. fumigatus |
| Ref | 1 | 2 | 3 |
| Ex 1 | 0.25 | 0.25 | < 0.3 |
| Ex 4 | 0.63 | 0.16 | NT |
| Ex 6 | 0.5 | 0.32 | 0.44 |
| Ex 8 | 0.63 | 0.5 | NT |
| Ex 30 | 0.4 | 0.32 | NT |
| Ex 33 | 0.5 | 0.25 | 0.39 |

As a result of each of the above tests, it was shown that the compound of the formula (I) has an antibacterial activity (particularly antifungal activity), and it was confirmed that this effect is superior to conventional compounds represented by the comparative compounds. From this, the compound of the formula (I) can be used as an agent for treating various fungal infections, particularly infectious diseases by the following fungi.

### Acremonium;

Absidia (for example, Absidia corymbifera, and the like);
Aspergillus (for example, Aspergillus clavatus, Aspergillus flavus, Aspergillus fumigatus, Aspergillus nidulans, Aspergillus niger, Aspergillus terreus, Aspergillus versicolor, and the like);
Blastomyces (for example, Blastomyces dermatitidis, and the like);
Candida (for example, Candida albicans, Candida glabrata, Candida guilliermondii, Candida kefyr, Candida krusei, Candida parapsilosis, Candida stellatoidea, Candida tropicalis, Candida utilis, and the like);
Cladosporium (for example, Cladosporium tricoides, and the like);
Coccidioides (for example, Coccidioides immitis, and the like);
Cryptococcus (for example, Cryptococcus neoformans, and the like);
Cunninghamella (for example, Cunninghamella elegans, and the like);
Dermatophyte;
Exophiala (for example, Exophiala dermatitidis, Exophiala spinifera, and the like);
Epidermophyton (for example, Epidermophyton floccosum, and the like);
Fonsecaea (for example, Fonsecaea pedrosoi, and the like);
Fusarium (for example, Fusarium solani, and the like);
Geotrichum (for example, Geotrichum candidum, and the like);
Histoplasma (for example, Histoplasma capsulatum var. capsulatum, and the like);
Malassezia (for example, Malassezia furfur, and the like);
Microsporum (for example, Microsporum canis, Microsporum gypseum, and the like);
Mucor;
Paracoccidioides (for example, Paracoccidioides brasiliensis, and the like);
Penicillium (for example, Penicillium marneffei, and the like);
Phialophora;
Pneumocystis (for example, Pneumocystis jiroveci, and the like);
Pseudallescheria (for example, Pseudallescheria boydii, and the like);
Rhizopus (for example, Rhizopus microsporus var. rhizopodiformis, Rhizopus oryzae, and the like);
Saccharomyces (for example, Saccharomyces cerevisiae, and the like);
Scopulariopsis;
Sporothrix (for example, Sporothrix schenckii, and the like);
Trichophyton (for example, Trichophyton mentagrophytes, Trichophyton rubrum, and the like);
Trichosporon (for example, Trichosporon asahii, Trichosporon cutaneum, and the like).

The above fungi are well-known to cause various infections in skin, eyes, hair, nails, oral mucosa, gastrointestinal tract, bronchus, lungs, endocardium, brain, meninges, urinary organ, vaginal portion, oral cavity, ophthalmus, systemic, kidneys, heart, external auditory canal, bones, nasal cavity, paranasal cavity, spleen, liver, hypodermal tissue, lymph duct, gastrointestine, articulation, muscles, tendons, interstitial plasma cells in lungs, vessel, and the like.

Therefore, the compound of the formula (I) can be used for preventing and/or treating various infections, for example dermatophytosis (for example, trichophytosis, and the like), pityriasis versicolor, candidiasis, cryptococcosis, geotrichosis, piedra, aspergillosis, penicilliosis, fusariosis, zygomycosis, sporotrichosis, chromomycosis, coccidioidomycosis, histoplasmosis, blastomycosis, paracoccidioidomycosis, pseudallescheriasis, mycetoma, mycotic keratitis, otomycosis, Pneumocystis pneumonia, fungemia, and the like.

A preparation comprising one or two or more kinds of the compound of the formula (I) or a pharmaceutically acceptable salt thereof as an active ingredient can be prepared in accordance with a generally used method, using a pharmaceutical carrier, excipient, or the like, that is usually used in the art.

The administration can be carried out in any mode of oral administration via tablets, pills, capsules, granules, powders, liquid preparations, or the like, or parenteral administration via injections such as intraarticular, intravenous, intramuscular, or others, suppositories, eye drops, eye ointments, percutaneous liquid preparations, ointments, percutaneous patches, transmucosal liquid preparations, transmucosal patches, inhalations, and the like.

Regarding the solid composition for oral administration according to the present invention, tablets, powders, granules, or the like are used. In such a solid composition, one or two or more kinds of active ingredients are mixed with at least one inert excipient, for example, lactose, mannitol, glucose, hydroxypropylcellulose, microcrystalline cellulose, starch, polyvinyl pyrrolidone, and/or magnesium aluminometasilicate, or the like. According to a conventional method, the composition may contain inert additives for example, a lubricant such as magnesium stearate, a disintegrator such as carboxymethylstarch sodium, a stabilizing agent, and a solubilizing aid. As occasion demands, the tablets or the pills may be coated with a sugar coating, or a film of a gastric or enteric material.

The liquid composition for oral administration includes pharmaceutically acceptable emulsions, soluble liquid preparations, suspensions, syrups, elixirs, or the like, and contains a generally used inert diluent such as purified water or ethanol. In addition to the inert diluent, this liquid composition may contain an adjuvant such as a solubilizing agent, a moistening agent, and a suspending agent, a sweetener, a flavor, an aroma, and an antiseptic.

Injections for parenteral administration contain sterile aqueous or non-aqueous soluble liquid preparations, suspensions or emulsions. The aqueous solvent includes, for example, distilled water for injection or physiological saline. Examples of the non-aqueous solvent include for example, propylene glycol, polyethylene glycol, plant oils such as olive oil, alcohols such as ethanol, Polysorbate 80 (Japanese Pharmacopeia), and the like. Such a composition may further contain a tonicity agent, an antiseptic, a moistening agent, an emulsifying agent, a dispersing agent, a stabilizing agent, or a solubilizing agent These are sterilized, for example, by filtration through a bacteria retaining filter, blending of a bactericide, or irradiation. In addition, these can also be used by preparing a sterile solid composition, and dissolving or suspending it in sterile water or a sterile solvent for injection prior to its use.

The agent for external use includes ointments, plasters, creams, jellies, cataplasms, sprays, lotions, eye drops, eye ointments, and the like. The agents contain generally used ointment bases, lotion bases, aqueous or non-aqueous liquid preparations, suspensions, emulsions, and the like. Examples of the ointment bases or the lotion bases include polyethylene glycol, propylene glycol, white vaseline, bleached bee wax, polyoxyethylene hydrogenated castor oil, glyceryl monostearate, stearyl alcohol, cetyl alcohol, lauromacrogol, sorbitan sesquioleate, and the like.
Regarding the transmucosal agents such as an inhalation, a transnasal agent, and the like, those in the form of a solid, liquid, or semi-solid state are used, and can be prepared in accordance with a conventionally known method. For example, a known excipient, and also a pH adjusting agent, an antiseptic, a surfactant, a lubricant, a stabilizing agent, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device, and the like. The dry powder inhaler or the like may be for single or multiple administration use, and a dry powder or a powder-containing capsule may be used. Alternatively, this may be in a form such as a pressurized aerosol spray which uses an appropriate propellant, for example, a suitable gas such as chlorofluoroalkane, hydrofluoroalkane, carbon dioxide, and the like, or other forms.

In particular, in case of the treatment and/or prevention of fungal infections, the aforementioned injections for parenteral administration or transmucosal agents such as an inhalation, a transnasal agent and the like are preffered.

Generally, in the case of oral administration, the daily dose is from about 0.001 to 100 mg/kg, preferably from 0.1 to 30 mg/kg, and more preferably 0.1 to 10 mg/kg, per body weight, administered in one portion or in 2 to 4 divided portions. In the case of intravenous administration, the daily dose is suitably administered from about 0.0001 1 to 10 mg/kg per body weight, once a day or two or more times a day. In addition, a transmucosal agent is administered at a dose from about 0.001 to 100 mg/kg per body weight, once a day or two or more times a day. The dose is appropriately decided in response to the individual case by taking the symptoms, the age, the gender, and the like into consideration.

The compound of the formula (I) can be used in combination with various agents for treating or preventing diseases for which the compound of formula (I) described above is considered to be effective. These agents for treating or preventing, for example, include azoles such as fluconazole, voriconazole, itraconazole, ketoconazole, miconazole, ER30346 and SCH56592; polyenes such as amphotericin B, nystatin, liposomal and lipid forms thereof, for example Abelcet, AmBisome and Amphocil; purine or pyrimidine nucleotide inhibitors such as flucytosine; polyxins such as nikkomycines, particularly, nikkomycine Z or nikkomycine X; other chitin inhibitors; elongation inhibitors such as sordarin and the analog thereof; mannan inhibitors such as predamycin, bactericidal/permeability-inducing (BPI) protein products such as XMP.97 or XMP.127; or complex carbohydrate antifungal agents such as CAN-296, or immune suppressors such as tacrolimus, and they are effective to the infections above. The combination use may be administered by simultaneous administration, or separately and continuously or at desired time intervals. A simultaneous administration agent may be a combination drug or may be separately prepared.

### Examples

Hereinafter, the production processes for the compound of the formula (I) will be described in more detail with reference to Examples. The compound of the formula (I) is not limited to the compounds described in the following Examples. Further, the production processes of the starting compounds will be described in Production Examples.

Daiso-gel, SP-120-40/60-ODS-B (Trademark, made by Daiso Co., Ltd.) was used as ODS column chromatography, unless otherwise specified, in the production for the Example compounds and the Production Example compounds of the present invention. Further, in the description of developing solvent of column chromatography, "-" means gradation and "→" means that a solvent system was changed stepwisely.

### Production Example 1

A mixture of 720 mg of methyl 4-{2-[4-(cyclopentylmethyl)piperazin-1-yl]pyrimidin-5-yl}benzoate, 7 mL of ethanol and 2.02 mL of hydrazine monohydrate was heated under reflux overnight. The reaction mixture was left to cool to room temperature, and the precipitated solid was collected by filtration and dried, to obtain 610 mg of 4-{2-[4-(cyclopentylmethyl)piperazin-1-yl]pyrimidin-5-yl}benzohydrazide.

### Production Example 2

Under a nitrogen atmosphere, to a mixture of 500 mg of methyl trans-4-[5-(4-bromophenyl)-1, 3, 4-thiadiazol-2-yl]cyclohexanecarboxylate, 20 mL of DME, 356 mg of 4-isopropoxyphenylboronic acid and 2 mL of 2.0M aqueous sodium carbonate solution was added 76 mg of tetrakis(triphenylphosphine)palladium(0), followed by stirring at a bath temperature of 85°C overnight. After leaving to cool, the reaction mixture was poured into ice water and stirred, and the precipitated solid was collected by filtration and washed with water. Subsequently, the powder collected by filtration was suspended in acetonitrile, followed by stirring at room temperature. The solid was collected by filtration, washed with acetonitrile and dried at 50°C under reduced pressure, to obtain 510.6 mg of methyl trans-4-[5-(4'-isopropoxybiphenyl-4-yl-1, 3, 4-thiadiazol-2-yl]cyclohexanecarboxylate.

### Production Example 3

To a mixture of 100 mg of methyl trans-4-[5-(4'-isopropoxybiphenyl-4-yl-1, 3, 4-thiadiazol-2-yl]cyclohexanecarboxylate, 1 mL of THF and 0.5 mL of ethanol was added 92 µL of 5M aqueous potassium hydroxide solution, followed by stirring at room temperature overnight. Further, 46 µL of 5M aqueous potassium hydroxide solution was added thereto, followed by stirring for 5 hours at room temperature, and for 1 hour at a bath temperature of 70°C. After leaving to cool, the reaction mixture was diluted with water, followed by acidification with 1M hydrochloric acid. The precipitated solid was collected by filtration, washed with water and acetonitrile sequentially, and dried at 60°C under reduced pressure, to obtain 91.7 mg of trans-4-[5-(4'-isopropoxybiphenyl-4-yl-1, 3, 4-thiadiazol-2-yl]cyclohexanecarboxylic acid.

### Production Example 4

Under a nitrogen gas flow, to a mixture of 200 mg of trans-4-{5-[4-(4-cyclohexyl-4-methoxypiperidin-1-yl)phenyl]-1,3, 4-thiadiazol-2-yl}-N-methoxy-N-methylcyclohexanecarboxamide and 2 mL of DME was added dropwise 393 µL of 1.16M methyl lithium-diethylether solution at 0°C, followed by stirring for 2 hours. To the reaction mixture was added water, and the precipitated solid was collected by filtration and purified with silica gel column chromatography (chloroform:methanol=100:0-97:3), to obtain 73 mg of 1-(trans-4-{5-[4-(4-cyclohexyl-4-methoxypiperidin-1-yl)phenyl]-1, 3, 4-thiadiazol-2-yl}cyclohexyl)ethanone.

### Production Example 5

Under a nitrogen atmosphere, 1.05 g of trans-4-[5-(4'-isopropoxybiphenyl-4-yl)-1, 3, 4-thiadiazol-2-yl]-N-methoxy-N-methylcyclohexanecarboxamide was dissolved in 21 mL of toluene and 21 mL of THF, 3.08 mL of 0.99M diisobutylaluminum hydride-toluene solution were added dropwise at -78°C, followed by stirring for 2 hours at the same temperature. Subsequently, water and 1M hydrochloric acid were added thereto, and the precipitated solid was collected by filtration, washed with 1M hydrochloric acid and water sequentially and dried, to obtain 931 mg of trans-4-[5-(4'-isopropoxybiphenyl-4-yl)-1, 3, 4-thiadiazol-2-yl]cyclohexanecarboaldehyde as a solid.

### Production Example 6

A suspension of 15.86 g of monomethyl trains-1, 4 cyclohexanedicarboxylate, 18.32 g of bromobenzhydrazide, 13.81 g of 1-hydroxybenzotriazole, 17.81 mL of triethylamine and 220 mL of N, N-dimethylformamide(DMF) was added 24.5 g of 1-(3-dimethylaminopropyl)-3-ethylcarbodiimide hydrochloride, followed by stirring for 3 hours at room temperature. Subsequently, the reaction mixture was added to 1 L of water, followed by stirring for 30 minutes. The precipitated solid was collected by filtration, washed with water and then dried under reduced pressure, to obtain 31.58 g of methyl trans-4-{[2-(4-bromobenzoyl)hydrazino]carbonyl}cyclohexanecarboxylate.

### Production Example 7

A suspension of 1.5 g of 5-{4-[(6-methoxyhexyl)oxy]phenyl}-1, 3, 4-thiadiazol-2-amine, methyl trans-4-(bromoacetyl)cyclohexanecarboxylate in 15 mL of ethanol and 7.5mL of THF was heated under reflux for 6 hours. After leaving to cool, 1.5 mL of trifluoroacetic acid was added thereto, followed by heating under reflux again. After leaving to cool, diisopropyl ether was added thereto, followed by stirring, and the precipitated solid was collected by filtration. After washing with same solvent and drying, 2.0 g of methyl trans-4-(2-{4-[(6-methoxyhexyl)oxy]phenyl}imidazo[2,1-b][1, 3, 4]thiazol-6-yl)cyclohexanecarboxylate was obtained as a light brown powder.

### Production Example 8

To a suspension of 300 mg of trans-4-(2-{4-[(6-methoxyhexyl)oxy]phenyl}imidazo[2, 1-b][1, 3, 4]thiadiazol-6-yl)cyclohexanecarboxylic acid and 274 mg of 2-(1H-benzotriazol-1-yl)-1, 1, 3, 3-tetramethyluronium hexafluorophosphate in 3 mL of DMF was added 0.29 mL of diisopropylethylamine, followed by stirring for 1 hour at room temperature. Subsequently, 64 mg ofN, O-dimethylhydroxylamine and 0.11 mL of diisopropylethylamine were added thereto, followed by stirring for 14 hours at room temperature. To the reaction mixture was added 10 mL of water, followed by stirring, and the precipitates were collected by filtration, washed with water and diisopropyl ether and dried, to obtain 312 mg of trans-N-methoxy-4-(2-{4-[(6-methoxyhexyl)oxy]phenyl}imidazo[2, 1-b][1, 3, 4]thiadiazol-6-yl)-N-methylcyclohexanecarboxamide as a white solid.

### Production Example 9

A solution of 10.6 g of 4-{2-[4-(cyclohexyloxy)piperidin-1-yl]pyrimidin-5-yl}benzonitrile, 3.7 g of semithiocarbazide, 106 mL of toluene and 56 mL of trifluoroacetic acid was stirred at 85°C for 7 hours. After leaving to cool, the reaction mixture was concentrated under reduced pressure, 50 mL of THF was added thereto, and the obtained mixture was added to 500 mL of ice water. Subsequently, the pH was adjusted to about 7 with aqueous sodium hydroxide solution, and the precipitated solid was collected by filtration. The obtained solid was washed with diisopropyl ether and vacuum dried, to obtain 11.4 g of 5-(4-{2-[4-(cyclohexyloxy)piperidin-1-yl]pyrimidin-5-yl}phenyl)-1, 3, 4-thiadiazol-2-amine.

### Production Example 10

To a solution of 7.4 g of 4-[(6-bromohexyl)oxy]benzonitrile in 74 mL of methanol was added 25.3 mL of 28% sodium methylate-methanol solution, followed by heating under reflux for 3 hours. After leaving to cool, about half amount of the methanol was evaporated under reduced pressure, water and ethyl acetate were added to carry out separation operation. The obtained organic layer was washed with water and saturated brine and dried over anhydrous magnesium sulfate, and the solvent was evaporated under reduced pressure. The residue was purified with middle pressure silica gel column chromatography (hexane:ethyl acetate=90:10-66:34, gradient), to obtain 5.5 g of 4-[(6-methoxyhexyl)oxy]benzonitrile.

### Production Example 11

To a mixture of 1.07 g of ethyl 1-{2-[4-(tetrahydro-2H-pyran-2-yloxy)phenyl]pyrimidin-5-yl}piperidine-4-carboxylate and 10 mL of THF was added 1.3 mL of 6M hydrochloric acid, followed by stirring for 2 hours at room temperature. Subsequently, under ice cooling, the reaction solution was neutralized with1M aqueous sodium hydroxide solution, and the precipitated solid was collected by filtration, to obtain 0.76 g of ethyl 1-[2-(4-hydroxyphenyl)pyrimidin-5-yl]piperidine-4-carboxylate.

### Production Example 12

To a solution of 0.35 g of ethyl 1-[2-(4-hydroxyphenyl)pyrimidin-5-yl]piperidine-4-carboxylate in 4.5 mL of DMF were added 193 mg of potassium carbonate and 351 mg of 6-methoxy-6-methylheptyl 4-methylbenzenesulfonate, followed by stirring at 90°C overnight. After leaving to cool, the reaction mixture was poured into 100 mL of ice water, and the precipitated solid was collected by filtration and dried, to obtain 0.33 g of ethyl 1-(2-(4-[(6-methoxy-6-methylheptyl)oxy]phenyl)pyrimidin-5-yl)piperidine-4-carboxylate.

### Production Example 13

To a mixture of 40 g of 5-bromo-2-chloropyrimidine, 43.6 g of 4-(cyclohexyloxy)piperidine and 1 L of DMF was added 62.8 g of potassium carbonate, followed by stirring for 3 hours at room temperature. Subsequently, 74 mL of concentrated hydrochloric acid was added thereto until the pH became about 5.5 under ice cooling, followed by stirring for 30 minutes. The precipitated solid was collected by filtration and dried, to obtain 69.5 g of 5-bromo-2-[4-(cyclohexyloxy)piperidin-1-yl]pyrimidine.

### Production Example 14

Under a nitrogen gas flow, to the mixture of 63 g of 5-bromo-2-[4-(cyclohexyloxy)piperidin-1-yl]pyrimidine, 690 mL of DME and 25.8 g of (4-cyanophenyl)boronic acid, were added 185 mL of 2M aqueous sodium carbonate solution and 10.6 g of tetrakis(triphenylphosphine)palladium(0) sequentially, followed by stirring at 85°C overnight. After leaving to cool to room temperature, 800 mL of water was added thereto, and the precipitated solid was collected by filtration. The obtained solid was suspended in 100 mL of acetonitrile, followed by stirring for 30 minutes at 80°C. The precipitates were collected by filtration and dried, to obtain 42.6 g of 4-{2-[4-(cyclohexyloxy)piperidin-1-yl]pyrimidin-5-yl}benzonitrile.

### Production Example 15

Under ice cooling, to a solution of 9.5 g of 1-{4-[4-(cyclobutylmethoxy)piperidin-1-yl]phenyl}ethanone in 76 mL of acetic acid was added 6.6 mL of 30% hydrogen bromide-acetic acid solution, followed by stirring for 5 minutes at same temperature. Further, 11.6 g of pyridinium bromide perbromide was added thereto at room temperature, followed by stirring for 1 hour. Under ice cooling, to the reaction mixture was added 300 mL of water, and the precipitated solid was collected by filtration, washed with water and 500 mL of cooled hexane and dried, to obtain 8.9 g of 2-bromo-1-{4-[4-(cyclobutylmethoxy)piperidin-1-yl]phenyl}ethanone as a slightly green solid.

### Production Example 16

To a solution of 11.2 g of 5-(4-{2-[4-(cyclohexyloxy)piperidin-1-yl]pyrimidin-5-yl}phenyl)-1, 3, 4-thiadiazol-2-amine in 110 mL of acetonitrile, under ice cooling, was added 7.4 g of copper (II) bromide. Subsequently, 5.1 mL of 3-methylbutyl nitrite was added dropwise slowly, followed by stirring for 2 hours at room temperature. The reaction mixture was poured into 400 mL of water, and the pH was adjusted to about 7 with aqueous sodium hydroxide solution. The mixture was extracted with 800 mL of ethyl acetate, followed by washing with 15% aqueous ammonia and saturated brine sequentially. The organic layer was dried over anhydrous magnesium sulfate, and the solvent was evaporated under reduced pressure. Diisopropyl ether was added to the obtained crude product, and the precipitates were collected by filtration and dried, to obtain 8.6 g of 5-[4-(5-bromo-1, 3, 4-thiadiazol-2-yl)phenyl]-2-[4-(cyclohexyloxy)piperidin-1-yl]pyrimidine.

### Production Example 17

To a solution of 420 mg of {1-[5-(4-{2-[4-(cyclohexyloxy)piperidin-1-yl]pyrimidin-5-yl}phenyl)-1, 3, 4-thiadiazol-2-yl]piperidin-4-yl}methanol in 11 mL of dichloromethane (DCM), under ice cooling, was added 399 mg of Dess-Martin periodinane while stirring, followed by stirring for 1 hour at room temperature. To the reaction mixture was added 10% aqueous sodium thiosulfate solution, followed by extracting with 50 mL of DCM. The organic layer was washed with saturated brine and dried over anhydrous magnesium sulfate, and the solvent was evaporated under reduced pressure. The obtained residue was purified with silica gel column chromatography (chloroform:methanol=9:1), to obtain 385 mg of 1-[5-(4-{2-[4-(cyclohexyloxy)piperidin-1-yl]pyrimidin-5-yl}phenyl)-1, 3, 4-thiadiazol-2-yl]piperidine-4-carboaldehyde.

### Production Example 18

To a mixture of 600 mg of 5-[4-(5-bromo-1, 3, 4-thiadiazol-2-yl)phenyl]-2-[4-(cyclohexyloxy)piperidin-1-yl]pyrimidine, 207 mg of piperidin-4-ylmethanol and 9 mL of N, N-dimethylacetamide(DMA) was added 0.3 mL of triethylamine, followed by stirring at 90°C overnight. After leaving to cool, the precipitated solid was collected by filtration, washed with 20 mL of water and 20 mL of 50% aqueous acetonitrile and dried, to obtain 0.42 g of {1-[5-(4-{2-[4-(cyclohexyloxy)piperidin-1-yl]pyrimidin-5-yl}phenyl)-1, 3, 4-thiadiazol-2-yl]piperidin-4-yl}methanol.

### Production Example 19

To a mixture of 4.2 g of cesium carbonate, 181 mg of palladium(II) acetate, 336 mg of 2, 2'-bis(diphenylphosphino)-1, 1'-binaphthyl and 54 mL of toluene, under a nitrogen gas flow, were added 1.81 g of 5-bromo-2-[4-(tetrahydro-2H-pyran-2-yloxy)phenyl]pyrimidine and 1 g of ethyl piperidine-4-carboxylate sequentially, followed by stirring for 20 hours at 110°C. After leaving to cool, water was added thereto, and the precipitated solid was collected by filtration, washed with water and acetonitrile and dried, to obtain 1.07 g of ethyl 1-{2-[4-(tetrahydro-2H-pyran-2-yloxy)phenyl]pyrimidin-5-yl}piperidine-4-carboxylate.

### Production Example 20

Under a nitrogen gas flow, the suspension of 31.5 g of methyl trans-4-{[2-(4-bromobenzoyl)hydrazino]carbonyl}cyclohexanecarboxylate, 850 mL of THF and 21.9 g phosphorus pentasulfide was stirred for 48 hours at room temperature. The reaction mixture was poured into 3 L of water, 100 mL of 1M aqueous sodium hydroxide solution was added thereto, followed by stirring for 1 hour. The precipitated solid was collected by filtration, washed with water and dried. The obtained solid and 20 g of anhydrous magnesium sulfate, 15 g of silica gel and activated carbon were suspended in 500 mL of chloroform, followed by stirring for 5 minutes. The insoluble matter was separated by filtration, and the filtrate was concentrated under reduced pressure, to obtain 30.2 g of methyl trans-4-[5-(4-bromophenyl)-1, 3, 4-thiadiazol-2-yl]cyclohexanecarboxylate as a white solid.

### Production Example 21

Under a nitrogen atmosphere, a solution of 1.3 g of methyl trans-4-[5-(4-{2-[4-(cyclopentylmethyl)piperazin-1-yl]pyrimidin-5-yl}phenyl)-1, 3, 4-thiadiazol-2-yl]cyclohexanecarboxylate in anhydrous THF was cooled in an ice bath, 135 mg of lithium aluminum hydride was added thereto, while keeping the temperature of about 0°C. After stirring for 1 hour at room temperature and cooling in the ice bath again, 20 mL of chloroform, 600 mg of sodium fluoride and 2 mL of water were added thereto sequentially. The reaction mixture was filtrated through celite pad, the solvent was evaporated under reduced pressure, and the obtained solid was washed with acetonitrile, to obtain 652 mg of {trans-4-[5-(4-{2-[4-(cyclopentylmethyl)piperazin-1-yl]pyrimidin-5-yl}phenyl)-1, 3, 4-thiadiazol-2-yl]cyclohexyl methanol.

### Production Example 22

Under a nitrogen atmosphere, to a solution of 0.88 g of trans-4-[5-(4-{2-[4-(cyclohexyloxy)piperidin-1-yl]pyrimidin-5-yl}phenyl)-l, 3, 4-thiadiazol-2-yl]-N-methoxy-N-methylcyclohexanecarboxamide in 20 mL of THF, under ice cooling, was added 62 mg of lithium aluminium hydride, followed by stirring for 30 minutes. Subsequently, 252 mg of cesium fluoride and 3 mL of water were added thereto, followed by filtrating through celite pad. The filtrate was concentrated under reduced pressure, diisopropyl ether was added thereto, and the obtained solid was collected by filtration and vacuum dried, to obtain 0.69 g of {trans-4-[5-(4-{2-[4-(cyclohexyloxy)piperidin-1-yl]pyrimidin-5-yl}phenyl)-1, 3, 4-thiadiazol-2-yl]cyclohexyl}methanol.

### Production Example 23

To a mixture of 474 mg of the compound (product) of Example 138 of WO00/64927, 120 mg of 1,4-dioxane-2,5-diol, 4.8 mL of methanol and 4.8 mL of DMF, under room temperature, were added 0.082 mL of acetic acid and 63 mg of sodium cyanoborohydride, followed by stirring overnight. To the reaction mixture was added 100 mL of ethyl acetate, and the precipitated solid was collected by filtration and vacuum dried. The solid was dissolved in water and purified with ODS column chromatography (20% aqueous acetonitrile). The fractions containing the target compound were combined, acetonitrile was evaporated under reduced pressure, and the residue was subjected to freeze drying, to obtain 433 mg of the compound of Production Example 23.

### Production Example 24

A suspension of 630 mg of the compound of Production Example 29, 9.4 mL of methanol and 235 mg of 10% palladium-carbon (50% water content) was stirred at room temperature for 4 hours under the hydrogen atmosphere of 1 atm. The catalyst was removed by filtration, followed by washing with 50 mL of methanol. The filtrate was evaporated under reduced pressure, and the residue was dried, to obtain 0.24 g of the compound of Production Example 24.

### Production Example 25

A suspension of 630 mg of the compound of Production Example 23 in 13 mL of DCM was cooled to 5°C, 1.4 mL of trifluoroacetic acid was added dropwise, followed by stirring for 2 hours at room temperature. The reaction mixture was poured into the mixture of 100 mL of saturated aqueous sodium bicarbonate and 100 mL of pH 6.86 standard buffer. The organic solvent was evaporated under reduced pressure, the remaining aqueous solution was purified with ODS column chromatography (5→10→15→20% acetonitrile/water). The fractions containing the target compound were combined, acetonitrile was evaporated under reduced pressure, and the residue was subjected to freeze drying, to obtain 480 mg of the compound of Production Example 25.

### Production Example 26

To a solution of 30 mg of the compound (product) of Example 43 of WO01/60846 in 300 µL of DMF, was added 25 µL of piperidine, followed by stirring for 1 hour at room temperature. The reaction mixture was diluted with ethyl acetate, and the precipitated solid was collected by filtration and washed with ethyl acetate. The solid was dried under reduced pressure to obtain 22.4 mg of the compound of Production Example 26.

### Production Example 27

To a mixture of 1.04 g the compound of Production Example 1 of WO00/64927 in 14 mL of DMF and 14 mL of methanol was added 14 mL of (dimethoxymethyl) dimethylamine, followed by stirring for 48 hours. Subsequently, 6 mL of 1M aqueous sodium hydroxide solution was added thereto, followed by stirring overnight. The reaction mixture was neutralized with 1M hydrochloric acid and diluted with 500 mL. of water, and the obtained solution was purified with 180 mL of ODS column chromatography. The fractions containing the target compound were combined and the solvent was evaporated under reduced pressure. The residue was subjected to freeze drying to obtain 350 mg of the compound of Production Example 27.

### Production Example 28

To a mixture of 1.85 g of the compound (product) of Production Example 25 of WO03/068807 and 18 mL of DMF was added 0.3 mL of 3-bromo-1-propene and 360 mg of potassium carbonate, followed by stirring overnight. To the reaction mixture was added 50 mL of ethyl acetate, and the precipitated solid was collected by filtration. The obtained powder was purified with 180 mL of ODS column chromatography. The fractions containing the target compound were combined, the solvent was evaporated under reduced pressure, and the residue was subjected to freeze drying, to obtain 1.15 g of the compound of Production Example 28.

### Production Example 29

To a mixture of 1 g of the compound of Production Example 28, 20 mL of tert-butanol and 20 mL of water were added 1.28 g of AD-MIX-BETA (made by Aldrich corporation) and 135 mg of methane sulfonamide, followed by stirring overnight. To the reaction mixture was added 50 mL of ethyl acetate, and the precipitated solid was collected by filtration. The obtained solid was purified with 80 mL of ODS column chromatography. The fractions containing the target compound were combined, acetonitrile was evaporated under reduced pressure, and the residue was subjected to freeze drying, to obtain 512 mg of the compound of Production Example 29.

### Production Example 30

To a mixture of 328 mg of the compound of Production Example 27, 46.7 mg of O-(benzotriazol-1-yl)-N, N, N', N'-tetramethyluronium hexafluorophosphate and 1.4 mL of DMF, were added 0.35 mL ofN, N-dimethylethane-1, 2-diamine and 83 µL of N, N-diisopropylethylamine, followed by stirring overnight. Subsequently, 50 mL of ethyl acetate was added thereto, and the precipitated solid was collected by filtration and purified with 30 mL of ODS column chromatography. The fractions containing the target compound were combined, the solvent was evaporated under reduced pressure, and the residue was subjected to freeze drying, to obtain 131 mg of the compound of Production Example 30.

### Production Example 31

Under a nitrogen atmosphere, 168 mg of the compound of Production Example 103 was dissolved in 1.68 mL of methanol, 357 µL of 2M trimethylsilyldiazomethane-hexane solution was added thereto, followed by stirring for 4 hours at room temperature. The reaction mixture was concentrated under reduced pressure, and the obtained solid was dissolved in 2mL of DMF, and purified with ODS column chromatography (10→20→25→30→35% aqueous acetonitrile). The fractions containing the target compound were combined, and the solvent was evaporated under reduced pressure and freeze dried, to obtain 141.7 mg of the compound of Production Example 31 as a white solid.

### Production Example 32

Under a nitrogen atmosphere, a mixture of 80 g of the compound of Production Example 26, 320 mL of DMF, 30.69 mL of diisopropyl ethyl amine and 46.1 g of di-tert-butyl dicarbonate was stirred at room temperature overnight. Subsequently, 20 mL of toluene was added thereto, and the solvent was evaporated under reduced pressure. Under a nitrogen gas flow, the reaction mixture was added to 4 L of ethyl acetate while stirring, and the precipitated solid was collected by filtration. The solid was washed with 500 mL of ethyl acetate, and dried under reduced pressure at 45°C for 8 hours, subsequently at room temperature overnight, to obtain 118.36 g of the compound of Production Example 32 as a colorless solid.

### Production Example 33

Under a nitrogen gas flow, to a mixture of 1.0 g of methyl trans-4-[5-(4-bromophenyl)-1, 3, 4-thiadiazol-2-yl]cyclohexanecarboxylate, 10 mL of toluene, 593.8 mg of 4, 4-dimethylpiperidine, 206.4 mg of 2'-(dicyclohexylphosphino)-N, N-dimethylbiphenyl-2-amine and 779.4 mg of potassium phosphate, was added 150.8 mg of tris(dibenzylideneacetone)dipalladium, followed by stirring for 15 hours at 100°C. To the reaction mixture were added 100 mL of DCM and 30 mL of 1M hydrochloric acid, and the organic layer was separated. The organic layer was washed with saturated brine and dried over anhydrous magnesium sulfate, and the solvent was evaporated under reduced pressure. The residue was purified with silica gel column chromatography (chloroform:methanol=100:0-97:3), to obtain 350 mg of methyl 4-{5-[4-(4, 4-dimethylpiperidin-1-yl)phenyl]-1, 3, 4-thiadiazol-2-yl}cyclohexanecarboxylate.

### Production Example 34

To a mixture of 800 mg of methyl 4-(2-piperazin-1-ylpyrimidin-5-yl)benzoate dihydrochloride, 12 mL of DCM, 270 µL of cyclopentanecarboaldehyde and 600 µL of triethylamine, were added 639 mg of sodium triacetoxyborohydride and 123 µL of acetic acid, followed by stirring at room temperature overnight. To the reaction mixture were added DCM and water, and the organic layer was separated. The organic layer was washed with saturated brine and dried over anhydrous magnesium sulfate, and the solvent was concentrated under reduced pressure, to obtain 720 mg of methyl 4-{2-[4-(cyclopentylmethyl)piperazin-1-yl]pyrimidin-5-yl}benzoate.

### Production Example 35

To a solution of 2.5 g of tert-butyl 4-{5-[4-(methoxycarbonyl)phenyl]pyrimidin-2-yl}piperazine-1-carboxylate in 15 mL of 1,4-dioxane was added 15 mL of 4M hydrogen chloride-1, 4-dioxane solution, followed by stirring at room temperature overnight. The precipitated solid was collected by filtration and dried, to obtain 2.2 g of methyl 4-(2-piperazin-1-ylpyrimidin-5-yl)benzoate dihydrochloride.

### Production Example 36

Under a nitrogen atmosphere, to a solution of 1.75 g of 5-bromo-2-[4-(cyclohexyloxy)piperidin-1-yl]pyrimidine in 17.5 mL of THF, was added dropwise 3.85 mL of 1.67M n-butyllithium-hexane solution over 10 minutes under dry ice-acetone cooling, followed by stirring for 20 minutes. Subsequently, 1.42 mL of triisopropyl borate was added dropwise over 10 minutes, followed by stirring for 10 minutes. Further, 0.4 mL of methanol was added thereto, a cooling bath was taken off, the temperature was allowed to rise to room temperature, 50 mL of water was added thereto, and further the solution was acidified with 1M hydrochloric acid. To the mixed solution was added ethyl acetate, to carry out separation operation. The organic layer was washed with water and saturated brine sequentially, dried over anhydrous magnesium sulfate, and concentrated under reduced pressure, to obtain 1.47 g of {2-[4-(cyclohexyloxy)piperidin-1-yl]pyrimidin-5-yl}boronic acid.

### Production Example 125

500 mg of the compound of Production Example 186 was dissolved in 10 mL of DCM, under a nitrogen gas flow and under dry ice-acetone cooling, 1.354 mL of 1M diisobutylaluminum hydride/toluene solution was added dropwise, followed by stirring for 2 hours at the same temperature. 1 mL of water was slowly added to the reaction mixture, subsequently 10 mL of 1M hydrochloric acid and 50 mL of chloroform were added thereto to carry out separation operation. The organic layer was washed with saturated brine and dried over anhydrous magnesium sulfate, and the solvent was evaporated under reduced pressure. 369.1 mg of the compound of Production Example 125 was obtained as crude product, and was used in next reaction without further purification.

### Production Example 126

Under ice cooling, to a solution of 0.154 g of the compound of Production Example 187 in 2 mL of THF, were added 21 mg of lithium borohydride and 0.7 mL of methanol sequentially, followed by stirring for 4 hours. Subsequently, 1M hydrochloric acid and water were added sequentially, and the precipitated solid was collected by filtration and vacuum dried, to obtain 0.12 g of the compound of Production Example 126.

Production Example 127

To a suspension of 627 mg of (methoxymethyl)trimethylphosphonium chloride and 5 mL of THF was added dropwise 731 µL of 2.6M n-butyllithium/n-hexane solution, while keeping the internal temperature of 5°C or less under ice/brine bath cooling, followed by stirring for 30 minutes at the same temperature. Subsequently, the solution of 300 mg of tert-butyl 4-oxoazepine-1-carboxylate in 3 mL of THF was added dropwise thereto at the same temperature, followed by stirring for 30 minutes at the same temperature. Saturated aqueous ammonium chloride solution and ethyl acetate were added thereto to carry out separation operation. The organic layer was washed with saturated brine and dried over anhydrous magnesium sulfate, and the solvent was evaporated under reduced pressure. The residue was purified with silica gel column chromatography (hexane:ethyl acetate=100:0-90:10), to obtain 192 mg of the compound of Production Example 127.

### Production Example 128

To 711 mg of the compound of Production Example 192 were added 7 mL of DMF, 332 mg of 1-hydroxybenzotriazole(HOBt), 64 mg of 1-ethyl-3-(3-dimethylaminopropyl)carbodiimide (WSC) hydrochloride and 0.46 mL of triethylamine sequentially, followed by stirring for 1 hour at room temperature. Subsequently, 470 mg of the compound of Production Example 130 was added thereto, followed by stirring for 15 hours at room temperature. Water was added to the reaction mixture, and the precipitated solid was collected by filtration and dried under reduced pressure, to obtain 890 mg of the compound of Production Example 128.

### Production Example 129

A solution of 2.3 g of the compound of Production Example 128 in 23 mL of DMA was stirred at 130°C overnight. After leaving to cool to room temperature, water and ethyl acetate were added thereto to carry out separation operation. The organic layer was washed with saturated brine and dried over anhydous magnesium sulfate, and the solvent was evaporated under reduced pressure, to obtain 1.25 g of the compound of Production Example 129.

### Production Example 130

A mixutre of 820 mg of methyl trans-4-cyanocyclohexanecarboxylate, 10 mL of methanol, 1.02 mL of triethylamine and 511 mg of hydroxylammonium chloride was heated under reflux for 6 hours. After leaving to cool to room temperature, water and ethyl acetate were added thereto to carry out separation operation. The organic layer was washed with water and saturated brine and dried over anhydrous magnesium sulfate, and the solvent was evaporated under reduced pressure, to obtain 0.48 g of the compound of Production Example 130.

### Production Example 131

2.380 g of the compound of the Production Example 141 was dissolved in 200 mL of water, followed by purification with 150 mL of ODS column chromatography (0% →10% →20% →35% acetonitrile/water). The fractions containing the target product were combined, acetonitrile was evaporated under reduced pressure, and the residue was subjected to freeze drying, to obtain 0.441 g of the compound of Production Example 126.

### Production Example 132

To a solution of 100 mg of the compound of Production Example 127 in 1 mL of DCM were added 0.22 mL of anisol, 0.04 mL of water and 0.16 mL of trifluoroacetic acid sequentially, followed by stirring for 1 hour at room temperature. Further, 0.16 mL of trifluoroacetic acid was added thereto, followed by stirring for 1 hour at room temperature. The reaction mixture was concentrated under reduced pressure and dried under reduced pressure, to obtain 102 mg of 4-formyl-azepine trifluoroacetate.

### Production Example 133

Under a hydrogen atmosphere, a mixure of 570 mg of the compound of Production Example 205, 11.4 mL of methanol and 285 mg of 10% palladium/carbon (50% water content) was stirred for 4 hours at room temperature. The catalyst was removed by filtration, followed by washing with 10 mL of methanol, and the filtrate was concentrated under reduced pressure. Water was added to the residue, followed by freeze drying, to obtain 420 mg of the compound of Production Example 133.

### Production Example 134

To 5.50 g of the compound of Production Example 140 was added 100 mL of DMF, under ice cooling, at the internal temperature of 4°C, 2.30 mL of DIPEA, and 1.37 g of 9-fluorenylmethyl chloroformate (internal temperature of 6°C) were added thereto, followed by stirring for 2 hours at the same temperature. The reaction mixture was poured into 1000 mL of water, and 20 mL of acetonitrile was added. The mixture thereof was purified with 120 mL of ODS column chromatography (0% →20% →60% acetonitrile/water). Acetonitrile in the fraction containing the target product was evaporated under reduced pressure, and the residue was subjected to freeze drying, to obtain 5.95 g of the compound of Production Example 134.

### Production Example 135

To a suspension of 60g of the compound (product) of Example 2 of WO99/40108, 300 mL of water and 300 mL of 1, 4-dioxane were added dropwise 132 mL of 1M aqueous sodium hydroxide solution and the solution of 20 g of di-tert-butyl dicarbonate in 150 mL of 1, 4-dioxane, followed by stirring for 3.5 hours at room temperature. 1, 4-dioxane was evaporated under reduced pressure, and the residue was purified with 300 mL of ODS column chromatography (0% →50% acetonitrile/water). The fraction that leaked during charging in this operation was diluted with 1500 mL of water and purified with 300 mL of ODS column chromatography (after charging, saturated aqueous sodium bicarbonate and saturated brine were flowed out sequentially, followed by elution with 0% →50% acetonitrile/water). The fraction that leaked during charging in this operation was diluted with 3000 mL of water and purified with ODS column chromatography, as described above. Acetonitrile in the fraction containing the target product obtained in each of the purification operations was evaporated under reduced pressure, followed by freeze drying, to obtain 58.3 g of the compound of Production Example 135.

### Production Example 136

To 58.3 g of the compound of Production Example 135 were added 525 mL of DMF, 13.5 mL of benzyl bromide and 3.57 g of lithium hydroxide monohydrate sequentially, followed by stirring for 30 hours at room temperature. The reaction mixture was poured into 3500 mL of ethyl acetate, followed by stirring vigorously. The resultant was allowed to stand until the precipitated solid was settled down, the supernatant was removed, and the solid was collected by filtration and dried under reduced pressure. The obtained solid was suspended in 1000 mL of water, 14.4 g of sodium hydrogen carbonate was added thereto, followed by stirring and then insoluble matter was removed by filtration. The filtrate was purified with 700 mL of ODS column chromatography (0% →10% →50% acetonitrile/water). To 2000 mL of the fraction that leaked when 10% acetonitrile/water was used as eluent in this purificaton was added 1000 mL of water, and the solution was purified with ODS column chromatography (after charging, double the resin volume of saturated aqueous sodium bicarbonate and saturated brine were each flowed out, followed by elution with 0% → 10% →50% acetonitrile/water). To 1500 mL of the fraction that leaked during charging in this purification operation was added 1000 mL of water, and the solution was separated into three portions, then ehach portion was purified with ODS column chromatography as described above. Acetonitrile in the fraction containing the target product obtained in each of the purification operations was evaporated under reduced pressure, followed by freeze drying, to obtain 55.4 g of the compound of Production Example 136.

### Production Example 137

To 28.4 g of the compound of Production Example 136 was added 350 mL of DMF, 180 mL of 2.74M hydrogen chloride/methanol solution was added dropwise, followed by stirring for 6 hours under ice cooling. The reaction mixture was poured into 5000 mL of water, 1M aqueous sodium hydroxide solution was added to adjust the pH to about 7. The mixture was purified with 700 mL of ODS column chromatography (0% →10% →20% →50% acetonitrile/water). Acetonitrile in 500 mL of the fraction containing the target product was evaporated under reduced pressure, followed by freeze drying, to obtain 25.0 g of the compound of Production Example 137.

### Production Example 138

A mixture of 20.0 g of the compound of Production Example 137, 200 mL of DMF, 12.4 g of lithium hydroxide monohydrate and 50 mL of 1, 2-dibromoethane was stirred for 27 hours at room temperature. The reaction mixture was poured into a mixed solvent of 3500 mL of water and 900 mL of acetonitrile, followed by purification with 700 mL of ODS column chromatography (0% →10% →20% →30% →40% acetonitrile/water). 1500 mL of the fraction containing the target product was diluted with 1000 mL of water, acetonitrile was added until the precipitates were dissolved, followed by purification with 300 mL of ODS column chromatography (acetonitrile/water was used as an elution solvent, after triple the resin volume of eluent was flowed out at an acetonitrile concetration of 0%, followed by elution at acetonitrile concentration of 70%). Acetonitrile in the fraction of the target product was evaporated under reduced pressure, followed by freeze drying, to obtain 14.6 g of the compound of Production Example 138.

### Production Example 139

A mixture of 6.50 g of the compound of Production Example 138, 65 mL of DMF and 0.753 g of sodium azide was stirred at 70°C for 4 hours. The reaction mixture was diluted with 1600 mL of water, followed by purification with 120 mL of ODS column chromatography (0% →60% acetonitrile/water). The fractions containing the target product were combined, acetonitrile was evaporated under reduced pressure, and the residue was subjected to freeze drying, to obtain 5.74 g of the compound of Production Example 139.

### Production Example 140

Under a hydrogen atmosphere, a mixture of 5.74 g of the compound of Production Example 139, 110 mL of methanol and 1.70 g of 10% palladium/carbon (50% water content) was stirred for 12 hours at room temperature. After nitrogen substitution, the resultant was allowed to stand overnight, followed by stirring again for 4 hours at room temperature under a hydrogen atmosphere. The catalyst was removed by filtration through celite pad, and the filtrate was concentrated under reduced pressure. To the residue was added 500 mL of water, followed by purification with 120 mL of ODS column chromatography (0% →30% →70% acetonitrile/water). The fractions containing the target product were combined, acetonitrile was evaporated under reduced pressure, and the residue was subjected to freeze drying, to obtain 5.50 g of the compound of Production Example 140.

### Production Example 141

Under ice cooling, to a mixture of 2.00 g of the compound of Production Example 134 and 1.00 mL of water was added 20.0 mL of trifluoroacetic acid, followed by stirring for 3 hours at room temperature. The reaction mixture was concentrated under reduced pressure, and water was added to the residue. The mixture was sujected to freeze drying, to obtain 2.38 g of the compound of Production Example 141.

The production Example compounds described in the following Tables were produced using corresponding starting materials respectively, similarly to the methods of the Production Examples. The structures, the production processes, and the physicochemical data of the compounds in Production Example 1 to Example 203 are shown in Table 2 to Table 23, and Table 40 to Table 53.

### Example 1 (Production Process A)

To a mixture of 134 mg of trans-4-{5-[4-(4-cyclohexyl-4-methoxypiperidin-1-yl)phenyl]-1, 3, 4-thiadiazol-2-yl}cyclohexanecarboaldehyde, 2 mL of methanol, 2 mL of chloroform, 2 mL of DMF and 200 mg of the compound of Example 22 (product) of WO2005/005463 were added 26.4 mg of sodium cyanoborohydride and 35 µL of acetic acid, followed by stirring at room temperature overnight. To the reaction mixture was added 567 µL of piperidine, further followed by stirring for 1 hour. Ethyl acetate was added thereto, and the precipitated solid was collected by filtration, followed by purification with ODS column chromatography (acetonitrile:pH 3 aqueous hydrochloric acid =0:100→10:90→20:80→25:75→30:70). Acetonitrile in the fraction containing the target product was evaporated under reduced pressure and freeze dried, to obtain 214 mg of the compound of Example 1 as yellow amorphous.

### Example 2 (Production Process B)

To a mixture of 385 mg of 1-[5-(4-{2-[4-(cydohexyloxy)piperidin-1-yl]pyrimidin-5-yl}phenyl)-1, 3, 4-thiadiazol-2-yl]piperidine-4-carboaldehyde, 4.8 mL of methanol, 7.7 mL of DMF, 4.8 mL of DCM, and 1.2 g of the compound of Production Example 26 were added 0.12 mL of acetic acid and 91 mg of sodium cyanoborohydride, followed by stirring at room temperature overnight. To the reaction mixture was added 100 mL of ethyl acetate, the precipitated solid was collected by filtration and vacuum dried. The solid was dissolved in aqueous sodium hydroxide solution, followed by purification with 80 mL of ODS column chromatography (40% aqueous acetonitrile). The fractions containing the target compound were combined, acetonitrile was evaporated under reduced pressure, and the residue was subjected to freeze drying, to obtain 610 mg of the compound of Example 2.

### Example 3 (Production Process C)

To a solution of 40 mg of the compound of Example 16 in 1.2 mL of methanol, was added dropwise 1.24 mL of 10% hydrogen chloride-methanol solution at room temperature, followed by stirring for 1 hour at room temperature. To the reaction mixture was added 200 mL of water, followed by purification with 20 mL of ODS column chromatography (acetonitrile:water=0:100→10:90→20:80→30:70→40:60→45:55→50:50). The fraction containing the target product was diluted twice, followed by purification with 20 mL of ODS column chromatography (acetonitrile:water=80:20) again. The solvent was evaporated under reduced pressure, and the residue was subjected to freeze drying, to obtain 33 mg of the compound of Example 3.

### Example 43 (Production Process D)

A suspension of 111 mg of the compound of Production Example 204 in 2 mL of DCM was stirred at 5°C and 150 µL of trifluoroacetic acid was added dropwise thereto. After stirring for 2 hours at room temperature, it was poured into the mixture of 10 mL of saturated aqueous sodium bicarbonate and 10 mL of pH 6.86 standard buffer. The organic solvent was evaporated under reduced pressure, the remaining aqueous solution was purified with 20 mL of ODS column chromatography (5→20% acetonitrile/water). The fractions containing the target product were combined, and acetonitrile was evaporated under reduced pressure. The residue was subjected to freeze drying, to obtain 63 mg of the compound of Example 43.

### Example 44 (Production Process E)

To a mixture of 30 mg of the compound of Example 51, 0.5 mL of DMF, 0.5 mL of chloroform, 4 mg of formaldehyde and 0.5 mL methanol, were added 4 µL of acetic acid and 2.9 mg of sodium cyanoborohydride, followed by stirring at room temperature overnight. To the reaction mixture was added 10 mL of ethyl acetate, and the precipitated solid was collected by filtration and dried under reduced pressure. The solid was dissolved in water, followed by purification with 20 mL of ODS column chromatography (40% acetonitrile/water). The fractions containing the target product were combined, and acetonitrile was evaporated under reduced pressure. The residue was subjected to freeze drying, to obtain 29 mg of the compound of Example 44.

### Example 45 (Production Process F)

100 mg of the compound of Example 22, 32 mg of glyoxylic acid hydrate and 300 mg of molecular sieves 3Å, were suspended in DMF, followed by stirring at room temperature overnight. The reaction mixture was diluted with methanol, the molecular sieves was separated by filtration, followed by washing with methanol. The filtrate and the washings were combinded, followed by concentration under reduced pressure, ethyl acetate was added to the residue, and the precipitated solid was collected by filtration, followed by washing with ethyl acetate. The solid was dried and dissolved in water, followed by adjusting the pH to about 7.5 with 1M aqueous sodium hydroxide solution. This solution was purified with ODS column. (ODS gel:YMC GEL ODS-A 12nm S-50µm AA12S50 20cc, elution solvent:acetonitrile:water=5:95→20:80→30:70→40:60→45:55, detector:UV210nm, flow rate 50ml/minute), the fractions containing the target product were combinded and concentrated under reduced pressure, and then the residue was subjected to freeze drying, to obtain 48.1 mg of the compound of Example 45.

In the same manner as the above Examples, Example compounds shown in the following Tables were produced using corresponding starting materials respectively. The structure of the compound of Example 1 to Example 75 are shown in Table 24 to Table 37 and Table 54 to Table 64, the production processes and the physicochemical data are represented in Table 38 and Table 39, and Table 65 and Table 66.
Further, the following abbreviations are used in Production Examples, Examples, and the following Tables. Pre: Production Example number, Ex: Example number, Str: structural fomula, Syn: production process (among the Examples /Production Examples above, Production Example number produced in the same manner or Production Process as Example is shown. For example, it shows that the compound of Production Example 37 was produced in the same manner as the compound of Production Example 20, Example 4 was produced in the same manner as Example 1.), Dat: physicochemical data (NMR1: δ(ppm) in 1H NMR of DMSO-d₆, NMR2: δ(ppm) in 1H NMR of DMSO-d₆+D₂O, NMR3: δ(ppm) in 1H NMR of CDCl₃, ESI: ESI+(cation), ESI-(anion), EI: EI-MS(cation)), Sal: when a compound is acid addition salt, the factor of acid and base is shown ("-" means free form), Me: methyl, Et: ethyl, Boc: tert-butoxycarbonyl, Bn: benzyl, Z: benzyloxycarbonyl group, Fmoc: (9H-fluoren-9-ylmethoxy)carbonyl group.

**[Table 2]**

| Pre | Syn | Str | Dat |
|---|---|---|---|
| 1 | 1 | 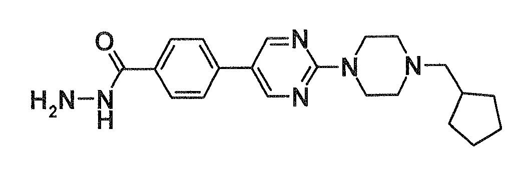 | NMR1: 1.14-1.24 (2H, m), 1.51-1.75 (6H, m), 2.10-2.24 (3H, m), 2.43 (4H, t, J = 5 Hz), 3.78 (4H, t), 4.50 (2H,br),7.73 (2H, d, J = 8.4 Hz), 7.90 (2H, d, J = 8.4 Hz), 8.76 (2H, s), 9.81 (1H, br) |
| 2 | 2 | 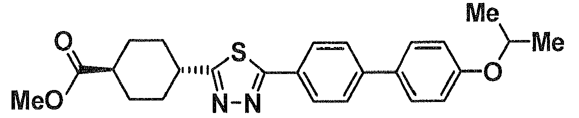 | ESI+: 459 (M+Na) |
| 3 | 3 | 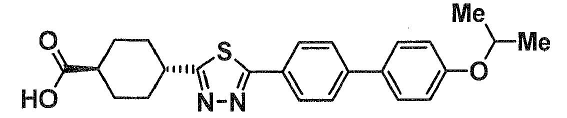 | ESI-: 420.8 |
| 4 | 4 | 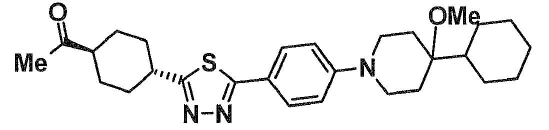 | ESI+: 482.4 |
| 5 | 5 | 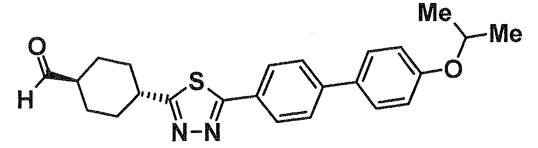 | ESI-: 405.1 |
| 6 | 6 | 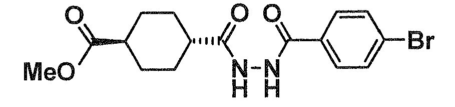 | ESI+: 405.1, 407.0 |

**[Table 3]**

| | | | |
|---|---|---|---|
| 7 | 7 | 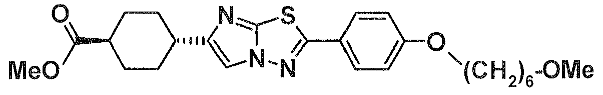 | ESI+: 472 |
| 8 | 8 | 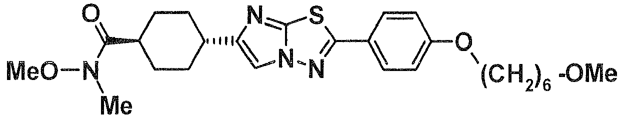 | ESI+: 501 |
| 9 | 9 | 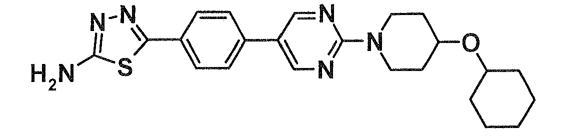 | ESI+: 437.6 |
| 10 | 10 | 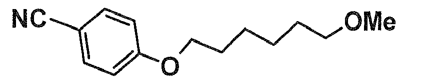 | ESI+: 256 |
| 11 | 11 | 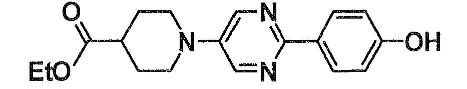 | ESI+: 327.4 |
| 12 | 12 | 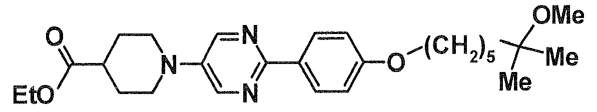 | ESI+: 469.6 |
| 13 | 13 | 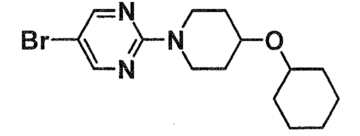 | ESI+: 340.2, 342.2 |
| 14 | 14 | 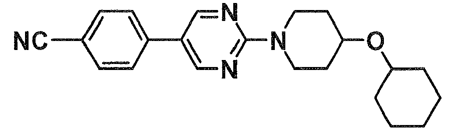 | ESI+: 363.5 |
| 15 | 15 | 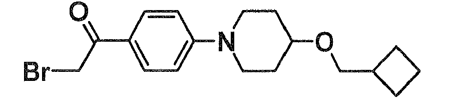 | ESI+: 366, 368 |

**[Table 4]**

| | | | |
|---|---|---|---|
| 16 | 16 | 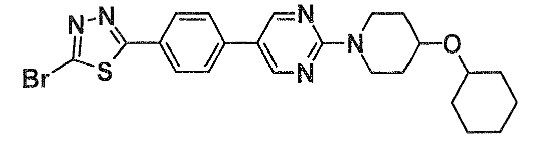 | ESI+: 500.5, 502.5 |
| 17 | 17 | 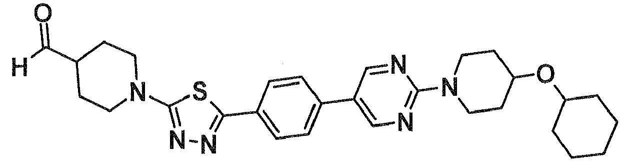 | ESI+: 533.5 |
| 18 | 18 | 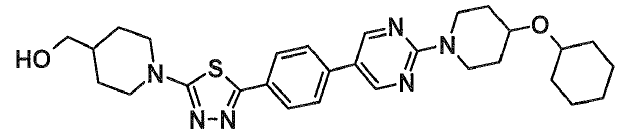 | ESI+: 535.2 |
| 19 | 19 | 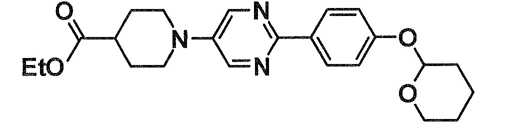 | ESI+: 412.5 |
| 20 | 20 | 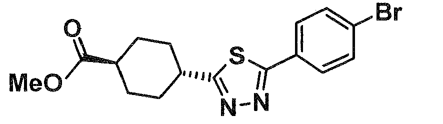 | ESI+: 403.1 |
| 21 | 21 | 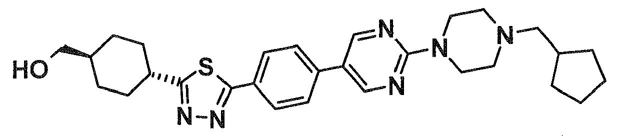 | ESI+: 521.2 |
| 22 | 22 | 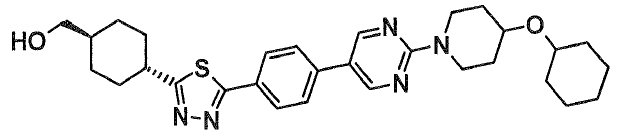 | ESI+: 534.6 |

**[Table 5]**

| | | | |
|---|---|---|---|
| 23 | 23 | 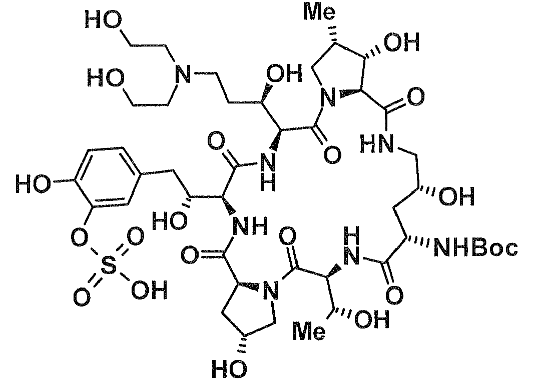 | ESI-: 1078.5 |
| 24 | 24 | 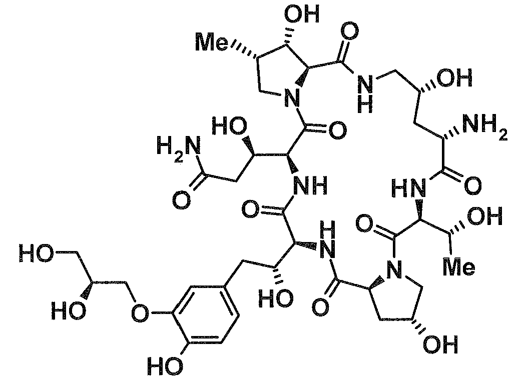 | ESI+: 899.4 |
| 25 | 25 | 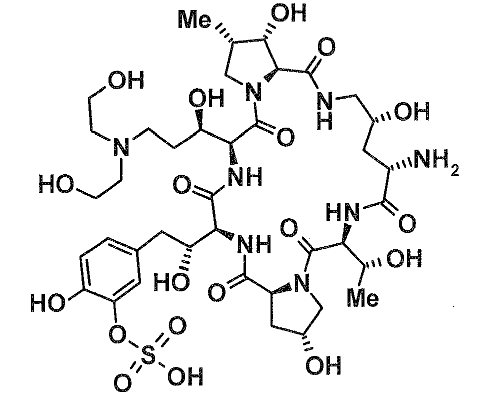 | ESI-: 978.3 |

**[Table 6]**

| | | | |
|---|---|---|---|
| 26 | 26 | 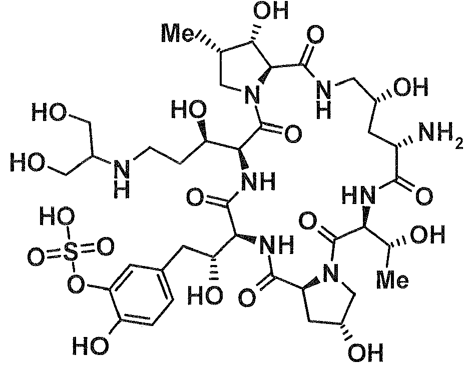 | ESI-: 963 |
| 27 | 27 | 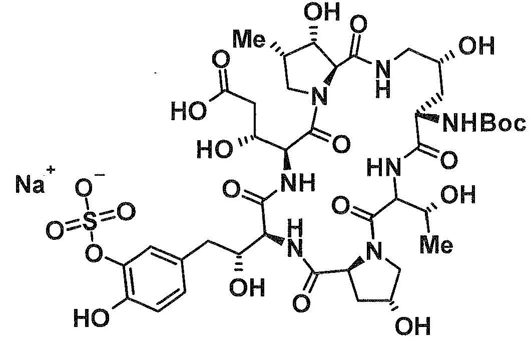 | ESI-: 1004.8 |
| 28 | 28 | 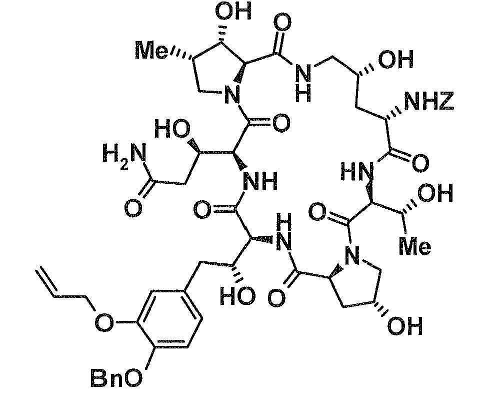 | ESI+: 1090.2 |

**[Table 7]**

| | | | |
|---|---|---|---|
| 29 | 29 | 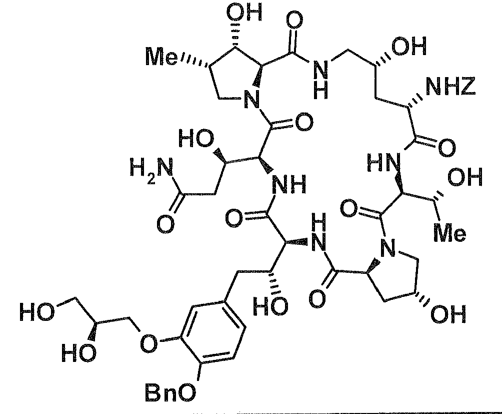 | ESI+: 1124.3 |
| 30 | 30 | 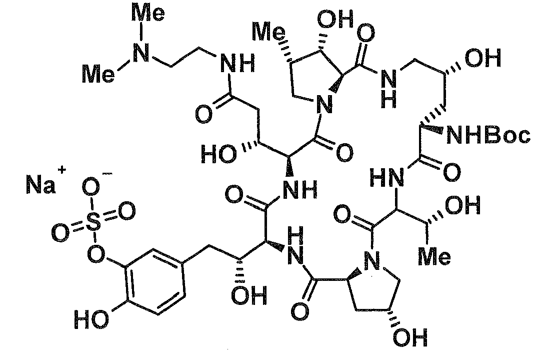 | ESI-: 1074.7 |
| 31 | 31 | 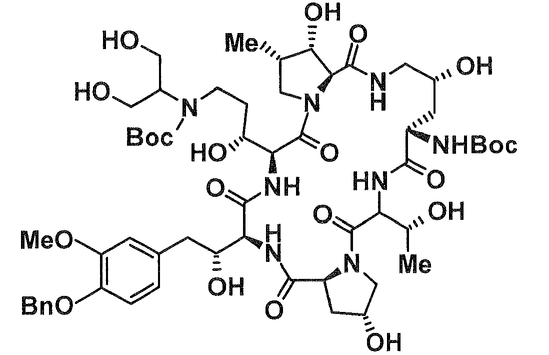 | ESI+: 1211.3 |

**[Table 8]**

| | | | |
|---|---|---|---|
| 32 | 32 | 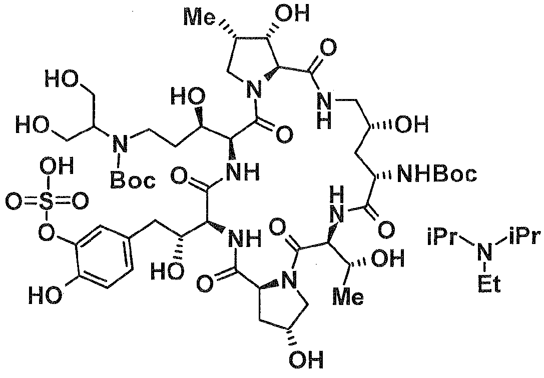 | ESI-: 1163.3 |
| 33 | 33 | 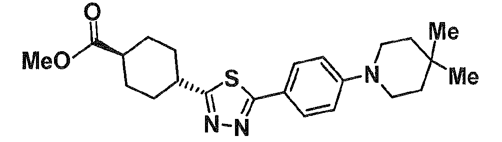 | ESI+: 413.6 |
| 34 | 34 | 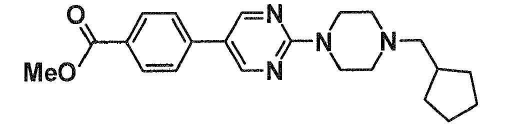 | ESI+: 380 |
| 35 | 35 | 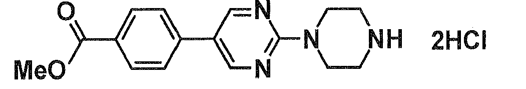 | ESI-: 297 |
| 36 | 36 | 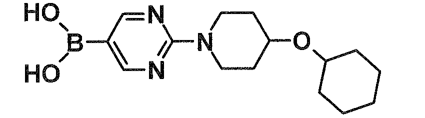 | ESI+: 306.3 |
| 37 | 20 | 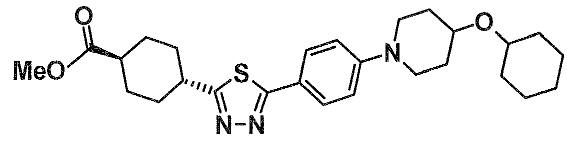 | ESI+: 484 |
| | 38 3 | 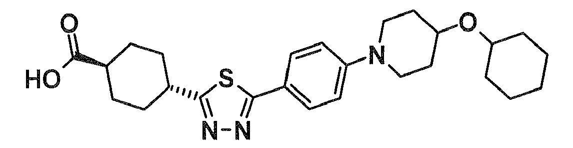 | ESI-: 468 |

**[Table 9]**

| | | | |
|---|---|---|---|
| 39 | 3 | 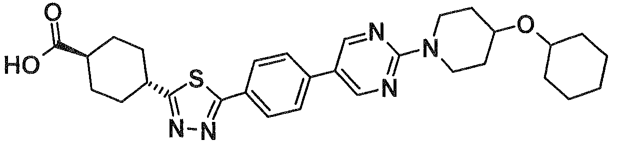 | ESI+: 548.3 |
| 40 | 2 | 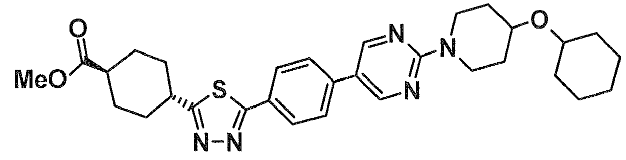 | ESI+:562.7 |
| 41 | 20 | 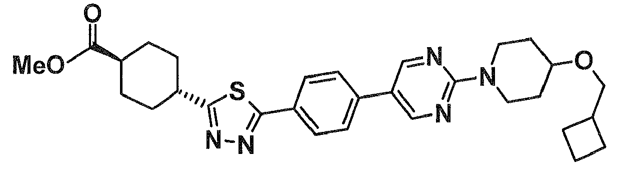 | ESI+: 548.1 |
| 42 | 13 | 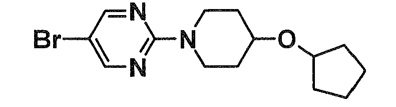 | ESI+: 326.3, 328.3 |
| 43 | 6 | 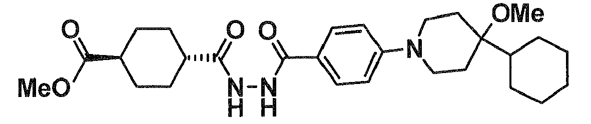 | ESI+: 522.2 |
| 44 | 3 | 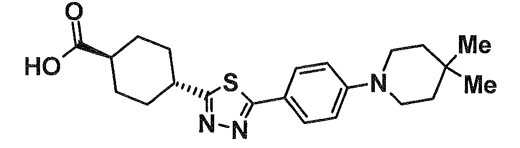 | ESI-: 398.2 |
| 45 | 6 | 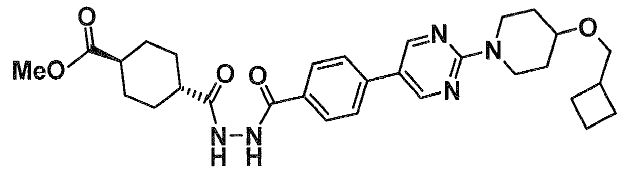 | ESI+: 550.45 |
| 46 | 3 | 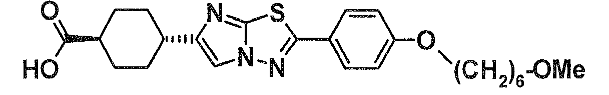 | ESI+: 458 |

**[Table 10]**

| | | | |
|---|---|---|---|
| 47 | 17 | 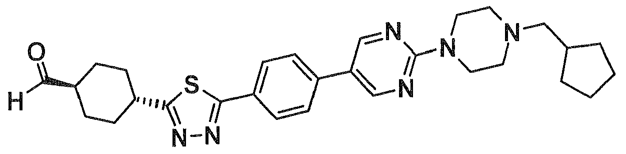 | ESI-: 515.2 |
| 48 | 2 | 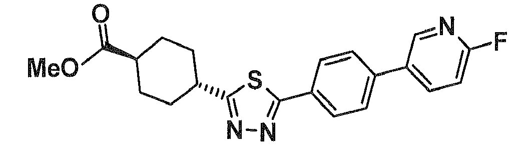 | ESI+: 398.4 |
| 49 | 13 | 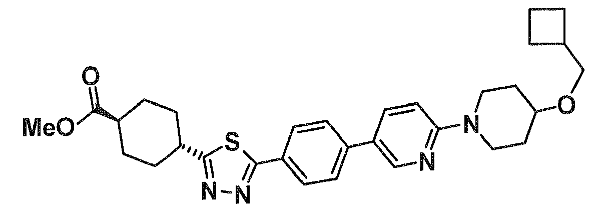 | ESI+: 547.3 |
| 50 | 2 | 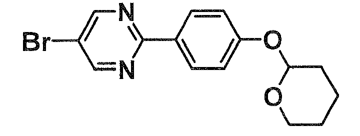 | ESI+: 335.0, 337.0 |
| 51 | 13 | 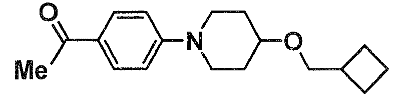 | ESI+: 288 |
| 52 | 7 | 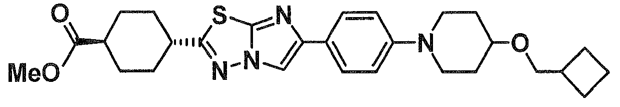 | ESI+: 509 |
| 53 | 5 | 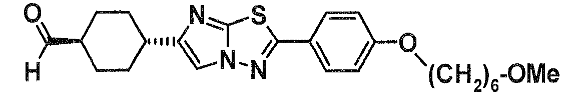 | ESI-: 440 |
| 54 | 21 | 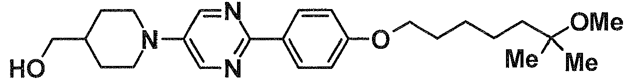 | ESI+: 428.6 |
| 55 | 17 | 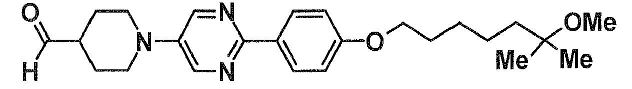 | ESI+: 426.6 |

**[Table 11]**

| | | | |
|---|---|---|---|
| 56 | 16 | 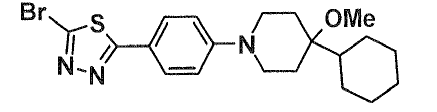 | ESI+: 436.2, 438.3 |
| 57 | 21 | 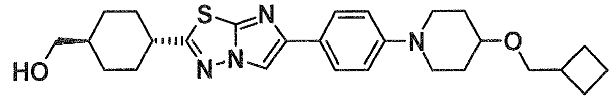 | ESI+: 481 |
| 58 | 17 | 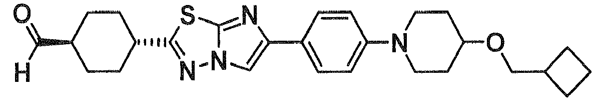 | ESI-: 477 |
| 59 | 8 | 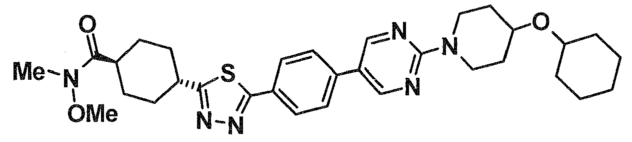 | ESI+: 591.5 |
| 60 | 17 | 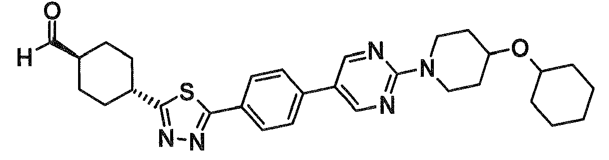 | ESI+: 532.6 |
| 61 | 18 | 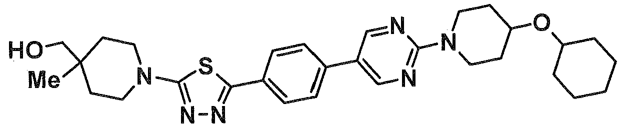 | ESI+: 549.6 |
| 62 | 17 | 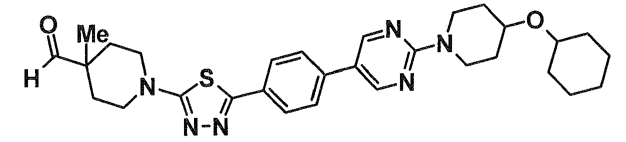 | ESI+: 547.3 |
| 63 | 8 | 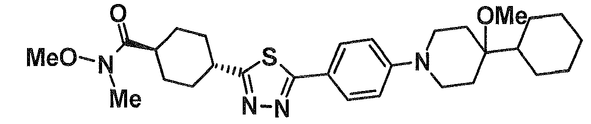 | ESI+: 527.3 |
| 64 | 14 | 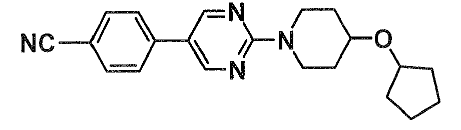 | ESI+: 349.3 |

**[Table 12]**

| | | | |
|---|---|---|---|
| | 65 9 | 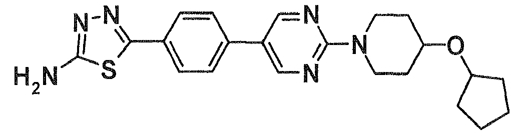 | ESI+: 423.3 |
| 66 | 16 | 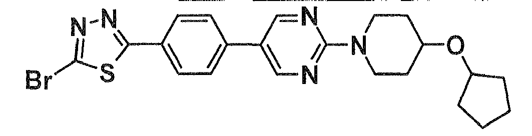 | ESI+: 486.6, 488.6 |
| 67 | 18 | 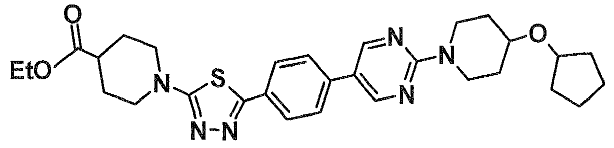 | ESI+: 563.4 |
| 68 | 21 | 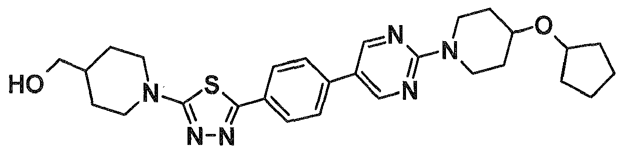 | ESI+: 521.5 |
| 69 | 17 | 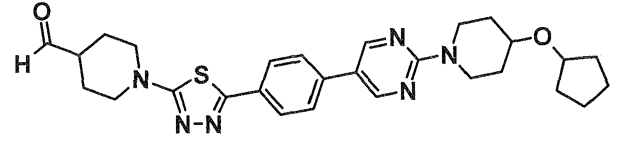 | ESI+: 519.3 |
| 70 | 8 | 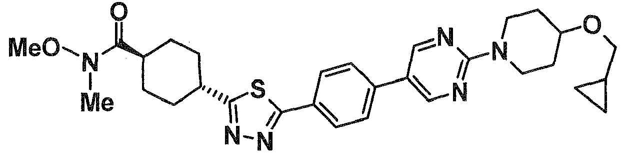 | ESI+: 563.8 |
| 71 | 22 | 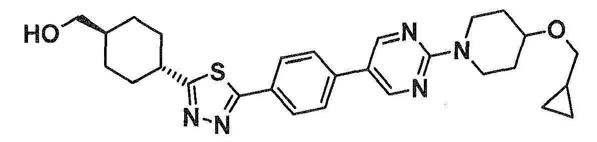 | ESI+: 506.3 |
| 72 | 17 | 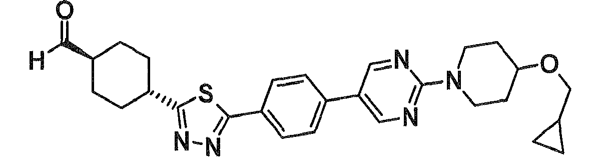 | ESI+: 504.7 |

**[Table 13]**

| | | | |
|---|---|---|---|
| 73 | 21 | 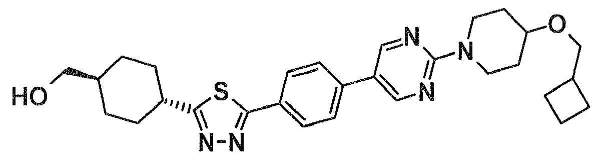 | ESI+: 520.1 |
| 74 | 17 | 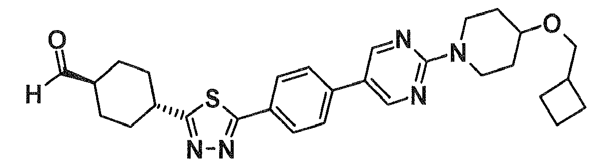 | ESI+: 518.5 |
| 75 | 17 | 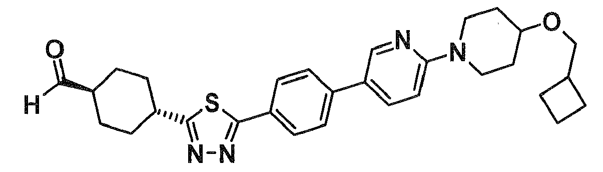 | ESI+: 517.3 |
| 76 | 21 | 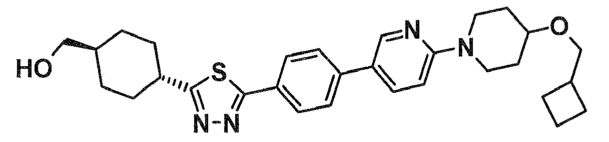 | ESI+: 519.1 |
| 77 | 8 | 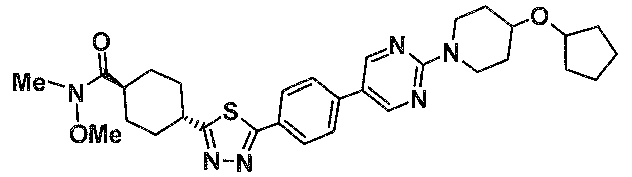 | ESI+: 577.3 |
| 78 | 5 | 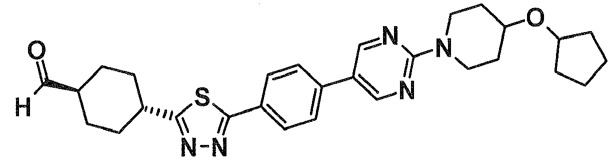 | ESI+: 518.4 |
| 79 | 8 | 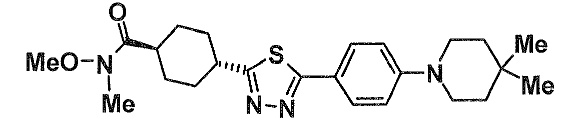 | ESI+: 443.4 |
| 80 | 5 | 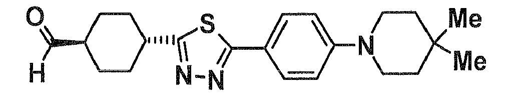 | ESI+: 384.2 |

**[Table 14]**

| | | | |
|---|---|---|---|
| 81 | 8 | 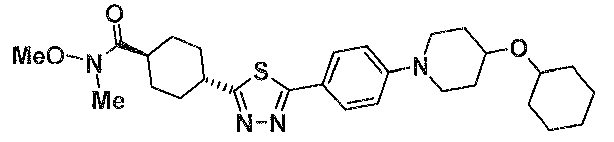 | ESI+: 513.3 |
| 82 | 5 | 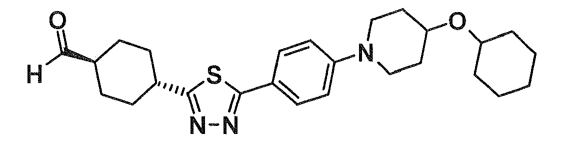 | ESI+: 454.3 |
| 83 | 18 | 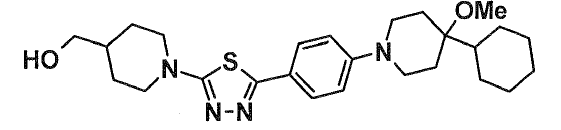 | ESI+: 471.4 |
| 84 | 17 | 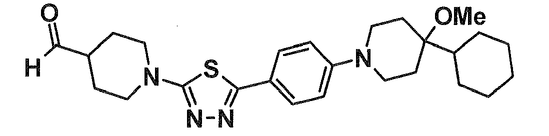 | ESI+: 469.4 |
| 85 | 8 | 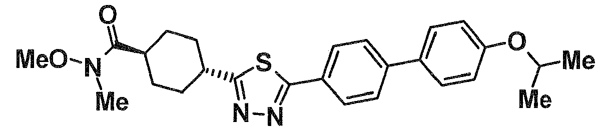 | ESI+: 466.3 |
| 86 | 20 | 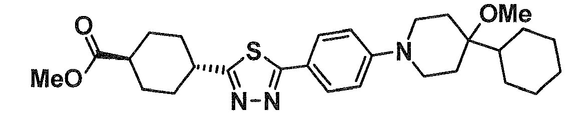 | ESI+: 498.2 |
| 87 | 20 | 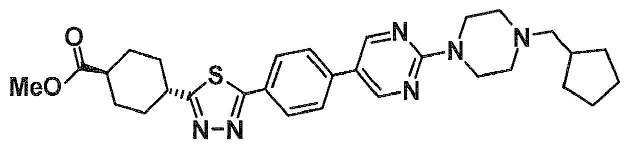 | ESI+: 547 |
| 88 6 | | 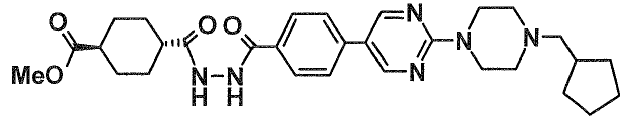 | ESI+: 549 |
| 89 | 21 | 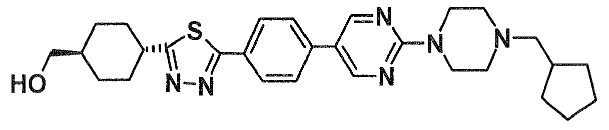 | ESI+: 521.1 |

**[Table 15]**

| | | | |
|---|---|---|---|
| 90 | 30 | 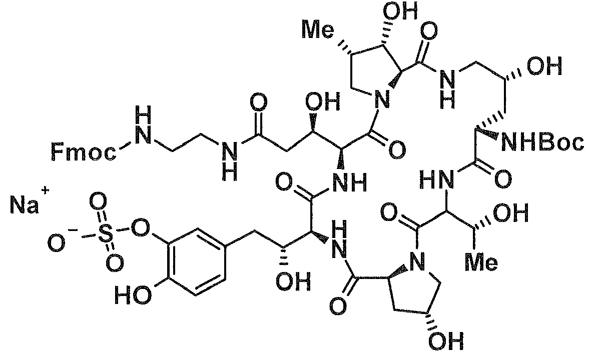 | ESI-: 1269.5 |
| 91 | 25 | 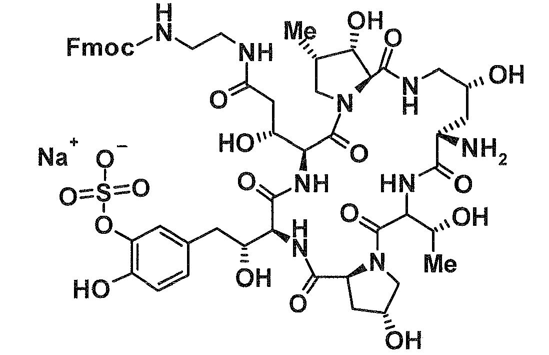 | ESI-: 1169.5 |
| 92 | 25 | 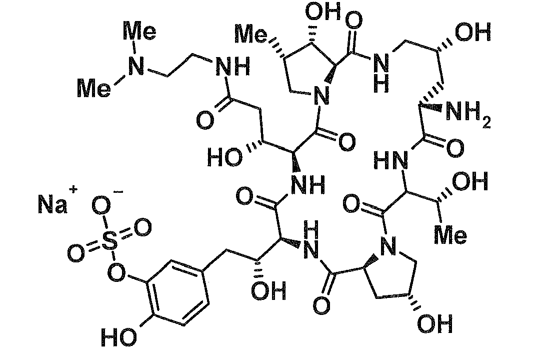 | ESI-: 974.7 |

**[Table 16]**

| | | | |
|---|---|---|---|
| 93 | 30 | 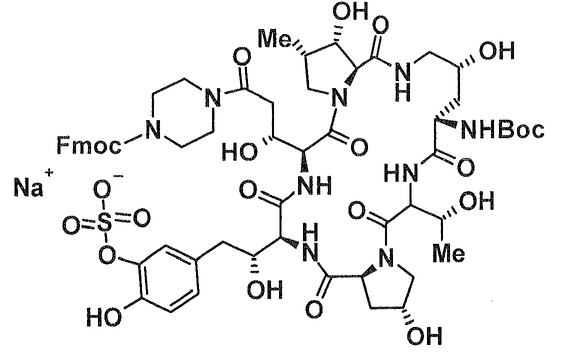 | ESI-: 1295.1 |
| 94 | 25 | 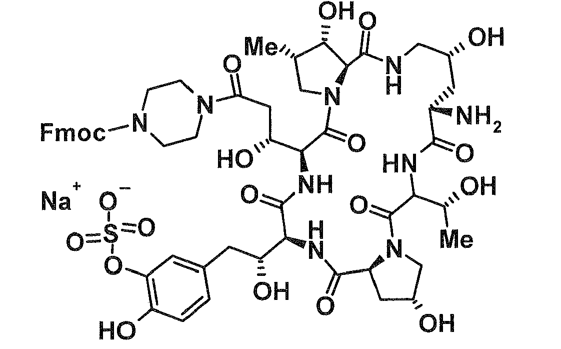 | ESI-: 1194.4 |
| 95 | 30 | 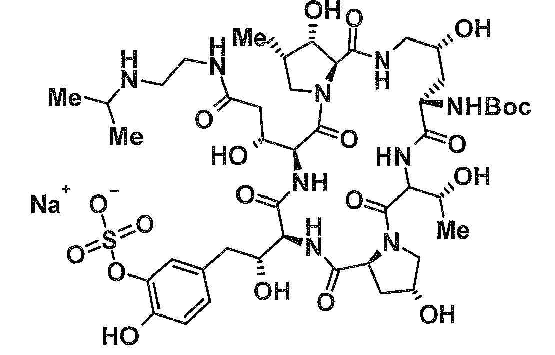 | ESI-: 1088.4 |

**[Table 17]**

| | | | |
|---|---|---|---|
| 96 | 25 | 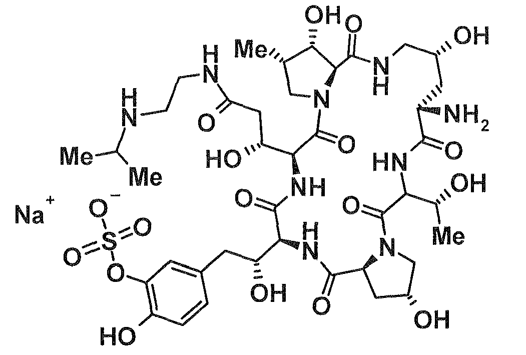 | ESI-: 988.4 |
| 97 | 30 | 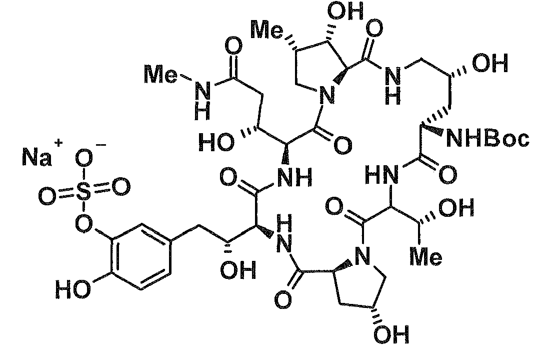 | ESI-: 1017.7 |
| 98 | 25 | 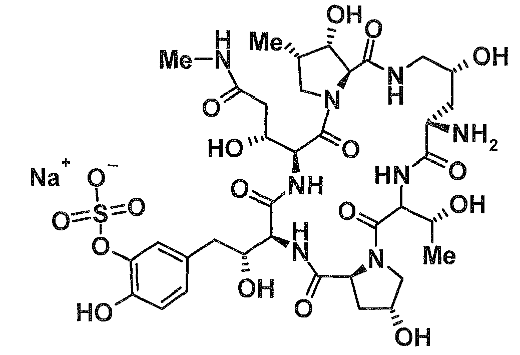 | ESI-: 917.4 |

**[Table 18]**

| | | | |
|---|---|---|---|
| 99 | 30 | 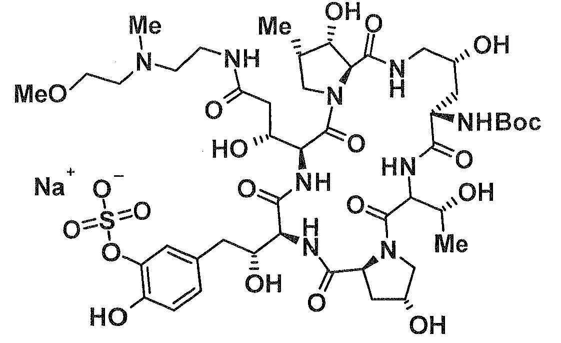 | ESI-: 1118.3 |
| 100 | 25 | 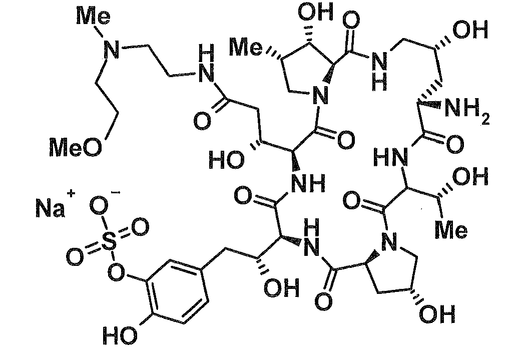 | ESI-: 1019.1 |
| 101 | 24 | 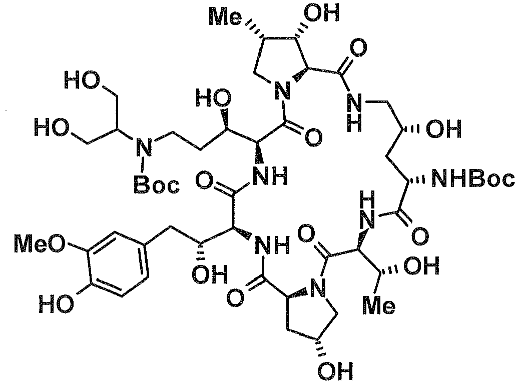 | ESI+: 1121.3 |

**[Table 19]**

| | | | |
|---|---|---|---|
| 102 | 28 | 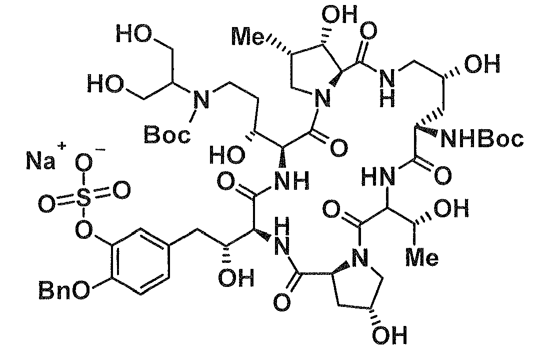 | ESI-: 1253.5 |
| 103 | C | 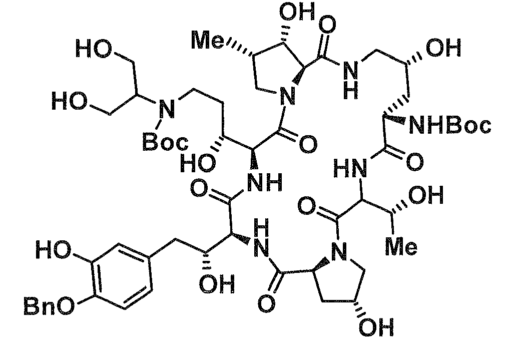 | ESI+: 1197.4 |
| 104 | 25 | 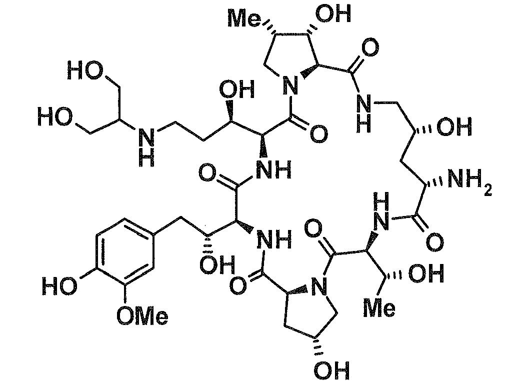 | ESI+: 899.3 |

**[Table 20]**

| Pre | Syn | Str | Dat |
|---|---|---|---|
| 105 | 3 | 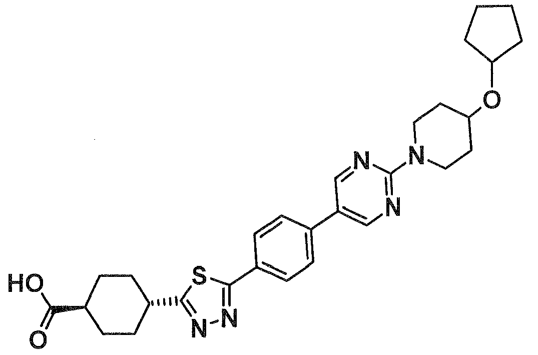 | NMR1:1.30-2.40 (22H, m), 3.20-3.70 (3H, m), 4.00-4.30 (3H, m), 7.83 (2H, d, J = 8.5 Hz), 7.99 (2H, d, J = 8.5 Hz), 8.78 (2H, s). |
| 106 | 20 | 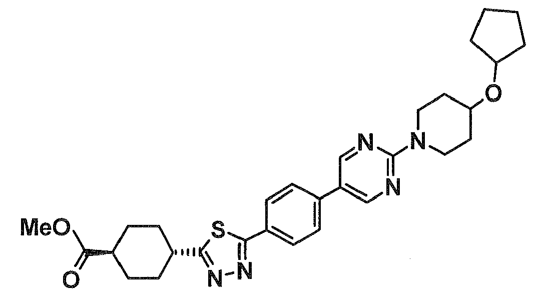 | NMR3:1.40-2.50 (22H, m), 3.10-3.80 (3H, m), 3.71 (3H, s), 4.02-4.15 (1H, m), 4.30-4.50 (2H, m), 7.58 (2H, d, J = 8.3 Hz), 7.99 (2H, d, J = 8.3 Hz), 8.58 (2H, s) |
| 107 | 6 | 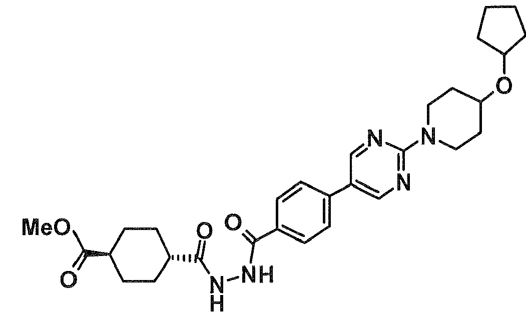 | NMR1:1.25-2.30 (22H, m), 3.25-3.70 (3H, m), 3.60 (3H, s), 4.00-4.12 (1H, m), 4.20-4.40 (2H, m), 7.78 (2H, d, J = 8.4 Hz), 7.94 (2H, d, J = 8.4 Hz), 8.78 (2H, s), 9.83 (1H, br s), 10.3 (1H, br s) |

**[Table 21]**

| | | | |
|---|---|---|---|
| 108 | 13 | 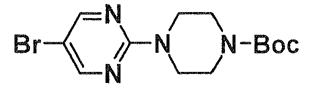 | ESI+:365,367 |
| 109 | 14 | 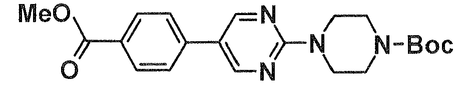 | ESI+: 421 |
| 110 | 3 | 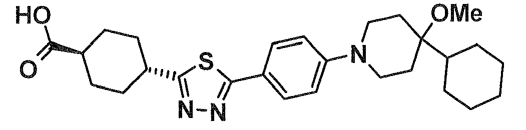 | ESI-: 482.4 |
| 111 | 9 | 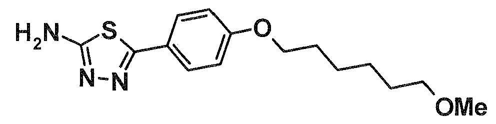 | ESI+: 308 |
| 112 | 6 | 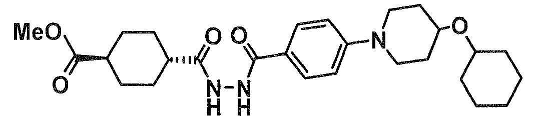 | ESI+: 508 |
| 113 | 5 | 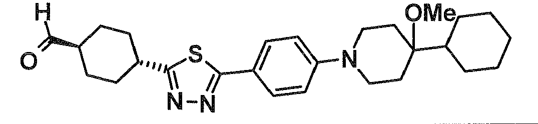 | ESI+: 468.2 |
| 114 | 9 | 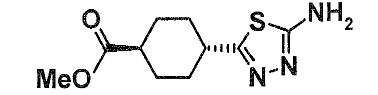 | ESI+: 242 |
| 115 | 1 | 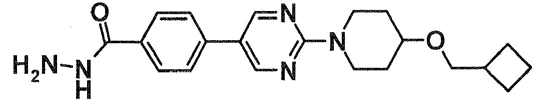 | ESI+: 382.34 |
| 116 | 14 | 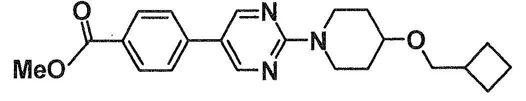 | ESI+: 382.30 |
| 117 | 13 | 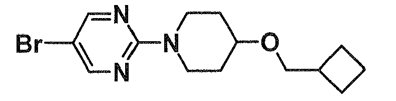 | ESI+: 326.15, 328.13 |

**[Table 22]**

| Pre | Syn | Str | Dat |
|---|---|---|---|
| 118 | 20 | 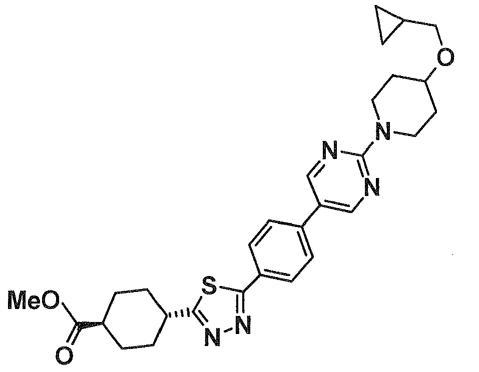 | NMR3:0.15-0.25 (2H, m), 0.48-0.63 (2H, m), 1.00-1.20 (1H, m), 1.50- 2.50 (14H, m), 3.05-3.75 (5H, m), 3.71 (3H, m), 4.30-4.50 (2H, m), 7.57 (2H, d, J = 8.3 Hz), 7.99 (2H, d, J = 8.3 Hz), 8.59 (2H, s) |
| 119 | 6 | 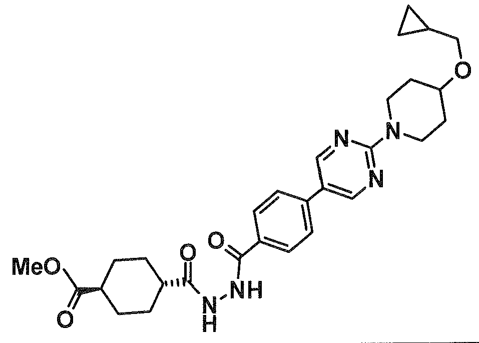 | NMR1:0.10-0.20 (2H, m), 0.40-0.52 (2H, m), 0.85-1.10 (1H, m), 1.20-2.50 (14H, m), 3.25-3.70 (8H, m), 4.15-4.35 (2H, m), 7.78 (2H, d, J = 8.4 Hz), 7.94 (2H, d, J = 8.4 Hz), 8.78 (2H, s), 9.34 (1H, br s), 10.3 (1H, br s) |
| 120 | 3 | 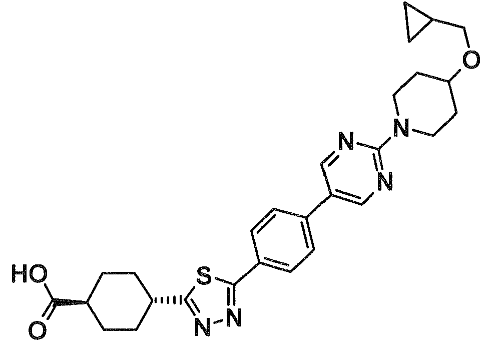 | NMR1:0.10-0.22 (2H, m), 1.40-1.55 (2H, m), 0.85-1.10 (1H, m), 1.30-2.60 (14H, m), 3.10-3.70 (5H, m), 4.15-4.33 (2H, m), 7.83 (2H, d, J = 8.2 Hz), 7.99 (2H, d, J = 8.2 Hz), 8.79 (2H, s) |

**[Table 23]**

| Pre | Syn | Str | Dat |
|---|---|---|---|
| 121 | 1 | 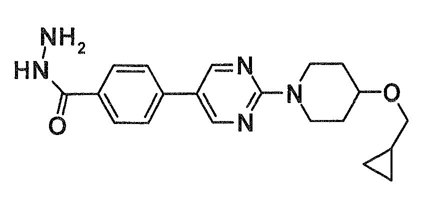 | NMR1: 0.10-0.22 (2H, m), 0.38-0.53 (2H, m), 0.87-1.10 (1H, m), 1.30-1.55 (2H, m), 1.78-1.95 (2H, m), 3.25-3.70 (5H, m), 4.15-4.33 (2H, m), 4.51 (2H, br s), 7.73 (2H, d, J = 8.4 Hz), 7.89 (2H, d, J = 8.4 Hz), 8.76 (2H, s), 9.81 (1H, br s) |
| 122 | 14 | 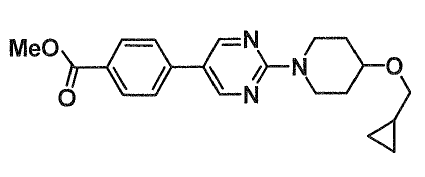 | NMR3: 0.15-0.25 (2H, m), 0.47-0.60 (2H, m), 0.97-1.18 (1H, m), 1.52-1.72 (2H, m), 1.88-2.10 (2H, m), 3.35 (2H, d, J = 6.8 Hz), 3.35-3.70 (3H, m), 3.95 (3H, s), 4.30-4.45 (2H, m), 7.54 (2H, d, J = 8.5 Hz), 8.09 (2H, d, J = 8.5 Hz), 8.58 (2H, s) |
| 123 | 1 | 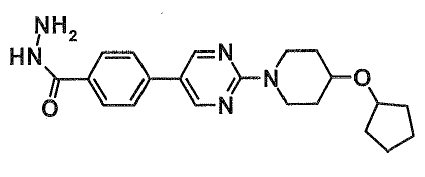 | NMR1: 1.25-2.00 (12H, m), 3.27-3.67 (3H, m), 4.00-4.12 (1H, m), 4.20-4.35 (2H, m), 4.51 (2H, br s), 7.73 (2H, d, J = 8.4 Hz), 7.89 (2H, d, J = 8.4 Hz), 8.75 (2H, s), 9.81 (1H, br s) |
| 124 | 14 | 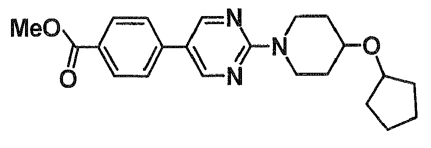 | NMR3: 1.45-2.00 (12H, m), 3.30-3.70 (3H, m), 3.94 (3H, s), 4.00-4.15 (1H, m), 4.30-4.47 (2H, m), 7.54 (2H, d, J = 8.4 Hz), 8.09 (2H, d, J = 8.4 Hz), 8.58 (2H, s) |

**[Table 24]**

| Ex | Str |
|---|---|
| 1 | 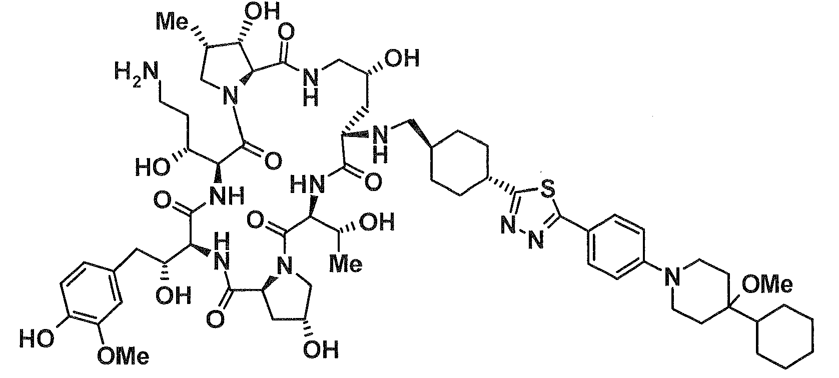 |
| 2 | 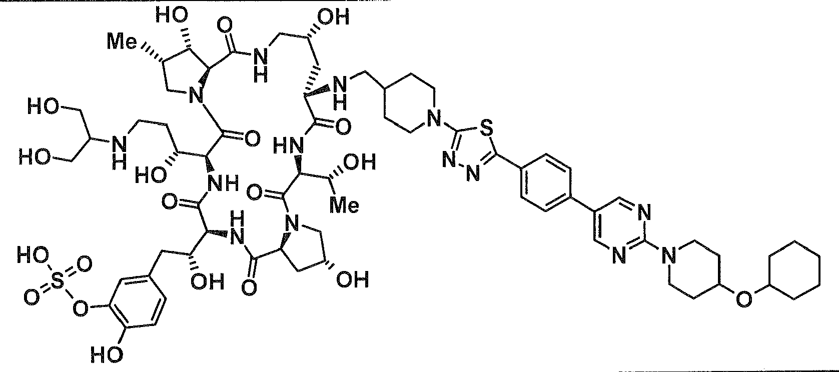 |
| 3 | 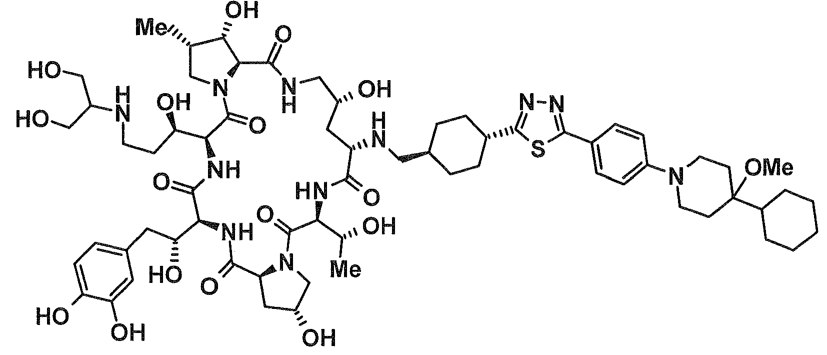 |

**[Table 25]**

| | |
|---|---|
| 4 | 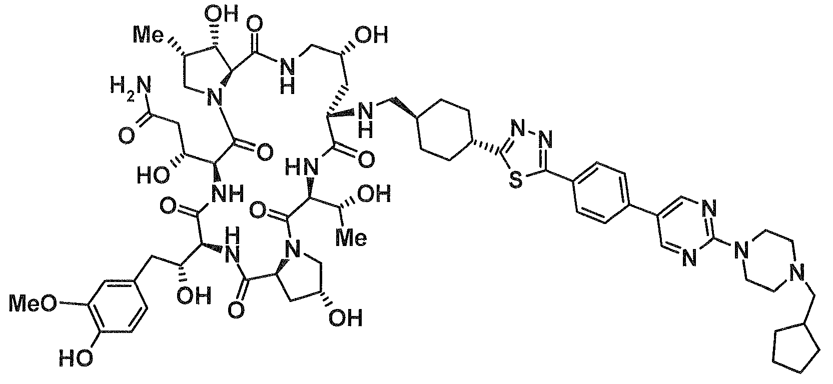 |
| 5 | 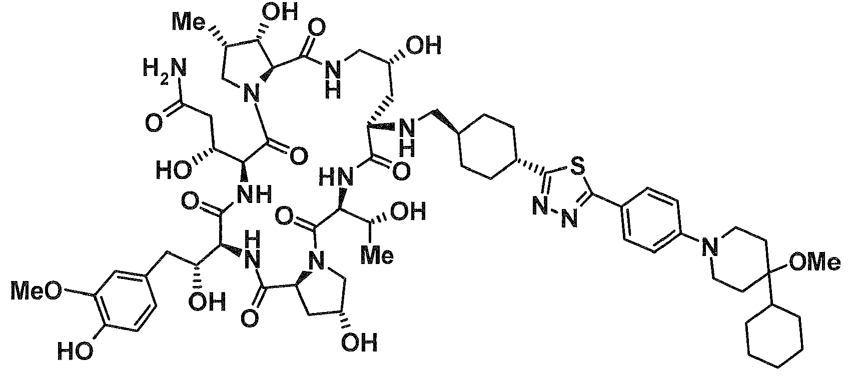 |
| 6 | 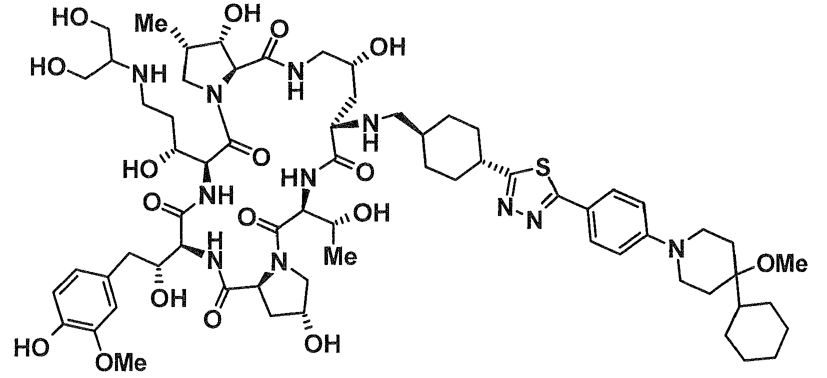 |

**[Table 26]**

| | |
|---|---|
| 7 | 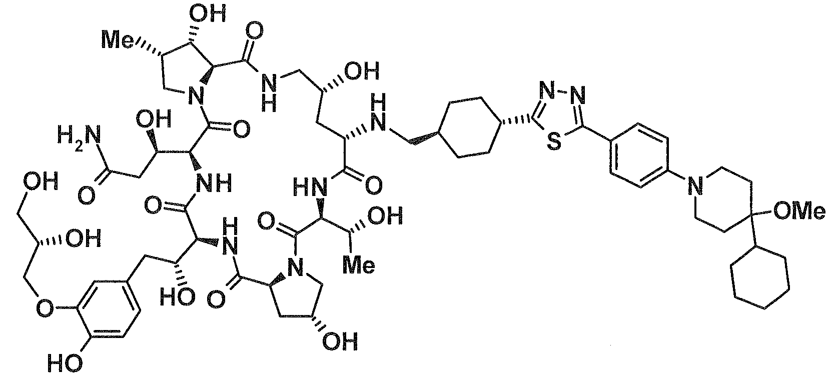 |
| 8 | 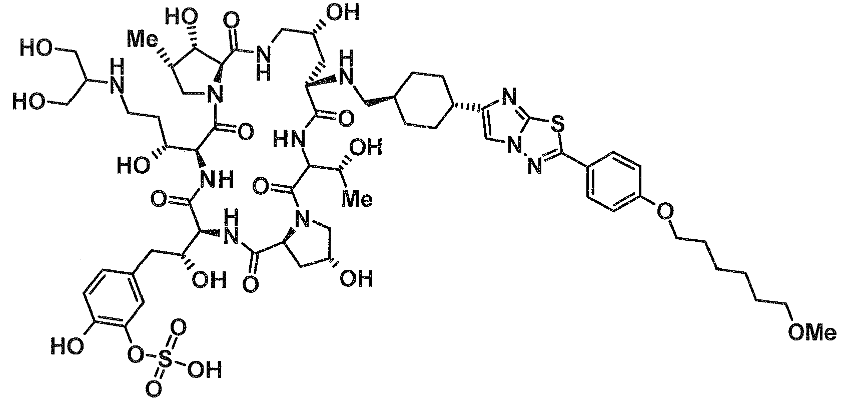 |
| 9 | 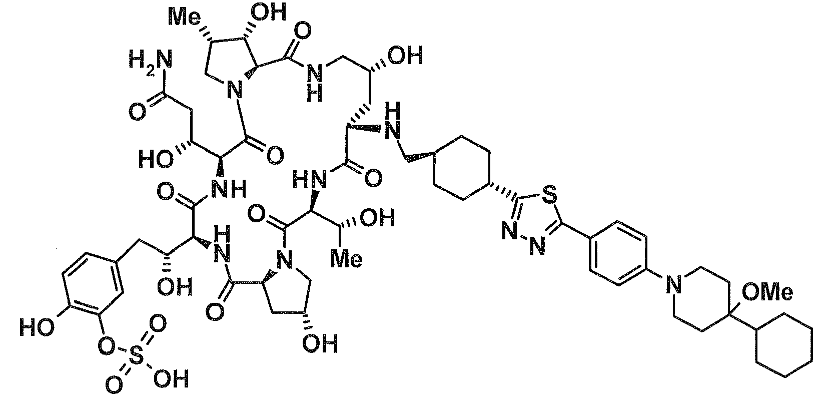 |

**[Table 27]**

| | |
|---|---|
| 10 | 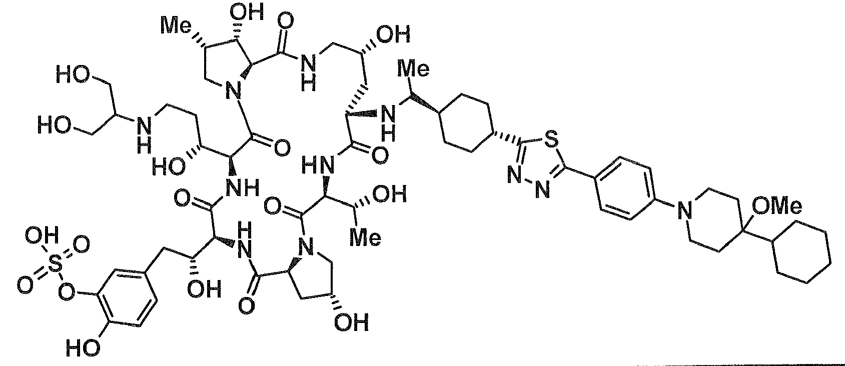 |
| 11 | 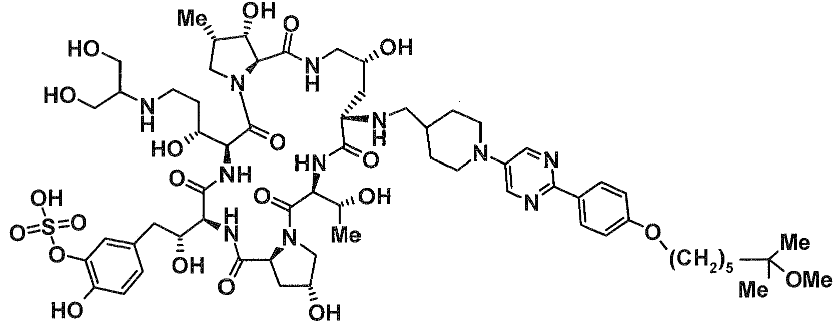 |
| 12 | 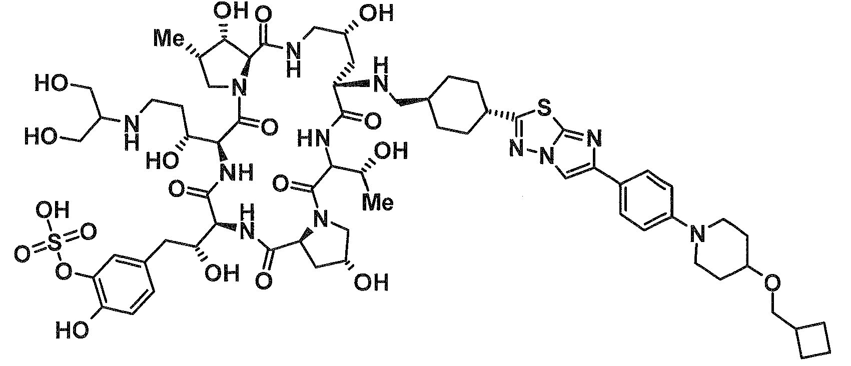 |

**[Table 28]**

| | |
|---|---|
| 13 | 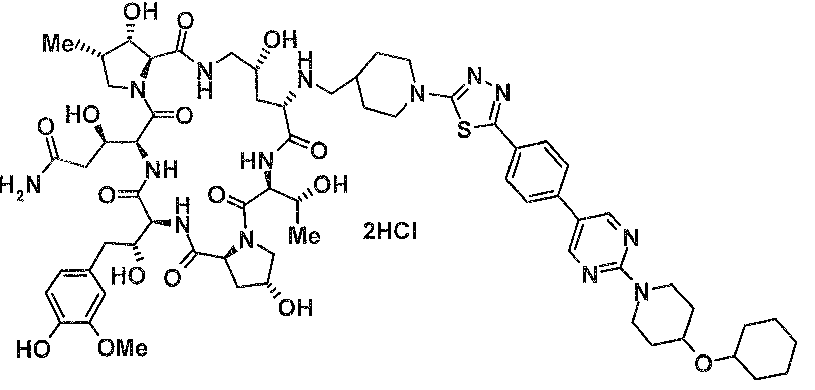 |
| 14 | 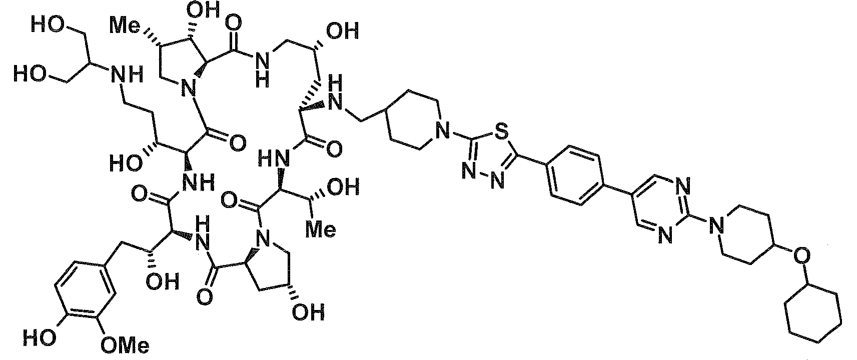 |
| 15 | 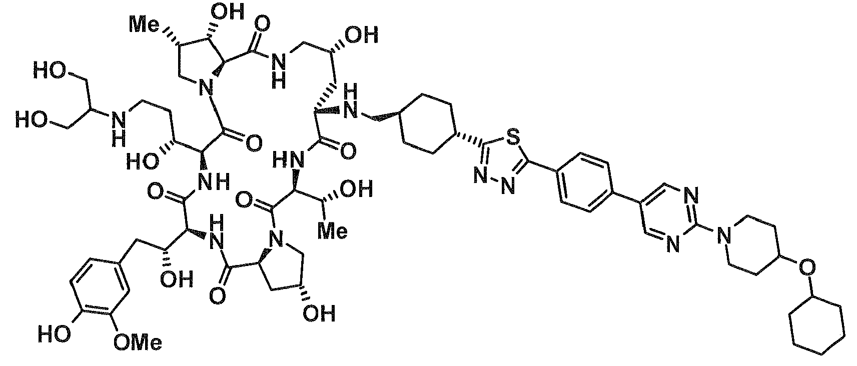 |

**[Table 29]**

| | |
|---|---|
| 16 | 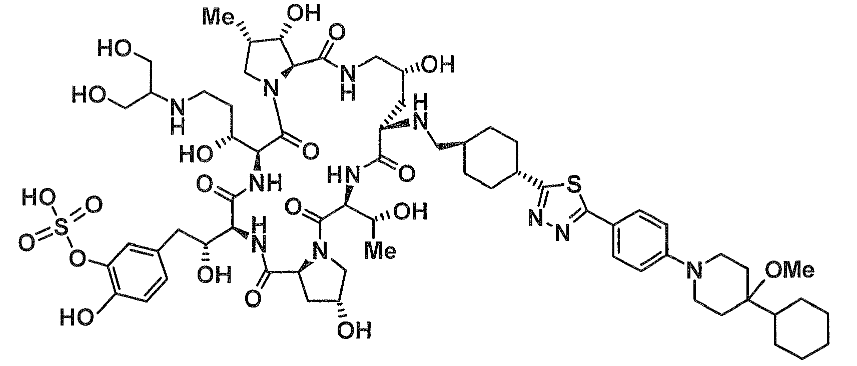 |
| 17 | 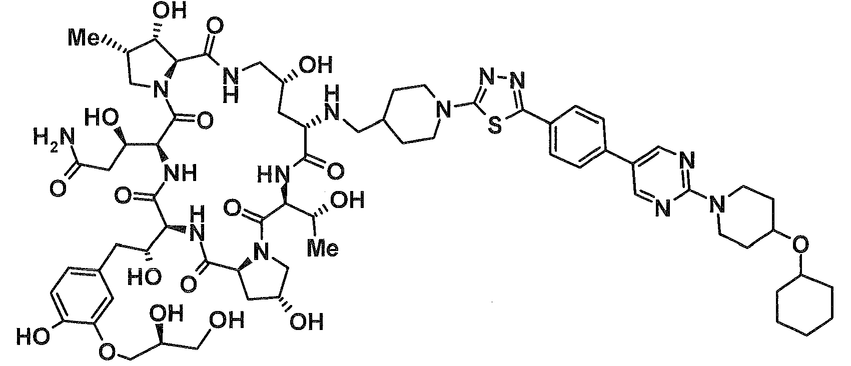 |
| 18 | 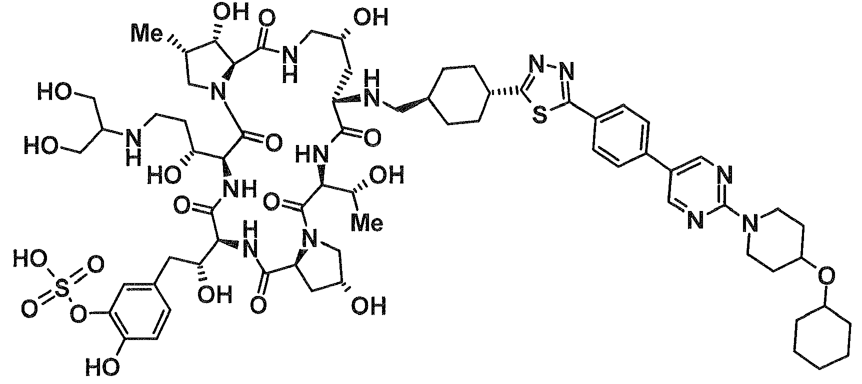 |

**[Table 30]**

| | |
|---|---|
| 19 | 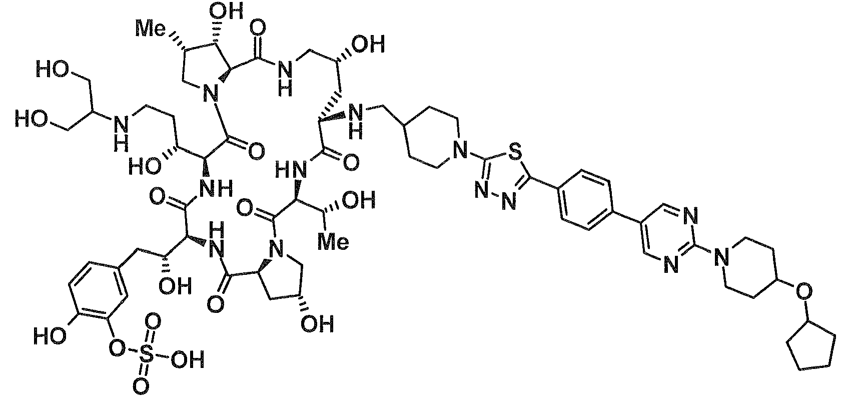 |
| 20 | 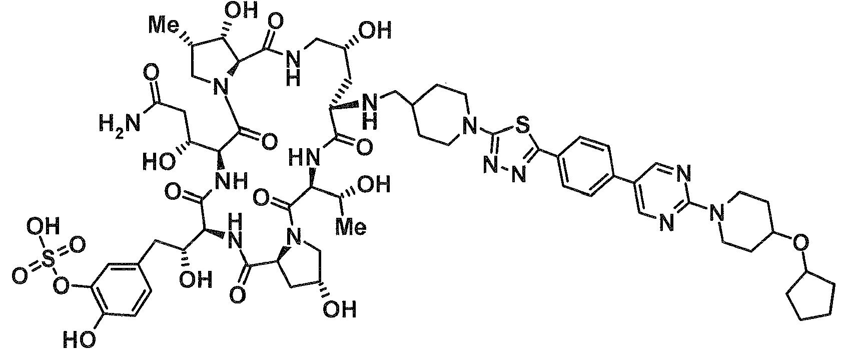 |
| 21 | 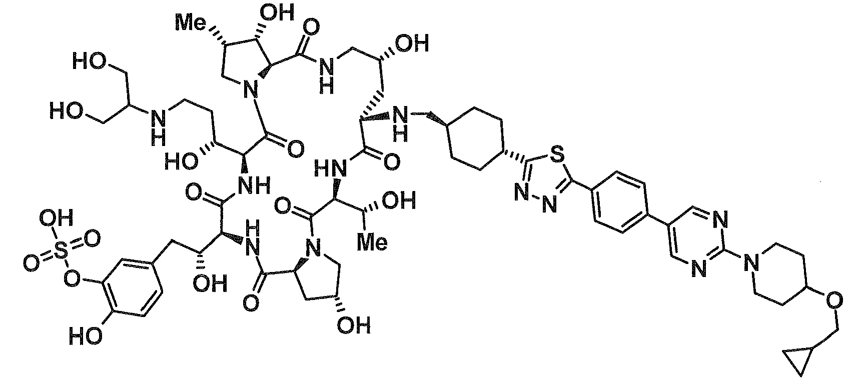 |

**[Table 31]**

| | |
|---|---|
| 22 | 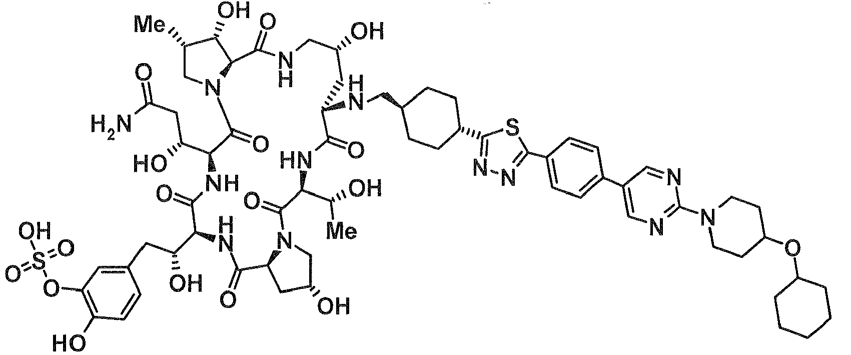 |
| 23 | 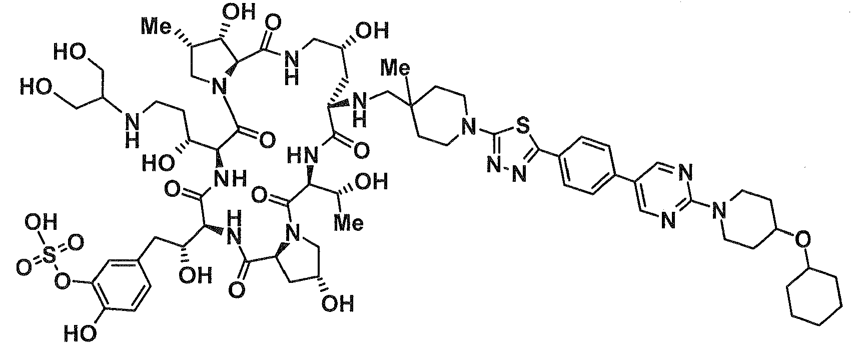 |
| 24 | 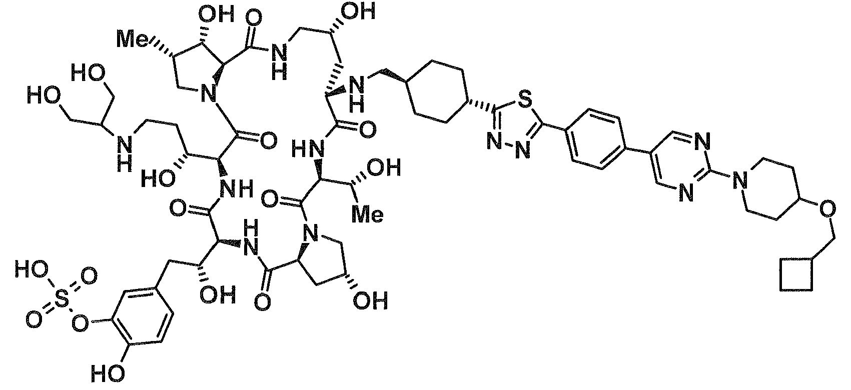 |

**[Table 32]**

| | |
|---|---|
| 25 | 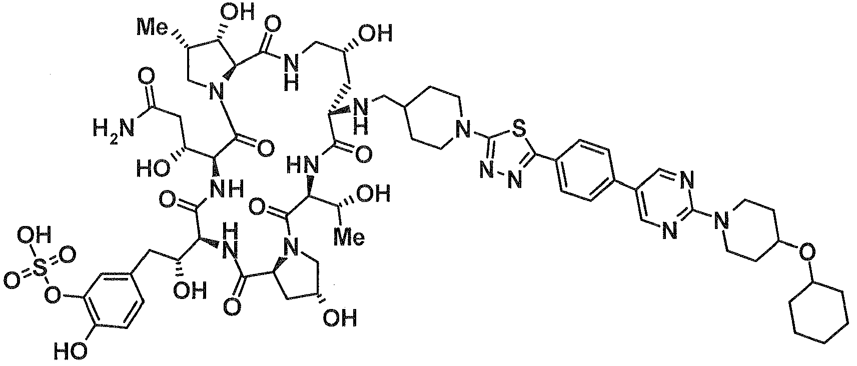 |
| 26 | 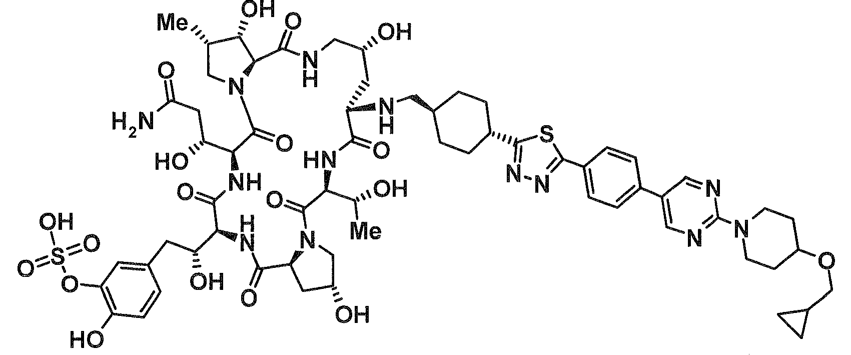 |
| 27 | 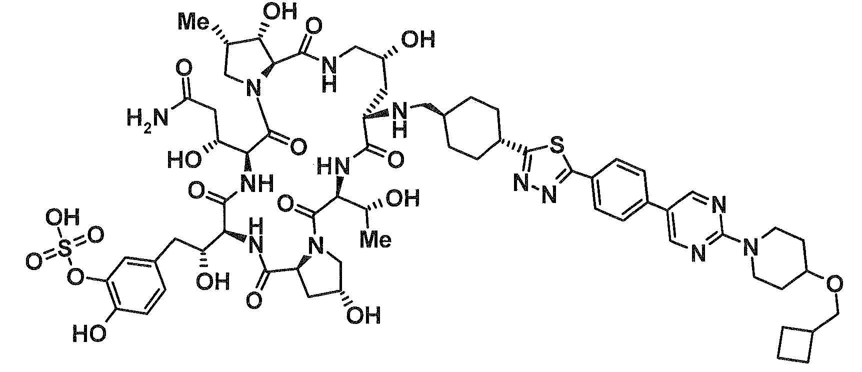 |

**[Table 33]**

| | |
|---|---|
| 28 | 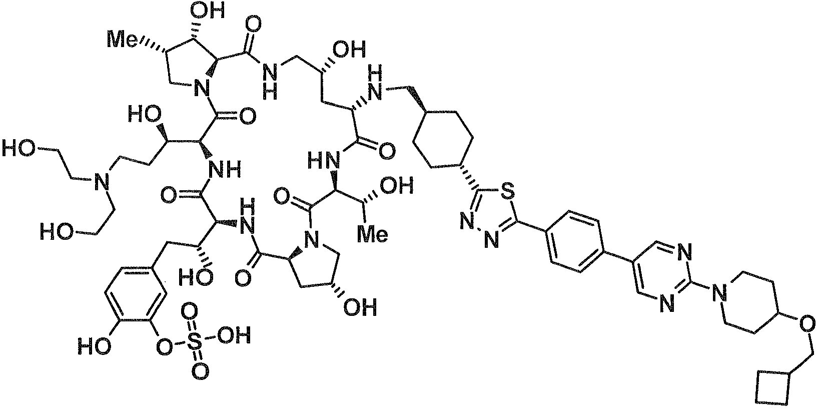 |
| 29 | 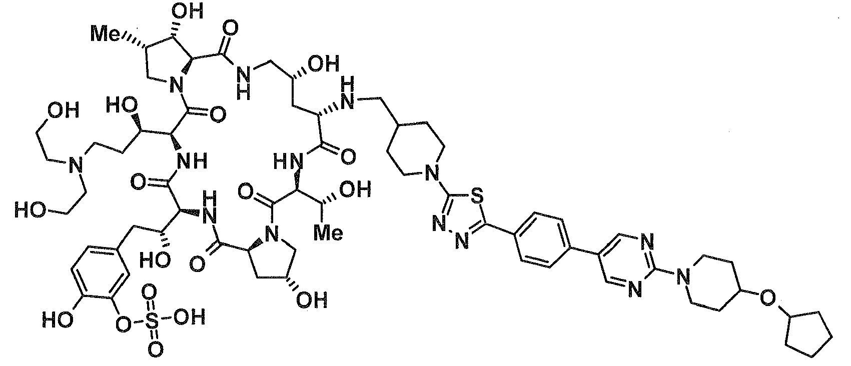 |
| 30 | 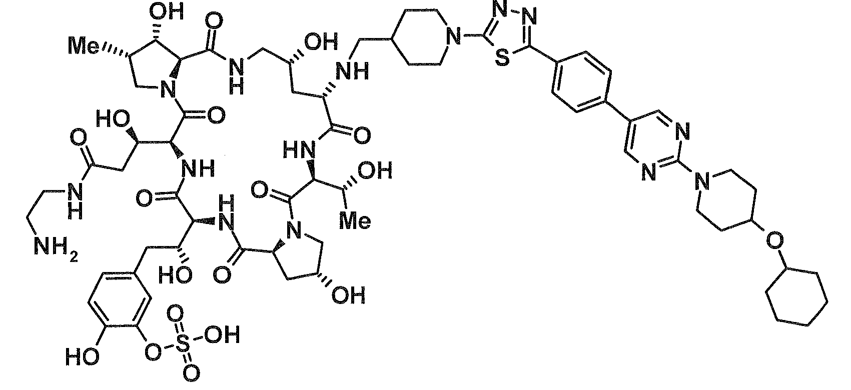 |

**[Table 34]**

| | |
|---|---|
| 31 | 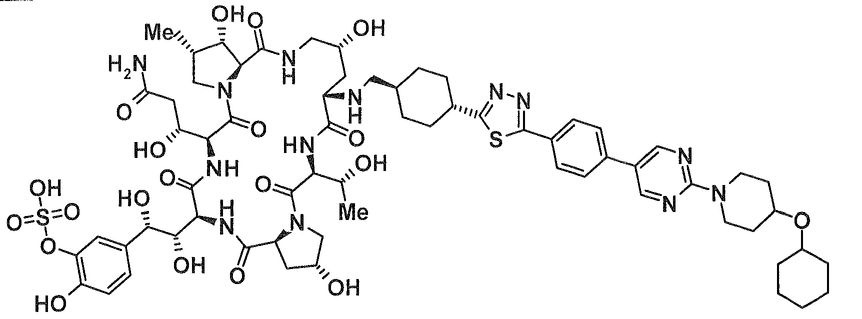 |
| 32 | 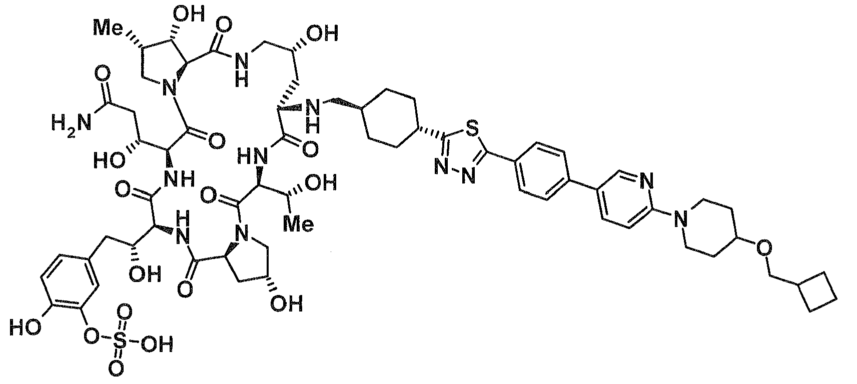 |
| 33 | 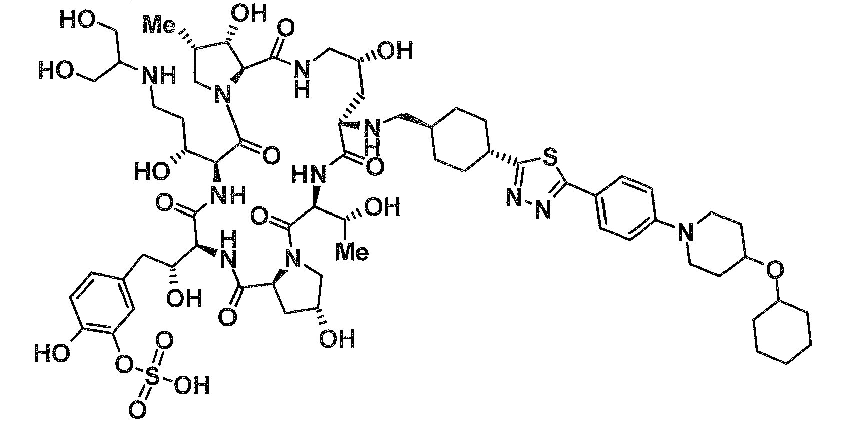 |

**[Table 35]**

| | |
|---|---|
| 34 | 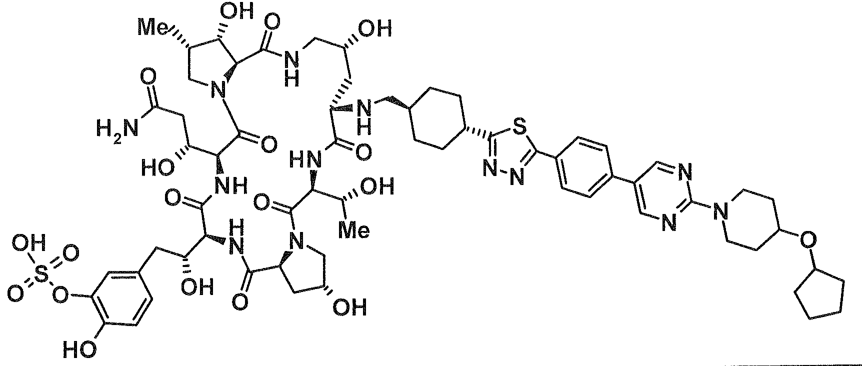 |
| 35 | 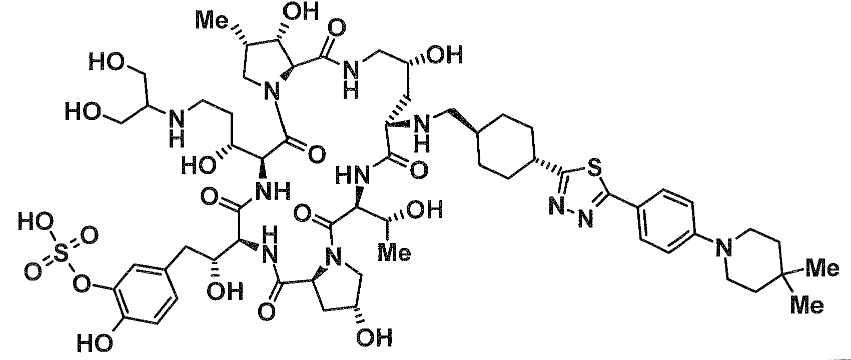 |
| 36 | 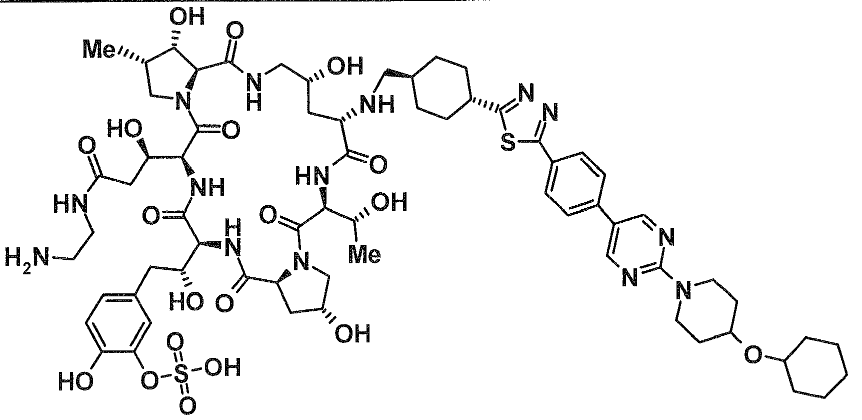 |

**[Table 36]**

| | |
|---|---|
| 37 | 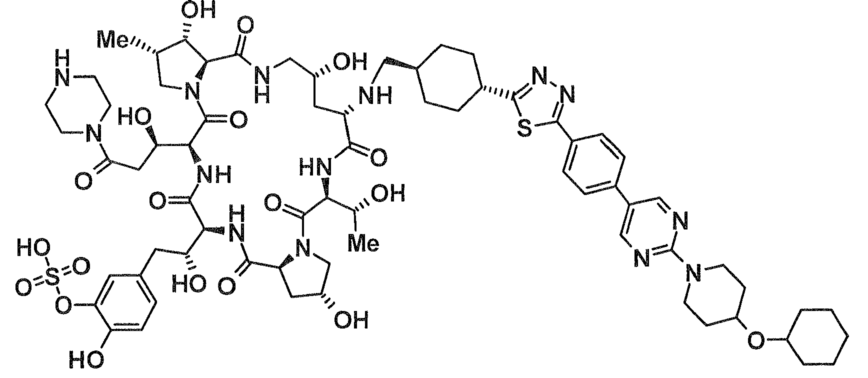 |
| 38 | 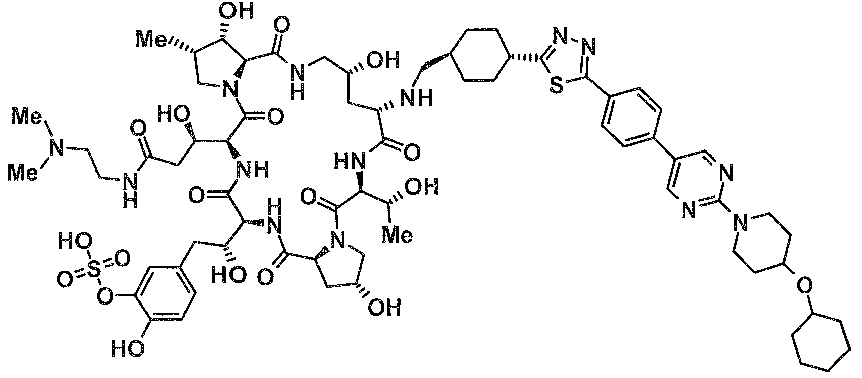 |
| 39 | 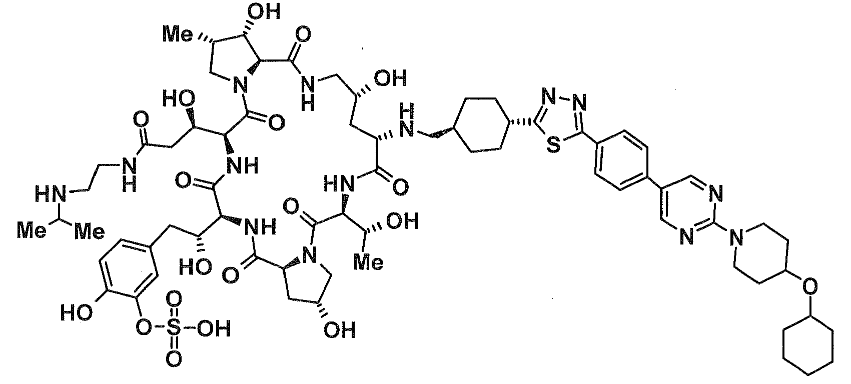 |

**[Table 37]**

| | |
|---|---|
| 40 | 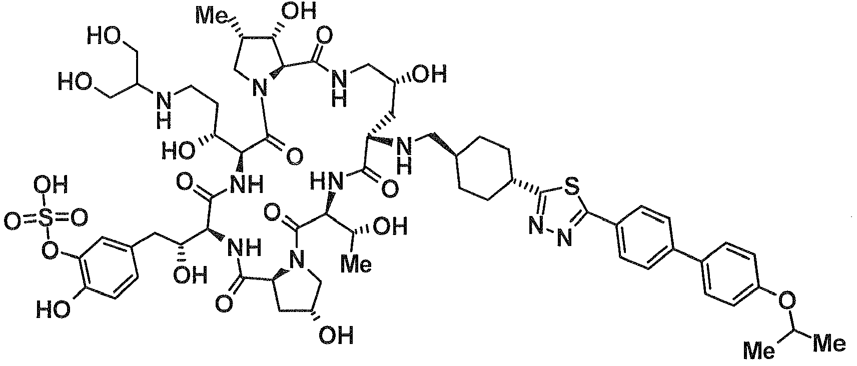 |
| 41 | 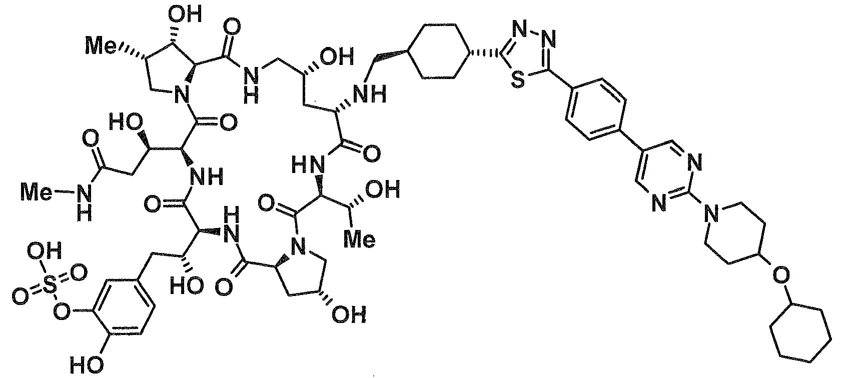 |
| 42 | 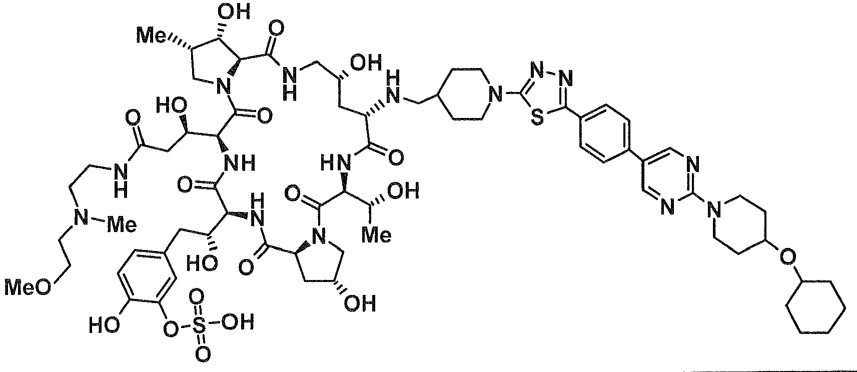 |

**[Table 38]**

| Ex | Syn | Sal | Dat |
|---|---|---|---|
| 1 | A | 2HCl | ESI+:1276.4 NMR2: 0.97 (3H, d, J = 6.8 Hz), 1.07 (3H, d, J = 5.7 Hz), 1.21- 2.53 (28H, m), 2.68-3.53 (17H, m), 3.71-3.73 (3H, m), 3.83-3.98 |
| 2 | B | - | (7H, m), 4.18-4.42 (8H, m), 4.80-4.82 (2H, m), 6.71-6.79 (2H, m), 7.03 (1H, d, J = 1.8 Hz), 7.76 (2H, d, J = 8.6 Hz), 7.72 (2H, d, J = 8.6 Hz), 8.75 (2H, s); ESI-: 1480.5 |
| 3 | C | H₂SO₄ | ESI+: 1336.4 NMR1: 0.96 (3H, d, J = 7 Hz), 1.10-1.32 (8H, m), 1.50-1.64 (8H, m), 1.81-2.54 (19H, m), 2.62-2.88 (3H, m), 3.42-4.86 (27H, m), |
| 4 | B | 2HCl | 3.72 (3H, s), 6.53-6.55 (1H, m), 6.63-6.66 (3H, m), 6.91 (1H, br), 7.30 (1H, br), 7.57 (1H, br), 7.68 (1H, br), 7.88 (2H, d, J = 8 Hz), 8.02 (2H, d, J = 8 Hz), 8.15 (1H, br), 8.32 (1H, br), 8.57 (1H, m), 8.90 (2H, s), 10.43 (1H, br); ESI+: 670.3 (M+1)/2 |
| 5 | B | - | ESI+: 1290 NMR2: 0.89-1.27 (14H, m), 1.30-2.01 (20H, m), 2.07-2.18 (3H, m), 2.20-2.94 (1H, m), 2.32-2.49 (3H, m), 2.54-2.63 (1H, m), 2.66- 2.18 (1H, m), 0.89-1.27 (14H, m), 2.90-3.07 (5H, m), 3.09 (3H, s), |
| 6 | A | 2HCl | 3.10-3.15 (1H, m), 3.22 (1H, t, J=8.0 Hz), 3.52-3.71 (8H, m), 3.74 (3H, s), 3.82-4.05 (5H, m), 4.16-4.22 (3H, m), 4.36-4.44 (3H, m), 4.78-4.82 (2H, m), 6.55-6.59 (1H, m), 6.67 (2H, d, J=8 Hz), 7.02 (2H, d, J=8.9 Hz), 7.73 (2H, d, J=8.8 Hz); ESI+: 1351.6 |
| 7 | B | HCl | ESI+: 1350.7 NMR1: 0.98 (3H, d, J = 6.8 Hz), 1.1 (3H, d, J = 5.9 Hz), 1.27-1.48 (14H, m), 1.52 (2H, m, J = 7.1 Hz), 1.64-2.58 (16H, m), 2.90-3.17 (5H, m), 3.21 (3H, s), 3.52-3.90 (10H, m), 4.06 (2H, t, J = 6.4 Hz), |
| 8 | B | - | 4.13-4.32 (4H, m), 4.33-4.49 (3H, m), 4.76-4.86 (3H, m), 4.86-5.05 (2H, m), 5.05-5.44 (8H, m), 6.71 (1H, d, J = 8.2 Hz), 6.77 (1H, dd, J = 1.8&8.4 Hz), 7.01 (1H, s), 7.11 (2H, d, J = 9.2 Hz), 7.43-7.55 (1H, bs), 7.65-7.75 (1H, bs), 7.83 (2H, d, J = 9.2 Hz), 7.88 (1H, s), 8.05-8.29 (2H, bs), 8.70 (1H, s); ESI-: 1388.5 |
| 9 | B | - | ESI-: 1355.5 |
| 10 | B | - | ESI-: 1430.5 |
| 11 | B | HCl | ESI+: 1375.1 |
| 12 | B | - | ESI-: 1425.4 |
| 13 | B | 2HCl | ESI+: 1356.4 |
| 14 | A | 3HCl | ESI+: 1415.7 |
| 15 | A | 3HCl | ESI+: 1415.5 |
| 16 | B | - | ESI-: 1414.6 |

**[Table 39]**

| | | | |
|---|---|---|---|
| 17 | B | HCl | ESI+: 1416.3 |
| 18 | B | - | ESI-: 1479.4 |
| 19 | B | - | ESI-: 1466.5 |
| 20 | B | - | ESI-: 1406.2 |
| 21 | B | - | ESI-: 1451.3 NMR2: 0.96 (3H, d, J = 6.7 Hz), 1.14 (3H, d, J = 6.0 Hz), 1.18-2.52 (33H, m), 2.58-3.63 (11H, m), 3.65-3.98 (5H, m), 4.15-4.45 (8H, m), |
| 22 | B | - | 477-4.83 (2H, m), 6.92-6.99 (2H, m), 6.99 (1H, d, J = 1.8 Hz), 7.83 (2H, d, J = 8.4 Hz), 7.99 (2H, d, J = 8.4 Hz), 8.78 (2H, s); ESI-: 1419.2 |
| 23 | B | - | ESI-: 1494.7 |
| 24 | B | - | ESI-: 1465.8 |
| 25 | B | - | ESI-: 1420.5 |
| 26 | B | - | ESI-: 1391.9 |
| 27 | B | - | ESI-: 1405.4 |
| 28 | B | - | ESI-: 1479.6 |
| 29 | B | - | ESI-: 1480.5 |
| 30 | A | - | ESI-: 1463.4 |
| 31 | B | - | ESI-: 1435.1 |
| 32 | B | - | ESI-: 1404.3 |
| 33 | B | - | ESI-: 1400.5 |
| 34 | B | - | ESI-: 1405.7 |
| 35 | B | - | ESI-: 1330.4 |
| 36 | A | - | ESI-: 1462.4 |
| 37 | A | - | ESI-: 1488.5 |
| 38 | A | - | ESI-: 1490.7 |
| 39 | A | - | EST-: 1504.7 |
| 40 | B | - | ESI-: 1353.6 |
| 41 | A | - | ESI-: 1433.3 |
| 42 | A | - | ESI-: 1535.7 |

**[Table 40]**

| Pre | Syn | Str | Dat |
|---|---|---|---|
| 125 | 125 | 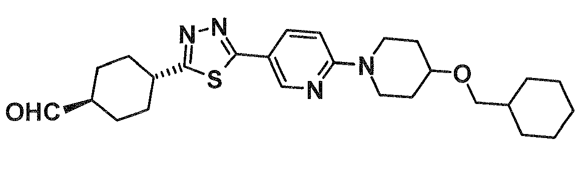 | NMR3: 0.935 (2H, q, J = 12.8 Hz), 1.11-1.32 (3H, m), 1.43-1.86 (12H, m), 1.87-2.04 (2H, m), 2.05-2.42 (7H, m), 3.11-3.20 (1H, m), 3.28 (2H, d, J = 6.4 Hz), 3.47-3.83 (3H, m), 3.93-4.02 (2H, m), 6.80-6.91 (1H, m), 8.21-8.41 (1H, m), 8.56 (1H, d, J = 2 Hz), 9.70 (1H, s) |
| 126 | 126 | 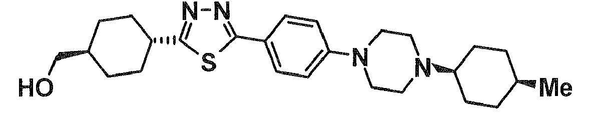 | ESI+: 485.1 |
| 127 | 127 | 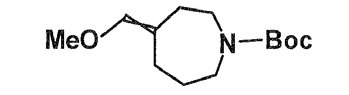 | ESI+: 264.2 |

**[Table 41]**

| | | | |
|---|---|---|---|
| 128 | 128 | 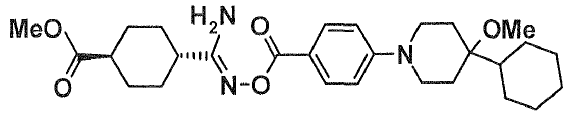 | ESI+: 500.65 |
| 129 | 129 | 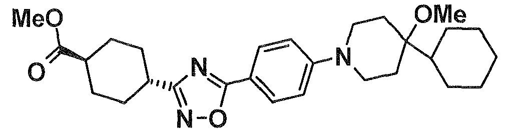 | ESI+: 482.5 |
| 130 | 130 | 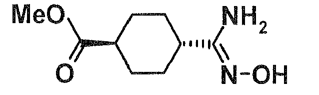 | ESI+: 201.1 |
| 131 | 131 | 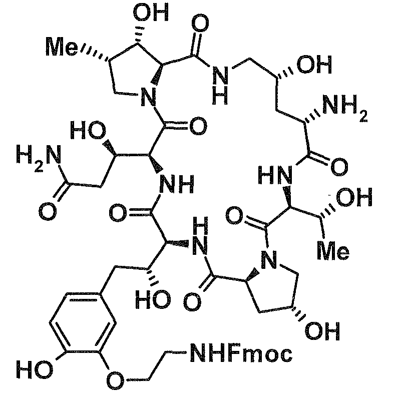 | ESI+: 1090.4 |
| 132 | 132 | 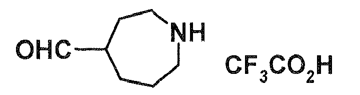 | ESI+: 128.2 |
| 133 | 133 | 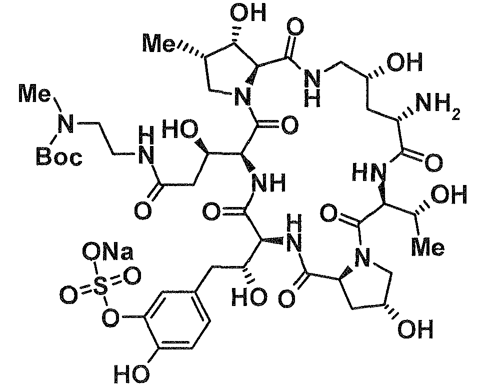 | ESI-: 1061.4 |

**[Table 42]**

| | | | |
|---|---|---|---|
| 134 | 134 | 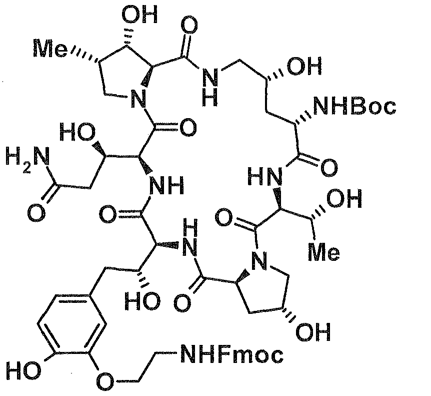 | ESI+: 1212.2 [M+Na] |
| 135 | 135 | 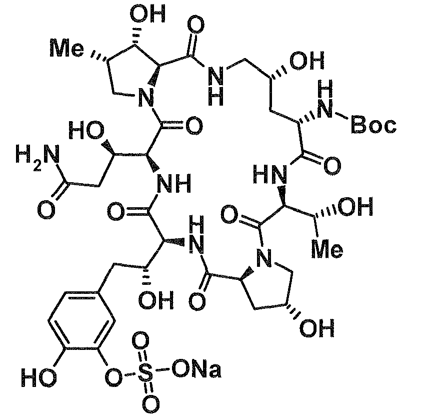 | ESI-: 1003.3 |
| 136 | 136 | 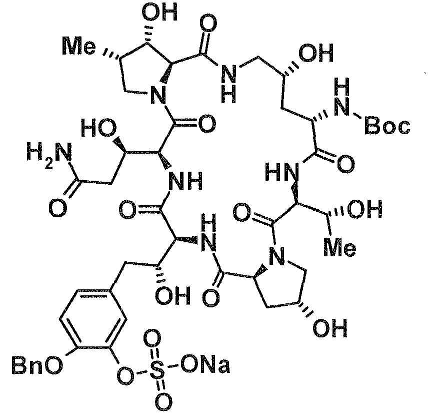 | ESI-: 1093.1 |

**[Table 43]**

| | | | |
|---|---|---|---|
| 137 | 137 | 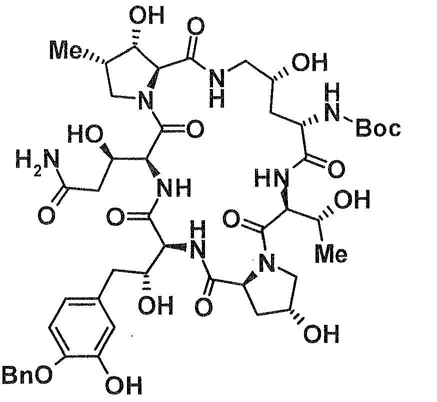 | ESI-: 1013.3 |
| 138 | 138 | 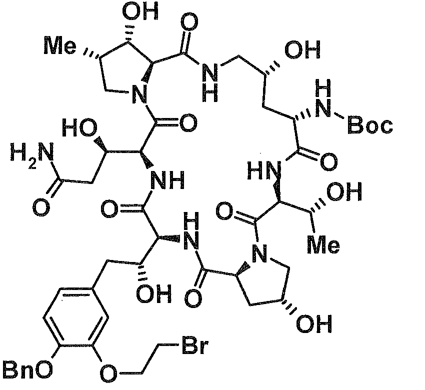 | ESI-: 1119.2, 1121.2 |
| 139 | 139 | 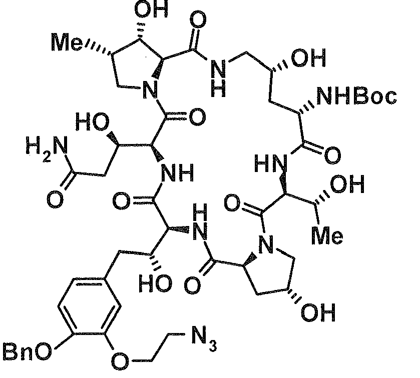 | ESI+: 1106.3 [M+Na] |

**[Table 44]**

| | | | |
|---|---|---|---|
| 140 | 140 | 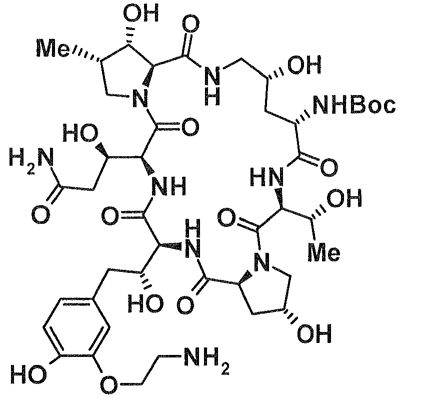 | ESI+: 495.9 [(M+H+Na)/2] |
| 141 | 141 | 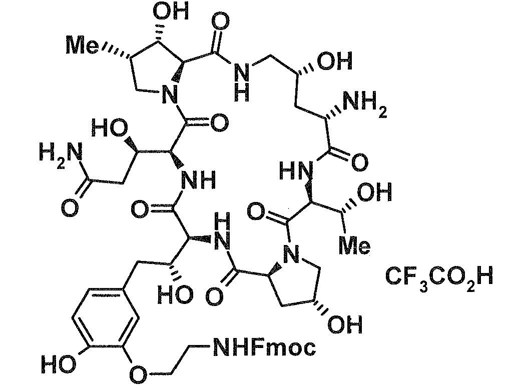 | ESI+: 1090.3 |

**[Table 45]**

| | | | |
|---|---|---|---|
| 142 | 27 | 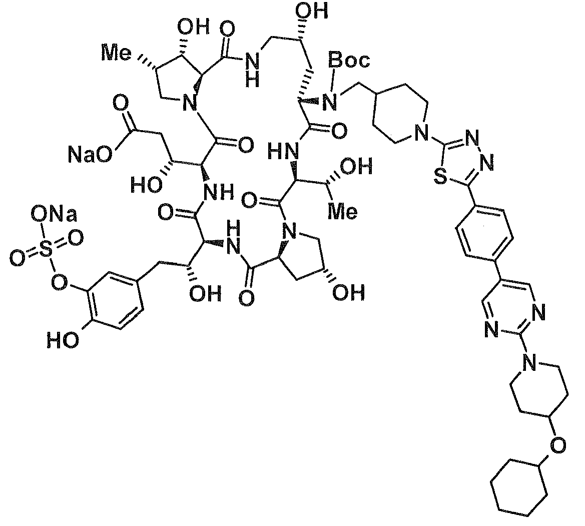 | ESI-: 1521.7 |
| 143 | 15 | 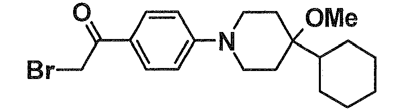 | ESI+: 395.3, 397.3 |
| 144 | 33 | 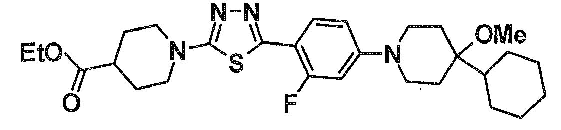 | ESI+: 531.5 |
| 145 | 33 | 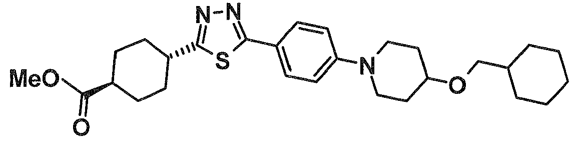 | ESI+: 498.2 |
| 146 | 33 | 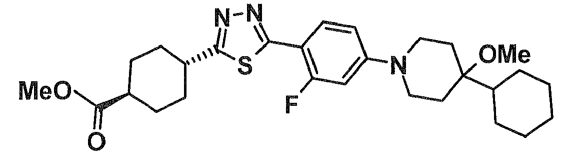 | ESI+: 516.5 |

**[Table 46]**

| | | | |
|---|---|---|---|
| 147 | 33 | 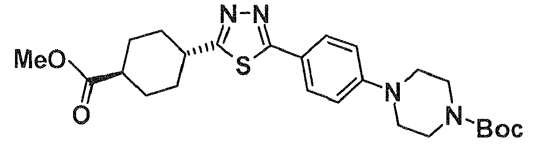 | ESI+: 487.3 |
| 148 | 5 | 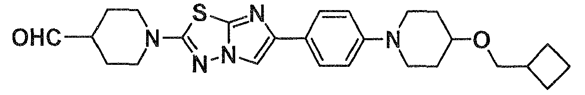 | ESI+: 480.4 |
| 149 | 5 | 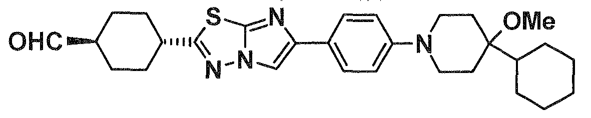 | ESI+: 507.3 |
| 150 | 125 | 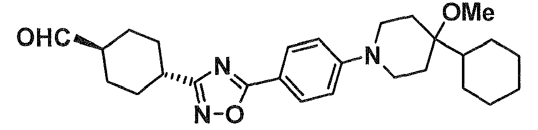 | ESI+: 452.5 |
| 151 | 125 | 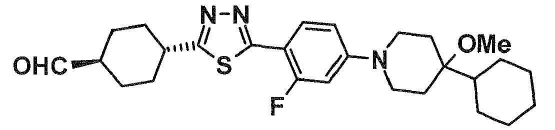 | ESI+: 486.5 |
| 152 | 125 | 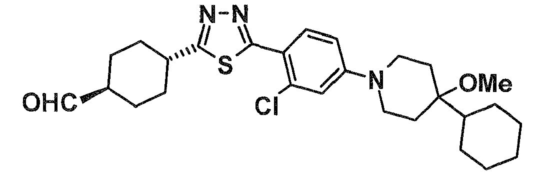 | ESI+: 502.45 |
| 153 | 125 | 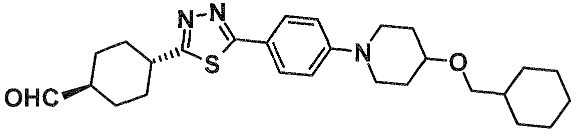 | ESI+: 468.2 |
| 154 | 21 | 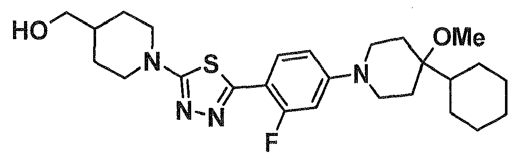 | ESI+: 489.4 |

**[Table 47]**

| | | | |
|---|---|---|---|
| 155 | 21 | 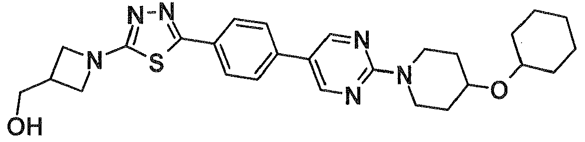 | ESI+: 507.3 |
| 156 | 21 | 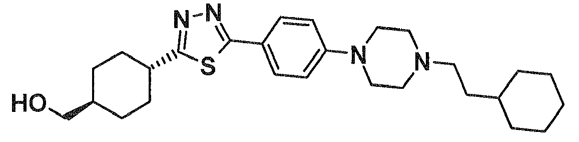 | ESI+: 469.4 |
| 157 | 21 | 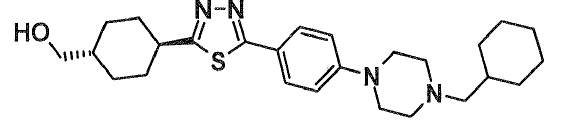 | ESI+: 455.4 |
| 158 | 21 | 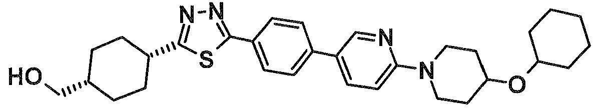 | ESI+: 533.3 |
| 159 | 34 | 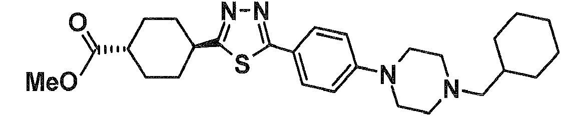 | ESI+: 483.4 |
| 160 | 34 | 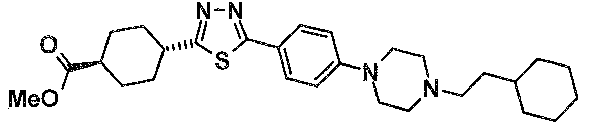 | ESI+: 497.4 |
| 161 | 18 | 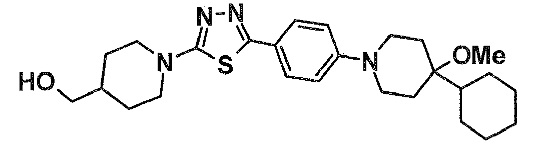 | ESI+: 471.4 |
| 162 | 18 | 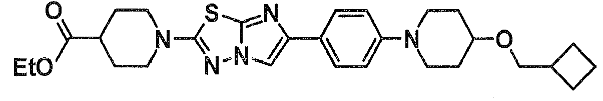 | ESI+: 524.3 |
| 163 | 18 | 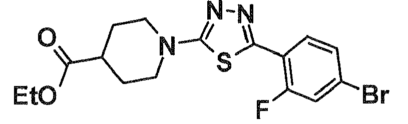 | ESI+: 415.2, 417.2 |

**[Table 48]**

| | | | |
|---|---|---|---|
| 164 | 18 | 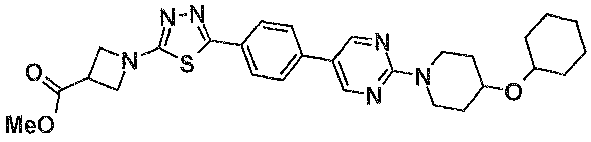 | ESI+: 535.5 |
| 165 | 18 | 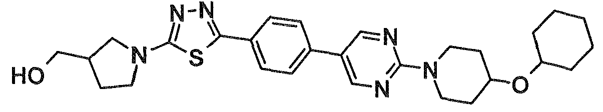 | ESI+: 521.3 |
| 166 | 18 | 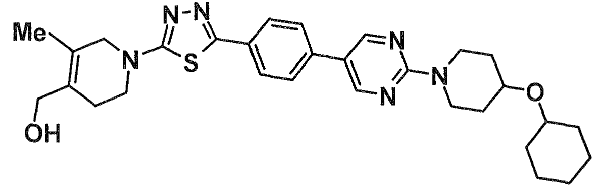 | ESI+: 547.3 |
| 167 | 18 | 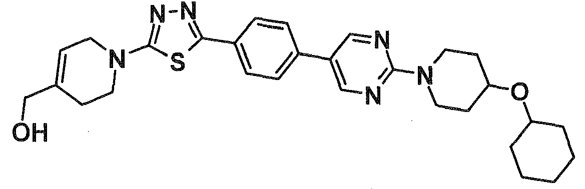 | ESI+: 532.7 |
| 168 | 13 | 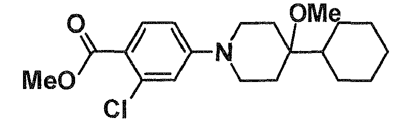 | ESI+: 366.4 |
| 169 | 13 | 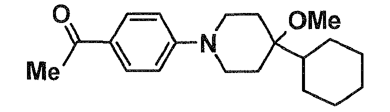 | ESI+: 316.3 |
| 170 | 13 | 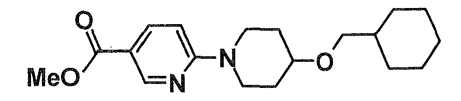 | ESI+: 333.3 |
| 171 | 13 | 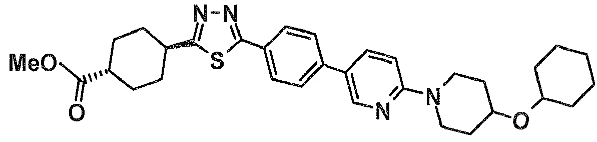 | ESI+: 561.5 |

**[Table 49]**

| | | | |
|---|---|---|---|
| 172 | 8 | 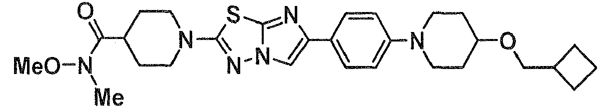 | ESI+: 539.4 |
| 173 | 8 | 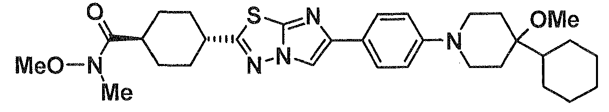 | ESI+: 566.5 |
| 174 | 9 | 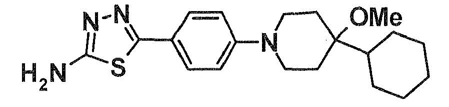 | ESI+: 373.5 |
| 175 | 9 | 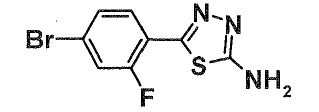 | ESI+: 274.5, 276.5 |
| 176 | 7 | 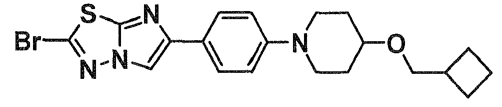 | ESI+: 448.5, 450.5 |
| 177 | 7 | 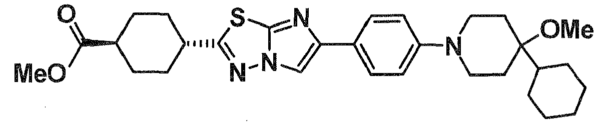 | ESI+: 537.5 |
| 178 | 16 | 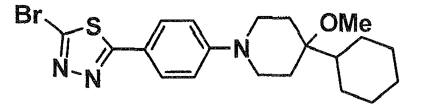 | ESI+: 436.2, 438.3 |
| 179 | 16 | 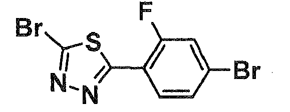 | ESI+: 338.4, 340.4 |
| 180 | 6 | 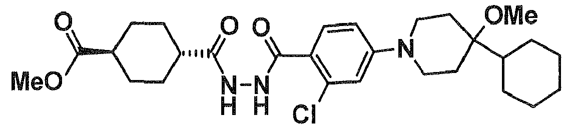 | ESI+: 535.1 |
| 181 | 6 | 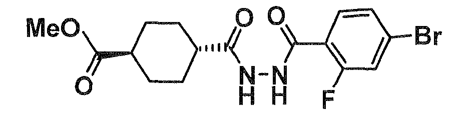 | ESI+: 401.2, 403.2 |

**[Table 50]**

| | | | |
|---|---|---|---|
| 182 | 6 | 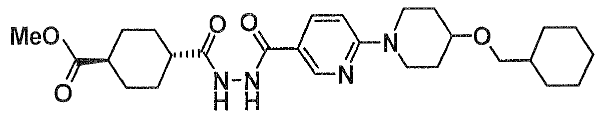 | ESI-: 499.3 |
| 183 | 6 | 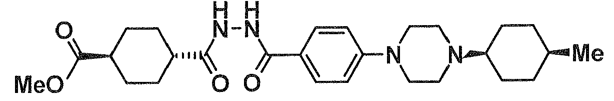 | ESI+: 484.9 |
| 184 | 20 | 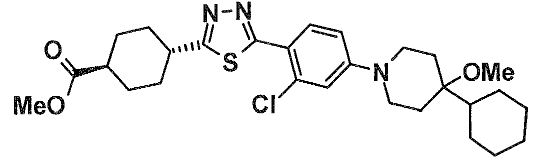 | ESI+: 532.5 |
| 185 | 20 | 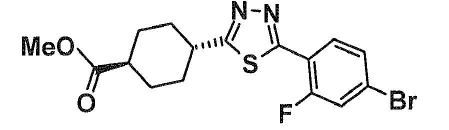 | ESI+: 399.2, 401.2 |
| 186 | 20 | 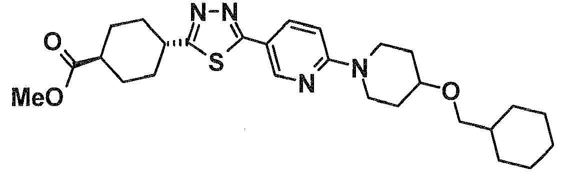 | ESI+: 521.3 [M+Na] |
| 187 | 20 | 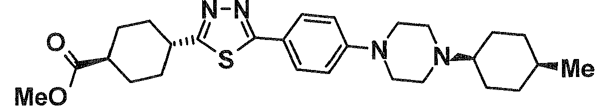 | ESI+: 484.2 |
| 188 | 1 | 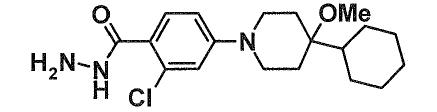 | ESI+: 366.4 |
| 189 | 1 | 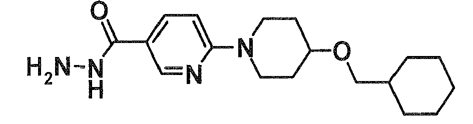 | ESI+: 355.3 [M+Na] |
| 190 | 3 | 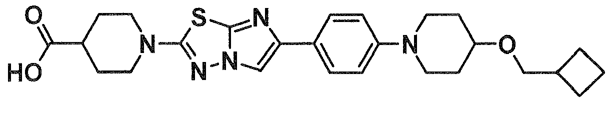 | ESI+: 496.3 |

**[Table 51]**

| | | | |
|---|---|---|---|
| 191 | 3 | 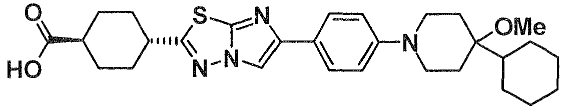 | ESI+: 523.6 |
| 192 | 3 | 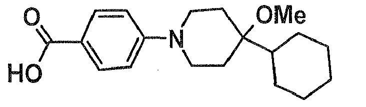 | ESI+: 318.3 |
| 193 | 17 | 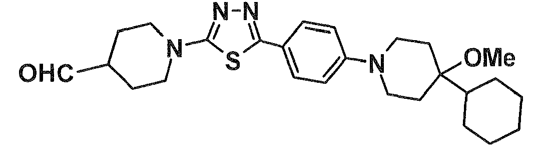 | ESI+: 469.4 |
| 194 | 17 | 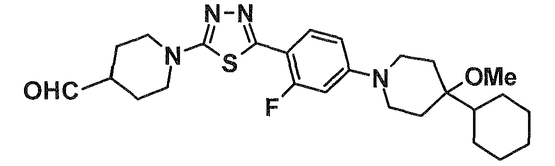 | ESI+: 487.4 |
| 195 | 17 | 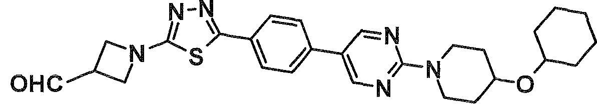 | ESI+: 505.3 |
| 196 | 17 | 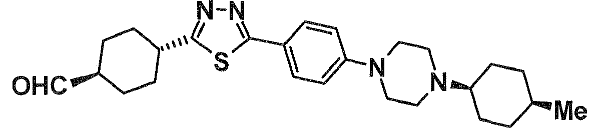 | ESI+: 453.1 |
| 197 | 17 | 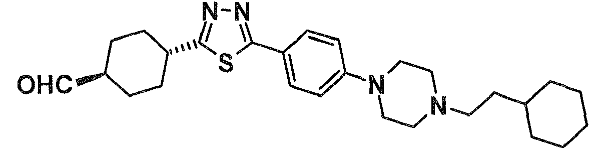 | ESI+: 467.4 |
| 198 | 17 | 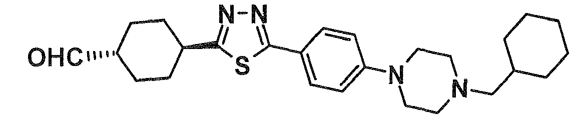 | ESI+: 453.4 |

**[Table 52]**

| | | | |
|---|---|---|---|
| 199 | 17 | 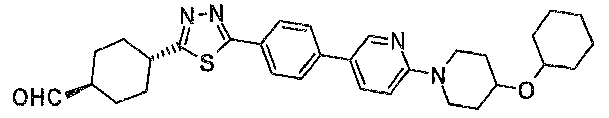 | ESI+: 531.9 |
| 200 | 17 | 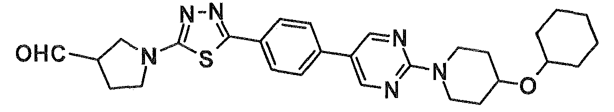 | ESI-: 517.2 |
| 201 | 17 | 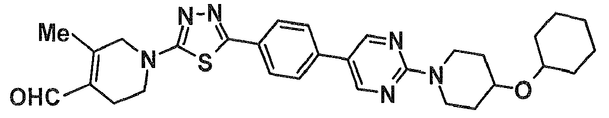 | ESI+: 545.3 |
| 202 | 17 | 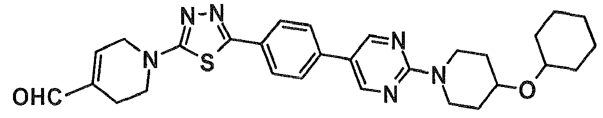 | ESI+: 531.3 |
| 203 | 35 | 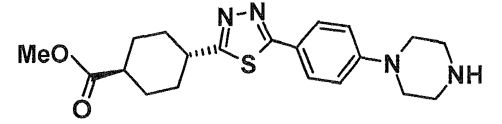 | ESI+: 387.3 |
| 204 | 30 | 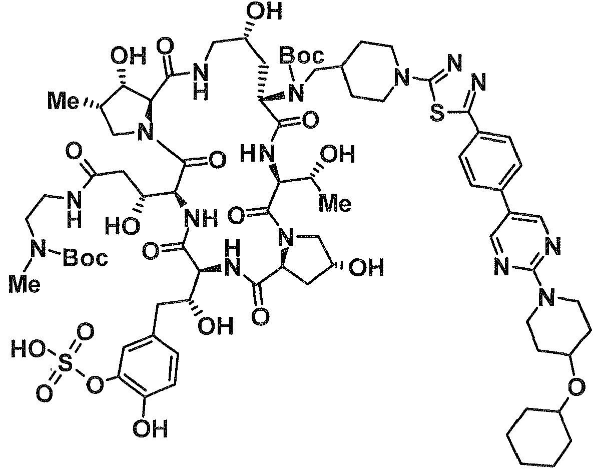 | ESI-: 1676.5 |

**[Table 53]**

| | | | |
|---|---|---|---|
| 205 | 30 | 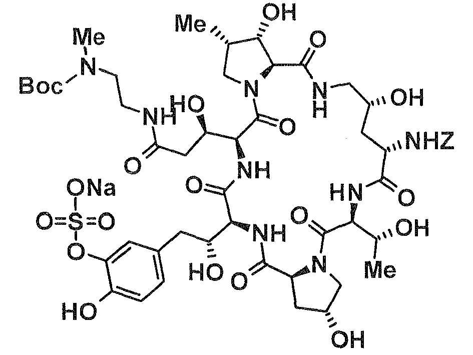 | ESI-: 1194.9 |
| 206 | 32 | 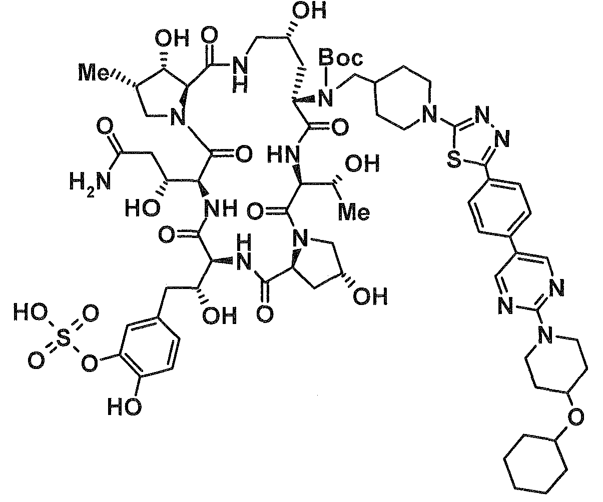 | ESI-: 1520.3 |
| 207 | 27 | 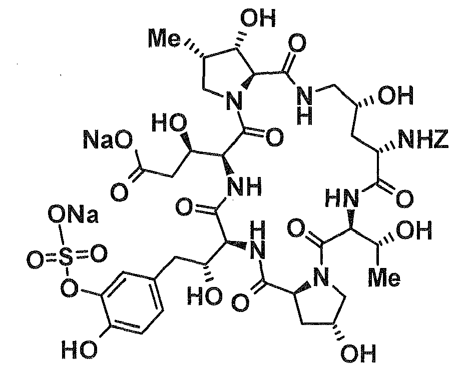 | ESI-: 1038.8 |

**[Table 54]**

| | |
|---|---|
| Ex 43 | Str 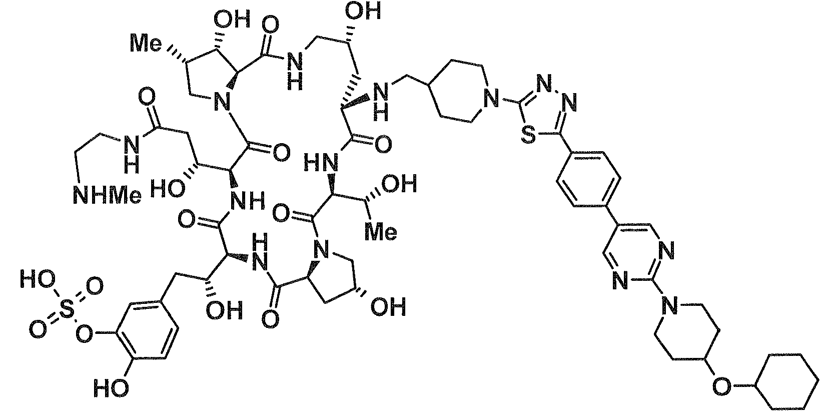 |
| 44 | 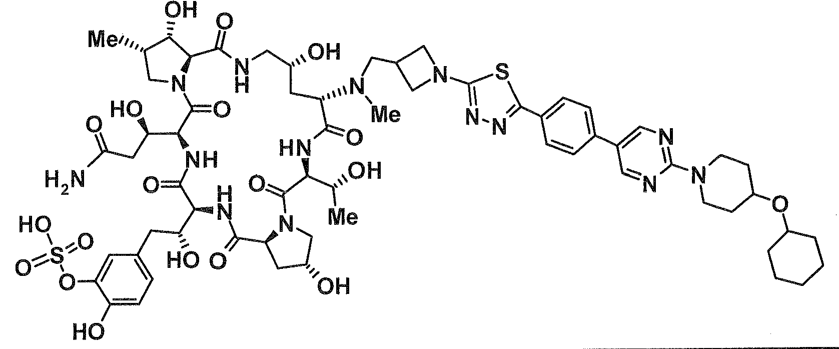 |
| 45 | 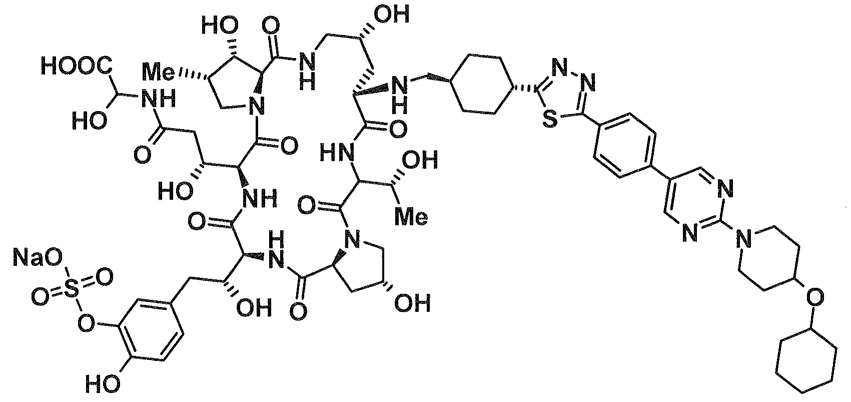 |

**[Table 55]**

| | |
|---|---|
| 46 | 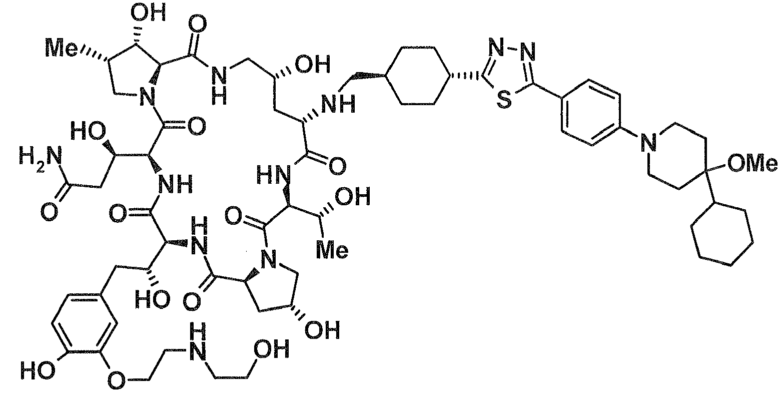 |
| 47 | 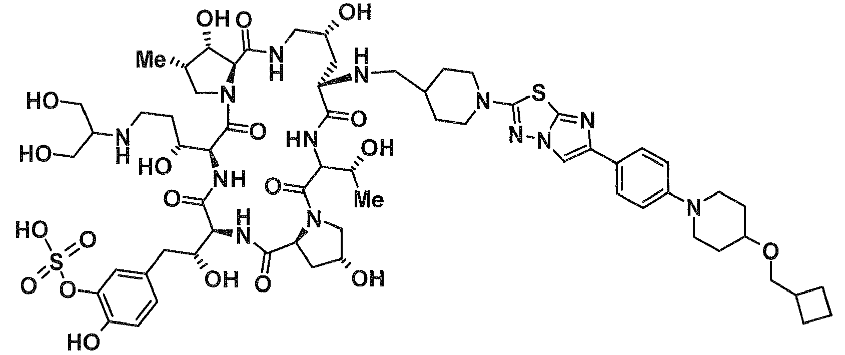 |
| 48 | 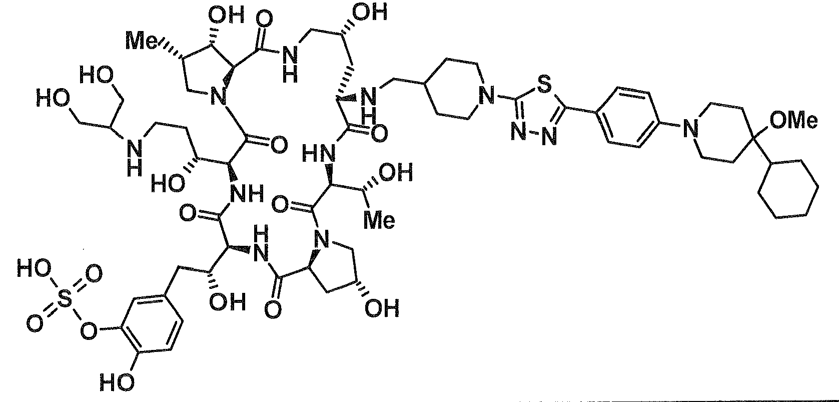 |

**[Table 56]**

| | |
|---|---|
| 49 | 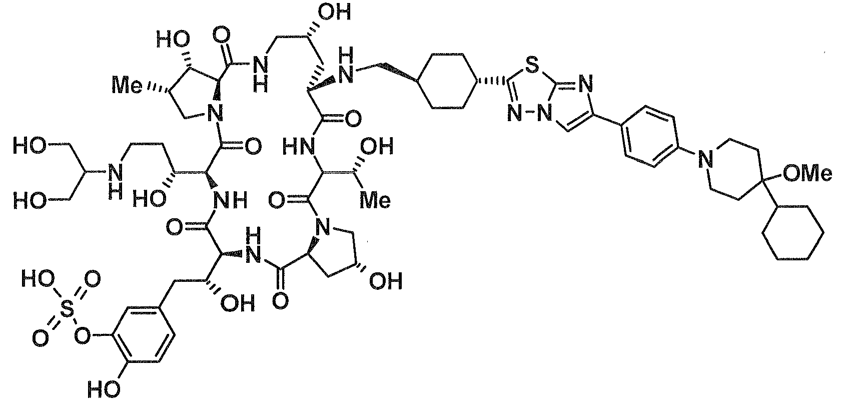 |
| 50 | 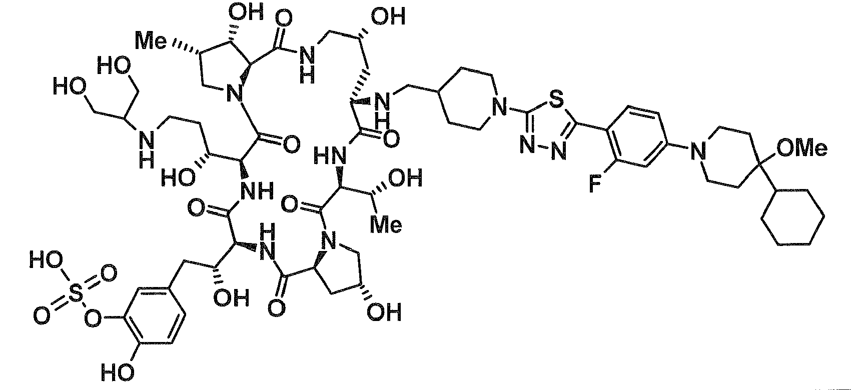 |
| 51 | 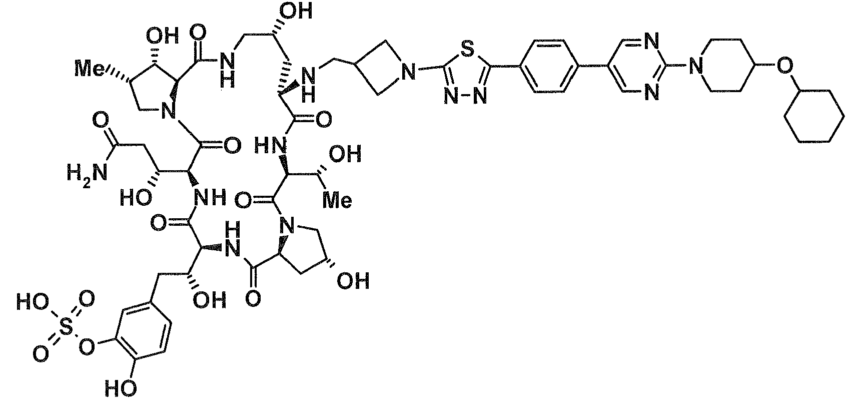 |

**[Table 57]**

| | |
|---|---|
| 52 | 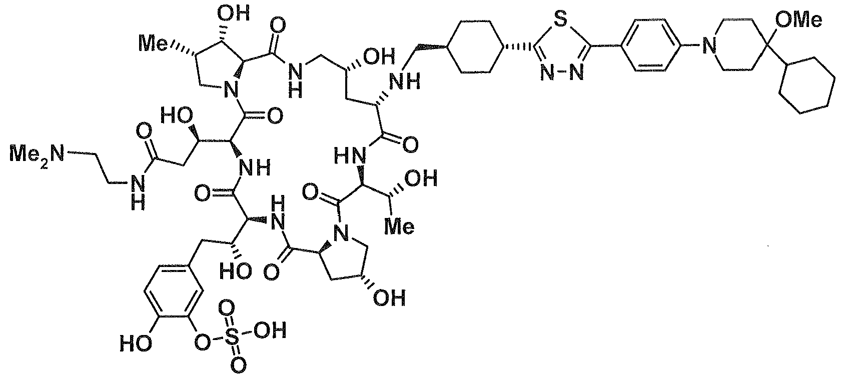 |
| 53 | 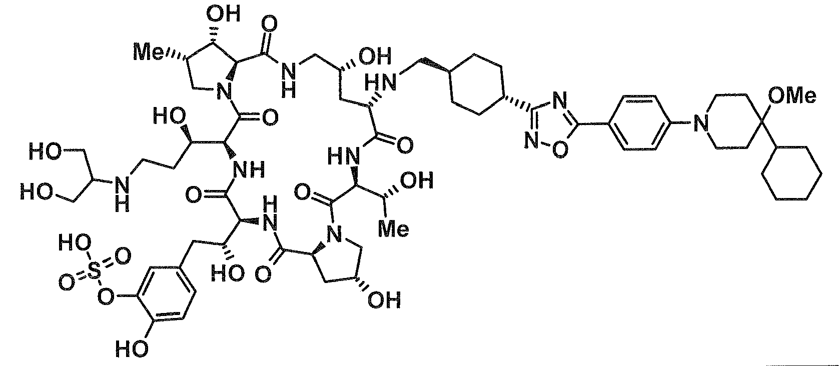 |
| 54 | 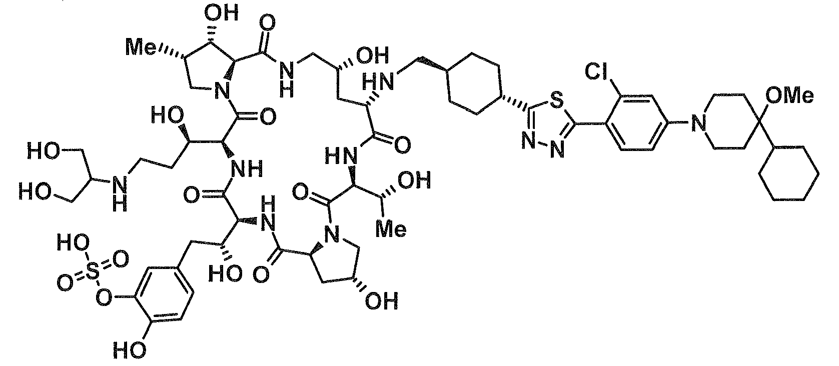 |

**[Table 58]**

| | |
|---|---|
| 55 | 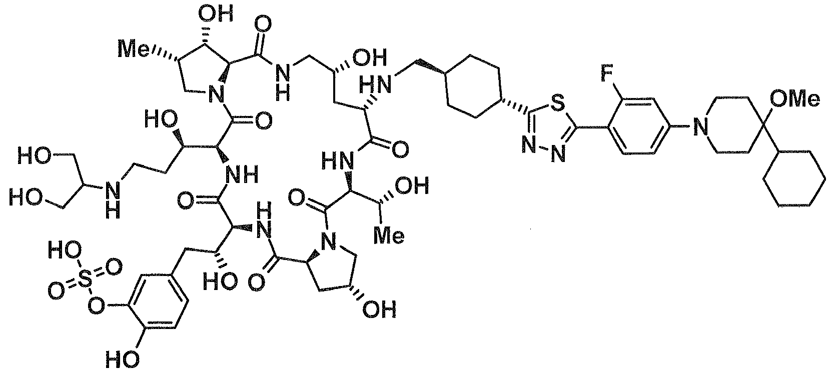 |
| 56 | 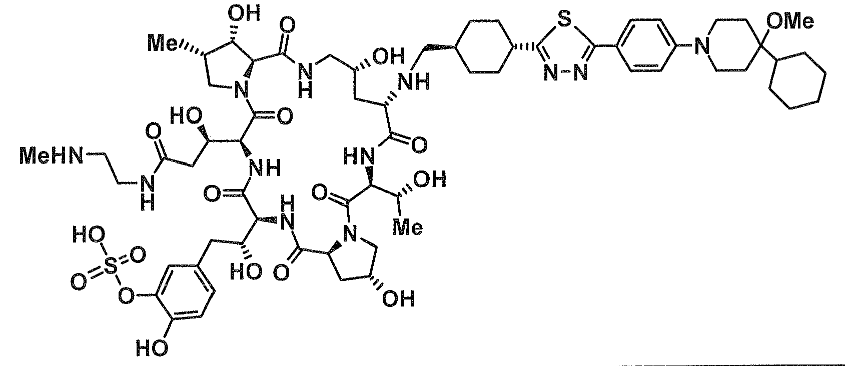 |
| 57 | 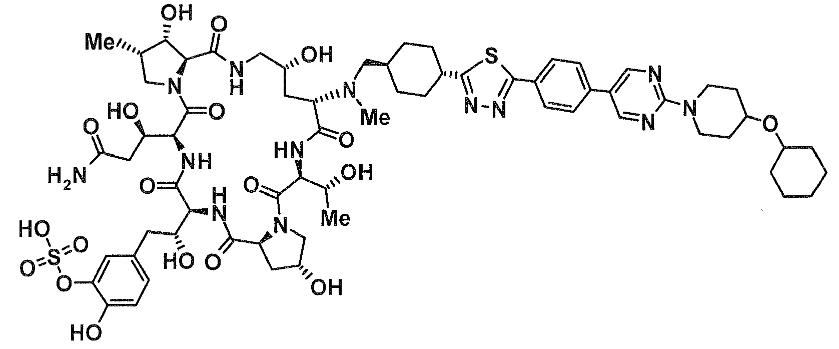 |

**[Table 59]**

| | |
|---|---|
| 58 | 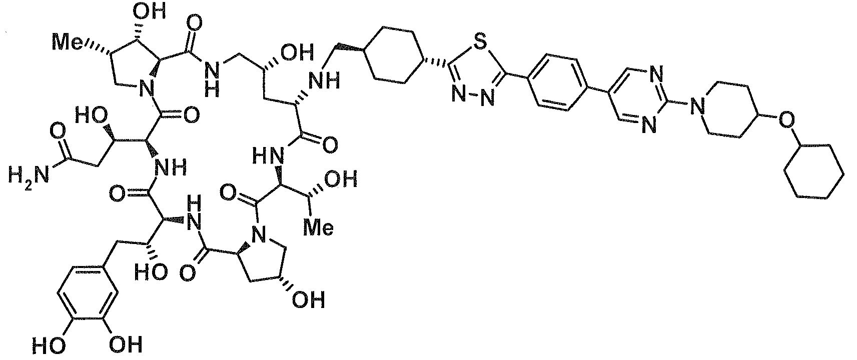 |
| 59 | 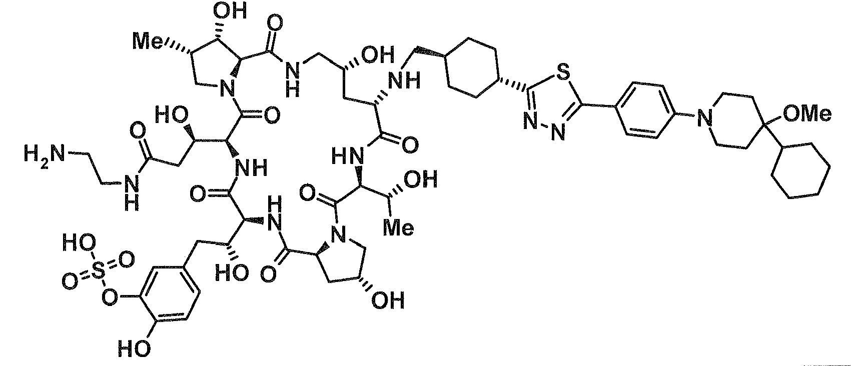 |
| 60 | 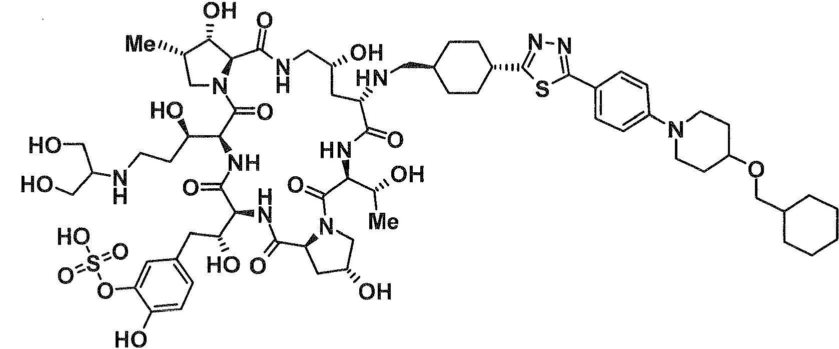 |

**[Table 60]**

| | |
|---|---|
| 61 | 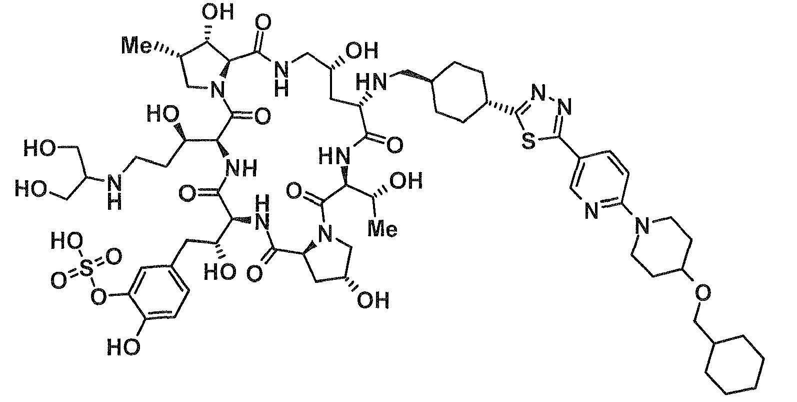 |
| 62 | 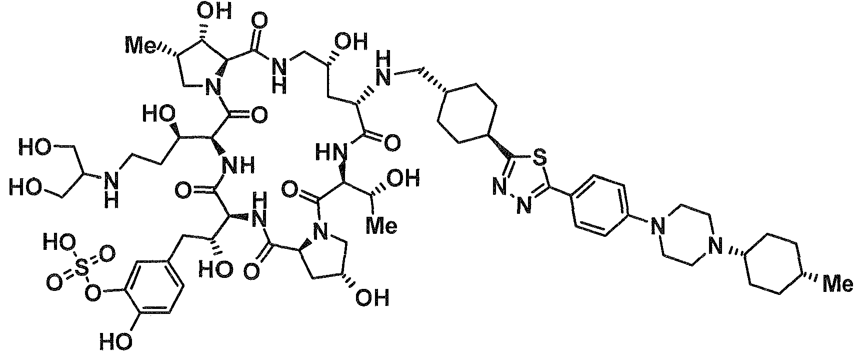 |
| 63 | 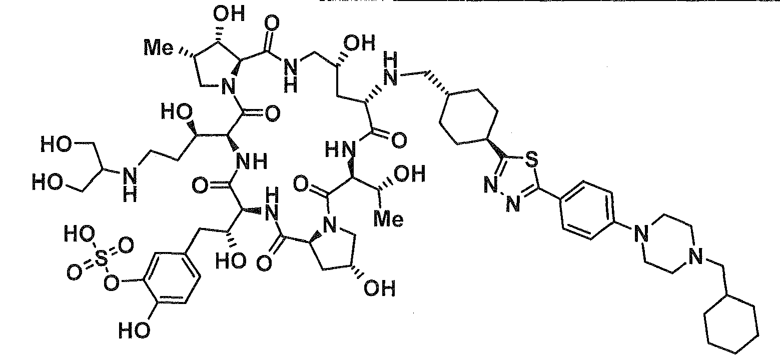 |

**[Table 61]**

| | |
|---|---|
| 64 | 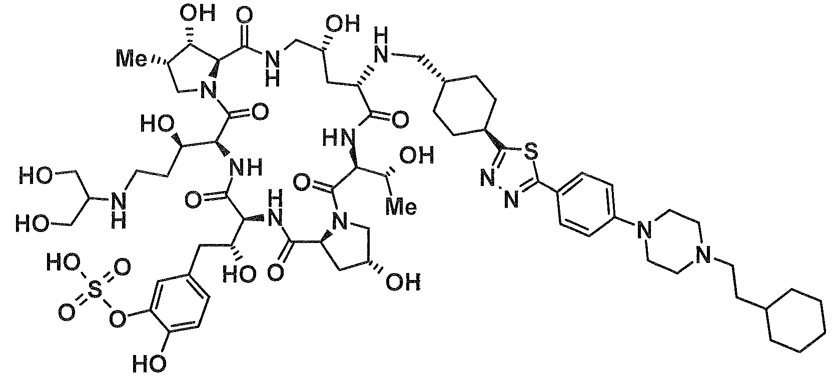 |
| 65 | 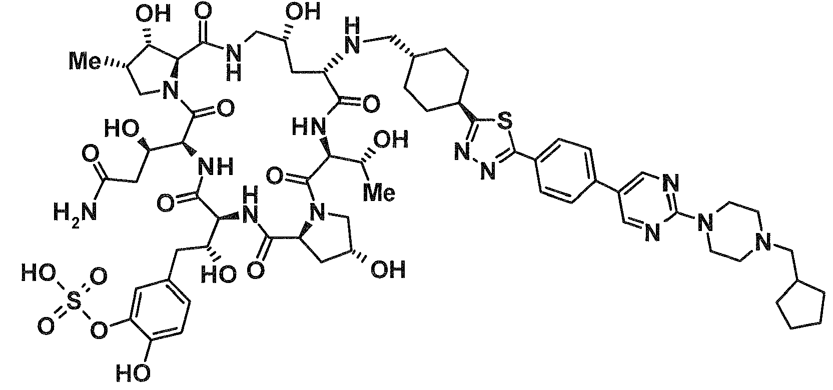 |
| 66 | 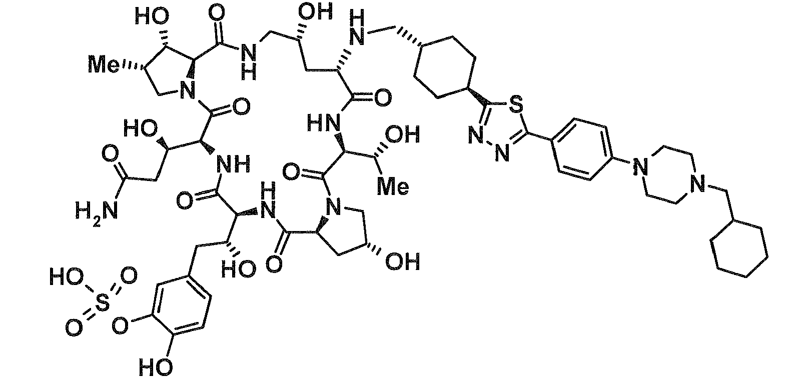 |

**[Table 62]**

| | |
|---|---|
| 67 | 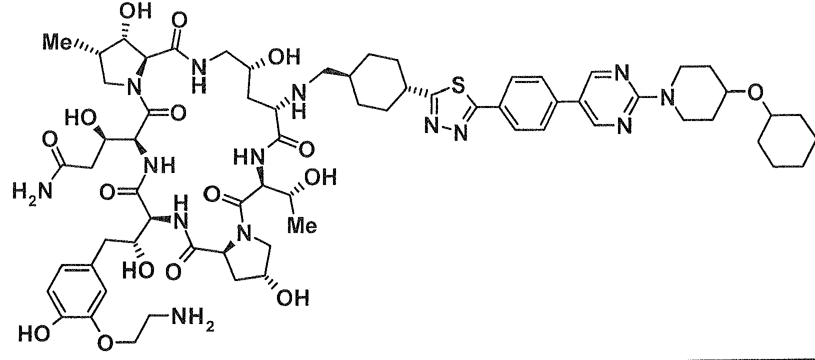 |
| 68 | 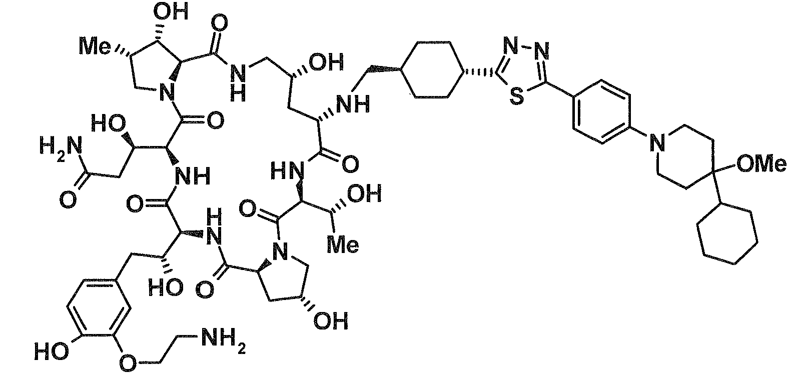 |
| 69 | 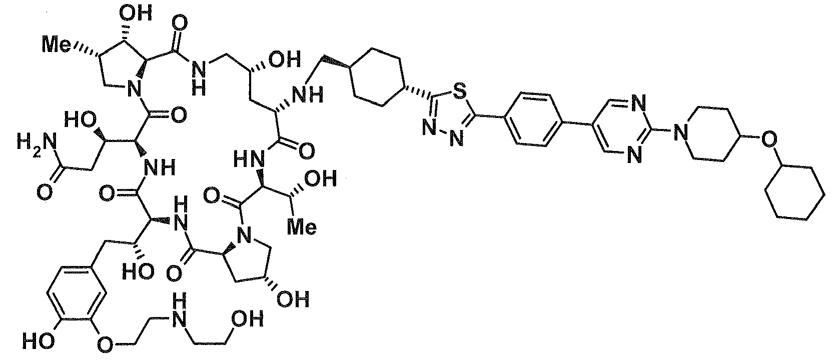 |

**[Table 63]**

| | |
|---|---|
| 70 | 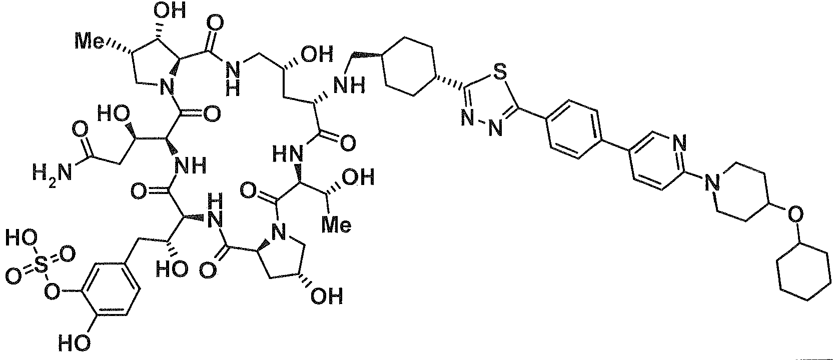 |
| 71 | 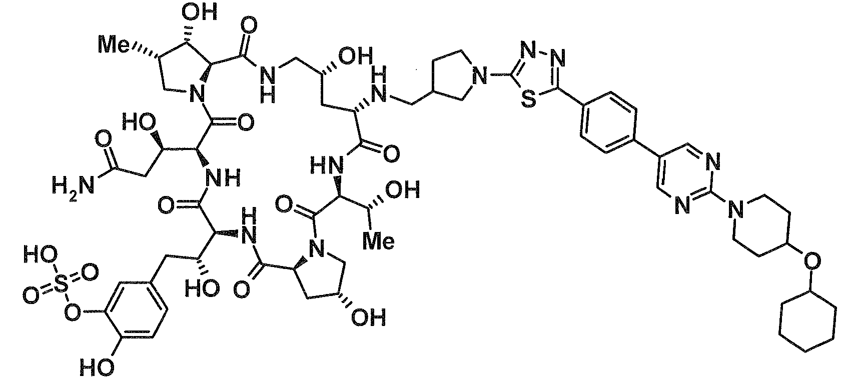 |
| 72 | 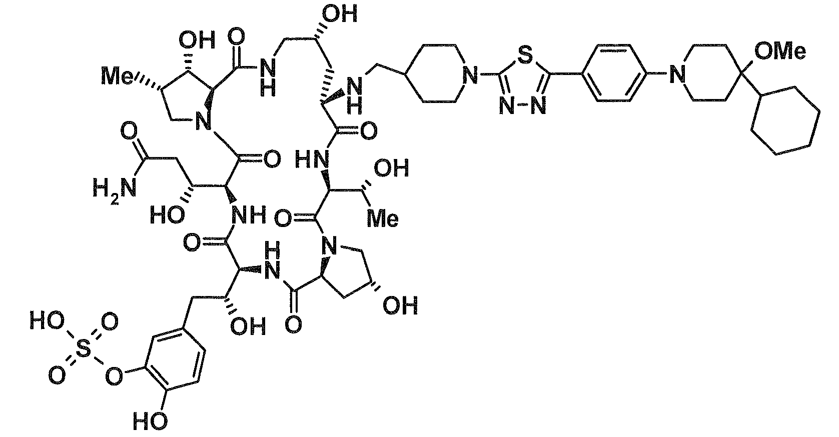 |

**[Table 64]**

| | |
|---|---|
| 73 | 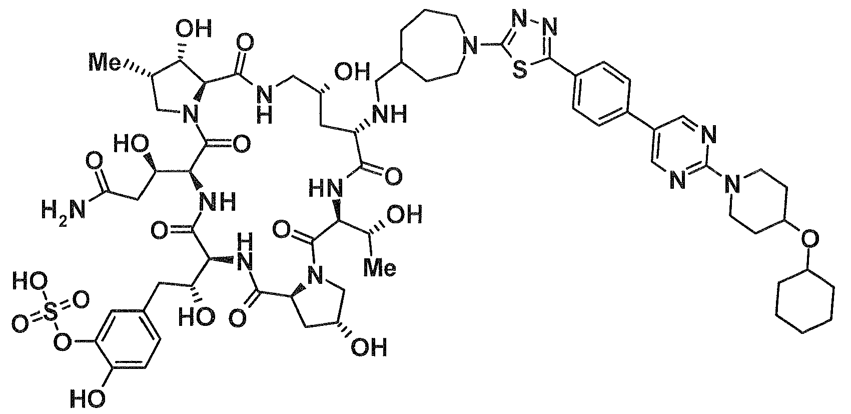 |
| 74 | 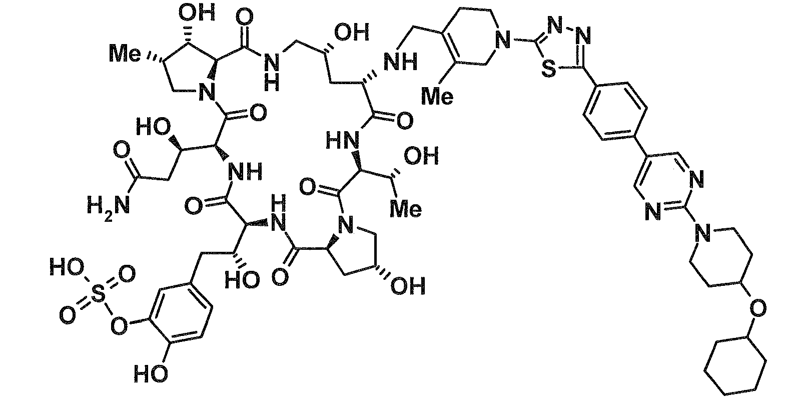 |
| 75 | 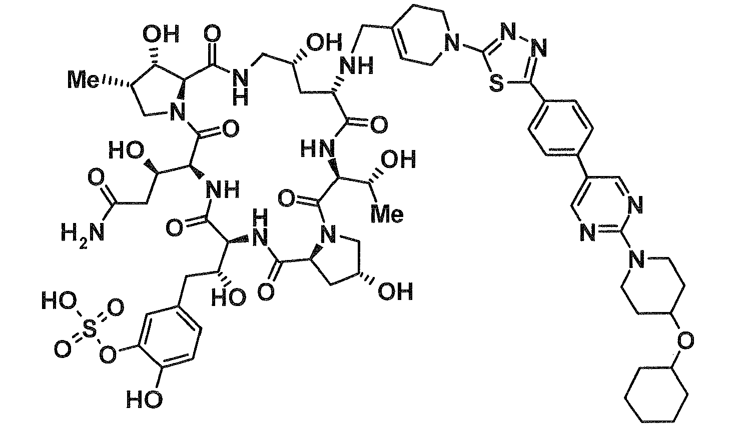 |

**[Table 65]**

| Ex | Syn | Sal | Dat |
|---|---|---|---|
| 43 | D | - | ESI-: 1477.1 |
| 44 | E | - | ESI-: 1406.5 |
| 45 | F | - | ESI+: 1493.3 NMR2: 0.87-1.29 (14H, m), 1.47-1.69 (12H, m), 1.69-1.88 (4H, m), 1.88-2.05 (2H, m), 2.05-2.31 (5H, m), 2.39-2.58 (2H, m), 2.59-2.73 (2H, m), 2.74-2.91 (2H, m), 2.91-3.02 (2H, m), 3.08 (3H, s), 3.13 (2H, t, J = 5.2 Hz), 3.14-3.25 (2H, m), 3.37-3.43 (2H, m), 3.60-3.68 (2H, m), |
| 46 | B | 2HCl | 3.71 (2H, t, J = 5.2 Hz), 3.73-3.86 (2H, m), 3.93-4.13 (4H, m), 4.13- 4.33 (6H, m), 4.33-4.41 (1H, m), 4.43-4.48 (1H, m), 4.49-4.58 (1H, m), 4.65-4.73 (1H, m), 4.81-4.89 (1H, m), 6.61-6.65 (1H, m), 6.69-6.76 (2H, m), 7.03 (2H, d, J = 8.9 Hz), 7.73 (2H, d, J = 8.9 Hz); ESI+: 682.8 [(M+2H)/2] |
| 47 | B | - | ESI-: 1427.3 |
| 48 | B | - | ESI+: 1416.6 |
| 49 | B | - | ESI-: 1454.1 |
| 50 | B | - | ESI-: 1434.4 |
| 51 | B | - | ESI-: 1391.3 |
| 52 | B | - | ESI-: 1426.5 |
| 53 | B | - | ESI+: 1398.4 |
| 54 | B | - | ESI-: 1450.1 |
| 55 | B | - | ESI-: 1434.2 |
| 56 | D | - | ESI-: 1412.5 |
| 57 | B | - | ESI-: 1433.5 |
| 58 | C | H₂SO₄ | ESI-: 1338.6 |
| 59 | A | - | ESI-: 1398.4 NMR1: 0.855-1.96 (36H, m), 2.06-2.18 (2H, m), 2.19-2.46 (3H, m), 2.67-2.82 (1H, m), 2.94-3.14 (6H, m), 3.24 (3H, d, J = 6.4 Hz), 3.41-4.05 (11H, m), 4.13-4.32 (2H, m), 4.35-4.49 (2H, m), 4.77-4.99 (3H, |
| 60 | B | - | m), 5.09-5.37 (6H, m), 6.71 (1H, d, J = 7.6 Hz), 6.77 (1H, dd, J = 1.6, 8.0 Hz), 7.03 (2H, d, J = 8.8 Hz), 7.41-7.61 (1H, m), 7.72 (1H, d, J = 7.6 Hz), 7.83-7.92 (1H, m), 8.05-8.37 (1H, m), 8.70 (1H, s); ESI-: 1414.6 |
| 61 | B | - | ESI-: 1415.5 |
| 62 | B | - | ESI-: 1400.5 |
| 63 | B | - | ESI-: 1399.4 |
| 64 | B | - | ESI-: 1414.5 |
| 65 | B | - | ESI-: 1403.3 |
| 66 | B | - | ESI-: 1340.5 |

**[Table 66]**

| | | | |
|---|---|---|---|
| 67 | A | 2HCl | ESI+: 692.5 [(M+2H)/2] NMR2: 0.88-1.29 (14H, m), 1.46-1.87 (16H, m), 1.88-2.30 (7H, m), 2.39-2.57 (2H, m), 2.59-2.70 (2H, m), 2.73-2.90 (2H, m), 2.97-3.28 (9H, m), 3.57-3.67 (2H, m), 3.70-3.85 (2H, m), 3.92-4.04 (2H, m), |
| 68 | A | 2HCl | 4.04-4.21 (6H, m), 4.21-4.31 (2H, m), 4.32-4.40 (1H, m), 4.42-4.48 (1H, m), 4.50-4.60 (1H, m), 4.65-4.73 (1H, m), 4.79-4.89 (1H, m), 6.62 (1H, dd, J = 8.0, 1.3 Hz), 6.70 (1H, d, J = 8.0 Hz), 6.75 (1H, d, J = 1.3 Hz), 7.16 (2H, d, J = 8.5 Hz), 7.78 (2H, d, J = 8.6 Hz); ESI+: 660.4 [(M+H)/2] |
| 69 | A | 2HCl | ESI+: 714.5 [(M+2H)/2] NMR2: 0.96 (3H, d, J = 6.7Hz), 1.14 (3H, d, J = 6.0Hz), 1.12-2.50 (33H, m), 2.58-3.68 (11H, m), 3.61-4.01 (5H, m), 4.05-4.44 (8H, m), |
| 70 | B | - | 472-4.82 (2H, m), 6.71-6.75 (2H, m), 6.94-6.99 (2H, m), 7.80 (2H, d, J = 8.8Hz), 7.91-7.99 (3H, m), 8.53 (1H, s); ESI-: 1418.1 |
| 71 | B | - | ESI-: 1405.4 |
| 72 | B | - | ESI-: 1356.4 |
| 73 | B | - | ESI-: 1433.3 |
| 74 | B | - | ESI-: 1432.6 |
| 75 | B | - | ESI-: 1418.1 |

## Claims

1. A compound of the formula (I) or a pharmaceutically acceptable salt thereof:
[chem. 11] 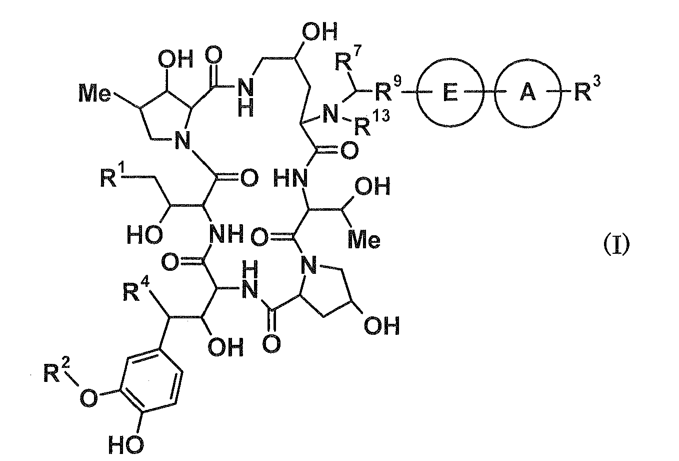
(wherein the symbols in the formula have the following meanings,
R¹ represents -CO-NR⁵R⁶, -R⁰⁰-NR⁵R⁶, -CO-(nitrogen-containing hetero ring) or -NH-R⁰⁰-NR⁵R⁶
R⁵ and R⁶ are the same as or different from each other, and represent H, R⁰, R^{A} or R^{B},
R^{A} represents lower alkyl which may be substituted with one to three hydroxyl groups,
R^{B} represents lower alkyl which may be substituted with one to three groups selected from -NH₂, -NHR⁰ and -N(R⁰)₂,
R² represents H, -S(O)₂-OH, R⁰, R^{A}, R^{B} or -R⁰⁰-NH-R⁰⁰-OH,
R⁴ represents H or -OH,
R⁷ is the same as or different from each other, and represents H, R⁰ or -R⁰⁰-OR⁰,
R⁹ represents a single bond,
R¹³ and R¹⁴ are the same as or different from each other, and represent H or R⁰,
E ring represents a group represented by the following formulae (II), (III), (IV), (V) or (XVIII),
[chem. 12] 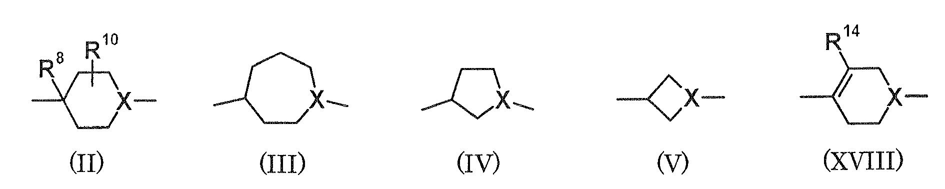 wherein X represents CH or N,
R⁸ is the same as or different from each other, and represents H, R⁰, -NH₂, -N(R⁰)₂, -NHR⁰, -OH or -R⁰⁰-OR⁰,
R¹⁰ represents H, R⁰, -NH₂, -N(R⁰)₂, -NHR⁰, -OH or -R⁰⁰-OR⁰,
A ring represents a nitrogen-containing hetero ring or phenyl,
R³ represents a group represented by the following formulae (VI),(VII),(VIII) or
(XIX), 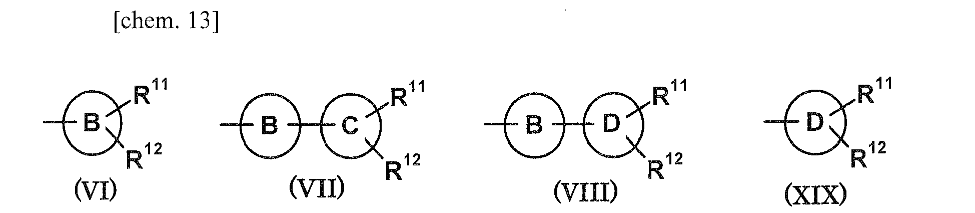
wherein B ring and D ring are the same as or different from each other, and represent aryl which may be substituted with halogen,
C ring represents a nitrogen-containing hetero ring,
R¹¹ and R¹² are the same as or different from each other, and represent a group selected from H, R⁰, cycloalkyl, -(cycloalkyl)-R⁰, -R⁰⁰-(cycloalkyl), -OR⁰, -O-(cycloalkyl), -O-(C₂₋₁₀ alkylene)-OR⁰, -O-R⁰⁰-(cycloalkyl), -(nitrogen-containing hetero ring)-R⁰⁰-(cycloalkyl), -(nitrogen-containing hetero ring)-O-(cycloalkyl) and -(nitrogen-containing hetero ring)-O-R⁰⁰-(cycloalkyl),
R⁰ represents lower alkyl, and
R⁰⁰ represents lower alkylene.)

2. The compound according to claim 1, wherein R¹ is -CO-NR⁵R⁶ or -R⁰⁰-NR⁵R⁶, R² is -S(O)₂-OH or R⁰, R⁴ is H, R⁷ is H or R⁰, R⁹ is a single bond, E ring is a group represented by the formula (II), A ring is a monocyclic nitrogen-containing hetero ring, R⁸ is H or R⁰, R³ is a group represented by the formula (VII) or (VIII), B ring and D ring, which are the same as or different from each other, are each monocyclic aryl, C ring is a monocyclic nitrogen-containing hetero ring, and R¹¹ and R¹², which are the same as or different from each other, are each H, cycloalkyl, -O-R⁰, -O-(cycloalkyl), -(nitrogen-containing hetero ring)-O-(cycloalkyl) or -(nitrogen-containing hetero ring)-O-R⁰⁰-(cycloalkyl).

3. The compound according to claim 1, wherein R¹ is -CO-NH₂, -CH₂-NH₂ or -CH₂-NH-CH(CH₂OH)₂, R² is -S(O)₂-OH or methyl, R⁴ is H, R⁷ is H, E ring is cyclohexanediyl, A ring is thiadiazolyl or imidazothiazolyl, R³ is a group represented by the formula (VII), B ring and D ring are phenyl, and C ring is, together with R¹¹ and R¹², a group represented by the following formulae (XX) or (XXI): 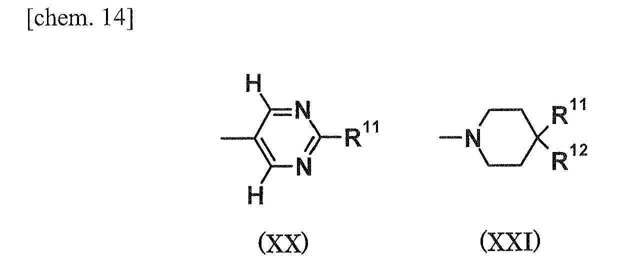 wherein either of R¹¹ and R¹² is H or -O-(methyl), and the other is a group selected from cyclohexyl, -O-(cyclohexyl), -O-CH₂-(cyclobutyl), and 4-(cyclohexyloxy) piperidin-1-yl.

4. (3R)-3-{(2R, 6S, 11R, 14aS, 15S, 16S, 20S, 23S, 25aS)-9-[({trans-4-[5-(4-{2-[4-(cyclopentylmethyl)piperazin-1-yl]pyrimidin-5-yl}phenyl)-1, 3, 4-thiadiazol-2-yl]cyclohexyl}methyl)amino]-2, 11, 15-trihydroxy-6-[(1R)-1-hydroxyethyl]-23-[(1R)-1-hydroxy-2-(4-hydroxy-3-methoxyphenyl)ethyl]-16-methyl-5, 8, 14, 19, 22, 25-hexaoxotetracosahydro-1H-dipyrrolo[2, 1-c:2', 1'-1][1, 4, 7, 10, 13, 16]hexaazacyclohenicosin-20-yl}-3-hydroxypropanamide dihydrochloride,
(2R, 6S, 9S, 11R, 14aS, 15S, 16S, 20S, 23S, 25aS)-9-{[(trans-4-{5-[4-(4-cyclohexyl-4-methoxypiperidin-1-yl)phenyl]-1, 3, 4-thiadiazol-2-yl}cyclohexyl)methyl]amino}-2, 11, 15-trihydroxy-6-[(1R)-1-hydroxyethyl]-20-[(1R)-1-hydroxy-3-{[2-hydroxy-1-(hydroxymethyl)ethyl]amino}propyl]-23-[(1R)-1-hydroxy-2-(4-hydroxy-3-methoxyphenyl)ethyl]-16-methylhexadecahydro-1H-dipyrrolo[2, 1-c:2', 1'-1][1, 4, 7, 10, 13, 16]hexaazacyclohenicosine-5, 8, 14, 19, 22, 25(9H, 25aH)-hexone dihydrochloride, 2-hydroxy-5-{(2R)-2-hydroxy-2-[(2R, 11R, 14aS, 15S, 16S, 20S, 23S, 25aS)-2, 11, 15-trihydroxy-6-[(1R)-1-hydroxyethyl]-20-[(1R)-1-hydroxy-3-{[2-hydroxy-1-(hydroxymethyl)ethyl]amino}propyl]-9-({[trans-4-(2-{4-[(6-methoxyhexyl)oxy]phenyl}imidazo[2, 1-b][1, 3, 4]thiadiazol-6-yl)cyclohexyl]methyl}amino)-16-methyl-5, 8, 14, 19, 22, 25-hexaoxotetracosahydro-1H-dipyrrolo[2, 1-c:2', 1'-1][1, 4, 7, 10, 13, 16]hexaazacyclohenicosin-23-yl]ethyl}phenyl hydrogen sulfate,
(2R, 6S, 11R, 14aS, 15S, 16S, 20S, 23S, 25aS)-20-[(1R)-3-amino-1-hydroxypropyl]-9-{[(trans-4-{5-[4-(4-cyclohexyl-4-methoxypiperidin-1-yl)phenyl]-1, 3, 4-thiadiazol-2-yl}cyclohexyl)methyl]amino}-2, 11, 15-trihydroxy-6-[(1R)-1-hydroxyethyl]-23-[(1R)-1-hydroxy-2-(4-hydroxy-3-methoxyphenyl)ethyl]-16-methylhexadecahydro-1H-dipyrrolo[2, 1-c:2', 1'-1][1, 4, 7, 10, 13, 16]hexaazacyclohenicosine-5, 8, 14, 19, 22, 25(9H, 25aH)-hexone dihydrochloride,
5-[(2R)-2-{(2R, 6S, 9S, 11R, 14aS, 15S, 16S, 20S, 23S, 25aS)-20-[(1R)-3-amino-1-hydroxy-3-oxopropyl]-9-[({trans-4-[5-(4-{2-[4-(cyclohexyloxy)piperidin-1-yl]pyrimidin-5-yl}phenyl)-1, 3, 4-thiadiazol-2-yl]cyclohexyl}methyl)amino]-2, 11, 15-trihydroxy-6-[(1R)-1-hydroxyethyl]-16-methyl-5, 8, 14, 19, 22, 25-hexaoxotetracosahydro-1H-dipyrrolo[2, 1-c:2', 1'-1][1, 4, 7, 10, 13, 16]hexaazacyclohenicosin-23-yl}-2-hydroxyethyl]-2-hydroxyphenyl hydrogen sulfate,
5-[(2R)-2-{(2R, 6S, 9S, 11R, 14a, 15S, 16S, 20S, 23S, 25aS)-20-{(1R)-3-[(2-aminoethyl)amino]-1-hydroxy-3-oxopropyl}-9-[({1-[5-(4-{2-[4-(cyclohexyloxy)piperidin-1-yl]pyrimidin-5-yl}phenyl)-1, 3, 4-thiadiazol-2-yl]piperidin-4-yl}methyl)amino]-2, 11, 15-trihydroxy-6-[(1R)-1-hydroxyethyl]-16-methyl-5, 8, 14, 19, 22, 25-hexaoxotetracosahydro-1H-dipyrrolo[2, 1-c:2', 1'-1][1, 4, 7, 10, 13, 16]hexaazacyclohenicosin-23-yl}-2-hydroxyethyl]-2-hydroxyphenyl hydrogen sulfate, and 5-[(2R)-2-{(2R, 6S, 11R, 14aS, 15S, 16S, 20S, 23S, 25aS)-9-({[trans-4-(5-{4-[4-(cyclohexyloxy)piperidin-1-yl]phenyl}-1, 3, 4-thiadiazol-2-yl)cyclohexyl]methyl}amino)-2, 11, 15-trihydroxy-6-[(1R)-1-hydroxyethyl]-20-[(1R)-1-hydroxy-3-{[2-hydroxy-1-(hydroxymethyl)ethyl]amino}propyl]-16-methyl-5, 8, 14, 19, 22, 25-hexaoxotetracosahydro-1H-dipyrrolo[2, 1-c:2', 1'-1][1,4, 7, 10, 13, 16]hexaazacyclohenicosin-23-yl-2-hydroxyethyl]-2-hydroxyphenyl hydrogen sulfate.

5. A pharmaceutical composition comprising the compound or a pharmaceutically acceptable salt thereof according to claim 1 and a pharmaceutically acceptable excipient.

6. A pharmaceutical composition for preventing or treating a fungal infection comprising the compound or a pharmaceutically acceptable salt thereof according to claim 1.

7. Use of the compound or a pharmaceutically acceptable salt thereof according to claim 1 for the manufacture of an agent for preventing or treating a fungal infection.

8. A method for preventing or treating a fungal infection comprising administering an effective amount of the compound or a pharmaceutically acceptable salt thereof according to claim 1 to a patient.
